# FASCICULE DE BREVET EUROPEEN

(11) **EP 1 756 029 B1**
(45) Date de publication et mention de la délivrance du brevet: **10.10.2007**
(21) Numéro de dépôt: 05753640.1
(22) Date de dépôt: 08.04.2005
(51) Int. Cl.: C07C 47/24, C07D 311/58

(54) **NOUVEAUX COMPOSES, LEUR PREPARATION ET LEUR UTILISATION POUR LA SYNTHESE REGIOSPECIFIQUE D'HETEROCYCLES A GROUPEMENT PERFLUORO-ALKYLE.**
NEUE VERBINDUNGEN, DEREN HERSTELLUNG UND DEREN VERWENDUNG FÜR DIE REGIOSPEZIFISCHE SYNTHESE VON HETEROCYCLEN MIT PERFLUOR(ALKYL)GRUPPE
NOVEL COMPOUNDS, THE PREPARATION AND THE USE THEREOF FOR A REGIOSPESIFIC SYNTHESIS OF PERFLUOR(ALKYL) GROUP HETEROCYCLES

(30) Priorité: 08.04.2004 FR 0403684
(43) Date de publication de la demande: 28.02.2007
(73) Titulaire: CENTRE NATIONAL DE LA RECHERCHE SCIENTIFIQUE C.N.R.S), 75016 Paris (FR); UNIVERSITE MONTPELLIER II SCIENCES ET TECHNIQUES DU LANGUEDOC, F-34095 Montpellier Cédex (FR)
(72) Inventeur: EL KHARRAT, Salem, F-FR MONTPELLIER (FR); LAURENT, Philippe, F-34280 CARNON (FR); BLANCOU, Hubert, F-34480 PUISSALICON (FR)
(74) Mandataire: Habasque, Etienne J. Jean-François
(86) Numéro de dépôt international: PCT/FR2005/000863
(87) Numéro de publication internationale: WO 2005/102980

(56) Documents cités:
- SEVENARD D V ET AL: "Regioselective 1,4-trifluoromethylation of alpha,beta-enones using 'protect-in-situ' methodology" TETRAHEDRON LETTERS, ELSEVIER SCIENCE PUBLISHERS, AMSTERDAM, NL, vol. 44, no. 41, 6 octobre 2003 (2003-10-06), pages 7623-7627, XP004455575 ISSN: 0040-4039
- SOSNOVSKIKH: "Regioselective Nucleophilic 1,4-Trifluoromethylation of 2-Polyfluoroalkylchromones with (Trifluoromethyl)trimethylsilane. Synthesis of Fluorinated Analogs of Natural 2,2-Dimethylchroman-4-ones and 2,2-Dimethylchromnes" J. ORG. CHEM., vol. 68, no. 20, 2003, pages 7747-7754, XP002305290
- AUBERT C ET AL: "Méthode Générale d'Accès aux Trifluorométhylcétones. 1ère Partie. Alkylation Directe du trifluoroaceétylacétate d'Ethyle" JOURNAL OF FLUORINE CHEMISTRY, ELSEVIER SEQUOIA. LAUSANNE, CH, vol. 44, 1989, pages 361-376, XP002128794 ISSN: 0022-1139 cité dans la demande

## Description

L'invention concerne de nouveaux composés utiles en tant qu'intermédiaires pour la synthèse régiospécifique d'hétérocycles à groupement perfluoro-alkylé, le procédé de synthèse de ces nouveaux composés ainsi que leur utilisation.

De nombreuses méthodes ont été mises au point pour l'élaboration de matières actives pharmaceutiques comportant des hétérocycles substitués par un groupement perfluoroalkyle, notamment par des groupes -CF₃.

Diverses molécules hétérocycliques mono-fluorées ont eu un succès incontestable dans le domaine de la pharmacie : Fluoro-5-uracile comme anti-cancéreux, oxazolidinones fluorées comme antibiotique, fluorocystéine comme antiviral. D'autres molécules trifluorométhylées, comme les trifluorométhylquinoléines comme antimalarien, les trifluorométhyl-bis-quinoléines comme anti-rejet dans la transplantation d'organes. Par ailleurs, les hétérocycles non fluorés sont très connus pour entrer dans la composition d'une grande variété de molécules biologiquement actives dérivées de l'isoxazole, du pyrazole et de la pyridine.

Majoritairement les méthodes mises au point font intervenir des cétones et plus particulièrement des cétones α,β-insaturées. Toute une revue de telles cétones est en particulier donnée dans Nenajdenko et al., «preparation of α,β-unsaturated ketones bearing a trifluoromethyl group and their application in organic synthesis », Molecules 1997, 2, 186-232.

On peut également citer Aubert et al., « méthode générale d'accès aux trifluométhylcétones 1ère partie : alkylation directe du trifluoroacétylacétate d'éthyle », Journal of Fluorine Chemistry, 44 (1989) 361-376, qui divulgue des trifluorométhylcétones à titre de synthons pour atteindre des composés cycliques portant un groupement -CF₃ sur un carbone alicyclique, obtenus dans des conditions particulières d'alkylation.

D'autres méthodes ont été testées avec comme produit de départ des composés β-dicarbonylés trifluorométhylés. A ce titre on peut citer C.Sloop et al., « Synthesis of fluorinated heterocycles », Journal of fluorine chemistry 118 (2002) 135-147.

Toutefois, les méthodes de préparation d'intermédiaires pour la synthèse de composés hétérocycliques à groupement perfluoro-alkylé proposées dans l'art antérieur présentent divers inconvénients : leur rendement est faible, elles sont limitées au groupement perfluoro-alkylé spécifique -CF₃, et ne peuvent être utilisées pour des groupements perfluoro-alkylés plus longs, et elles sont compliquées à mettre en oeuvre. De plus, certains intermédiaires connus de l'art antérieur sont peu stables car très hydrosolubles ou encore ne permettent pas des réactions régiospécifiques.

Il existe un besoin accru de trouver des solutions alternatives pour la synthèse de composés hétérocycliques comportant au moins un groupement perfluoro-alkyle.

La présente invention concerne des composés utiles comme intermédiaires pour la synthèse régiospécifique de composés hétérocycliques comportant au moins un groupement perfluoro-alkyle, leur procédé de préparation, ainsi que leur utilisation pour la préparation desdits composés hétérocycliques. Ces composés hétérocycliques présentent un grand intérêt dans le domaine des molécules à propriétés biologiques (molécules pharmaceutiques ou phytosanitaires).

Les composés de l'invention sont des composés analogues aux composés β-dicarbonylés, la cétone en α de la chaîne fluorée étant protégée par la fonction énol-éther.

Un avantage majeur de ces composés est qu'ils sont utiles pour préparer une large variété de composés hétérocycliques à groupements perfluoroalkylés régiospécifiquement en position α par rapport à l'hétéroatome.

Par ailleurs, leur procédé d'obtention présente l'avantage de donner de bons rendements et d'être très aisé dans la mesure où il comporte une seule réaction en deux temps à partir de produits fluorés disponibles commercialement.

Un composé, premier objet de la présente invention, répond à la formule (1) 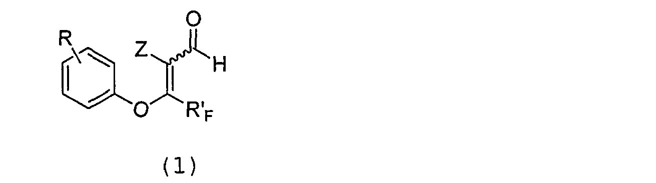 dans laquelle
Z représente un atome d'hydrogène ou un atome d'halogène, de préférence un atome d'hydrogène ;
R'_{F} représente un groupe perfluoroalkyle -CₙF₂ₙ₊₁ où n est un nombre entier pouvant être compris entre 1 et 12 ;
R est en position ortho, méta ou para, et il représente un groupe électrodonneur, un groupe électroattracteur, un groupe aryle, ou un groupe hétéroaryle, étant entendu que si R est un aryle ou un hétéroaryle, il peut former un groupe aromatique à noyaux condensés avec le groupe phényle qui le porte, ou bien R est un atome d'hydrogène ;

En tant que groupe électrodonneur, on peut citer les groupes alkyle comprenant de 1 à 6 atomes de carbone ou les groupes alcoxy comprenant de 1 à 6 atomes de carbone, et de préférence le groupe méthoxy, ou le groupe OH.

En tant que groupe électroattracteur, on peut citer :
- les groupes NO₂ et nitrile,
- les atomes d'halogène,
- un groupe carbonyle -C(=O)-R₉ dans lequel R₉ est un atome d'hydrogène, un groupe OH, un groupe alkyle, un groupe alkoxy, un groupe aryle, un groupe aryloxy, un groupe hétéroaryle, ou un groupe hétéroaryloxy, ledit groupe carbonyle étant fixé sur le groupe qui le porte soit directement, soit par l'intermédiaire d'un groupe alkylène, d'un groupe arylène, d'un groupe hétéroarylène ;
- un groupe -O-C(=O)-R₁₀ dans lequel R₁₀ est un atome d'hydrogène, un groupe alkyle, un groupe aryle, un groupe hétéroaryle, ledit groupe -O-C(=O)-R₁₀ étant fixé sur le groupe qui le porte soit directement, soit par l'intermédiaire d'un groupe alkylène, d'un groupe arylène, d'un groupe hétéroarylène.

Dans le cadre de la présente invention on préfère les composés (1) dans lesquels R'_{F} représente un groupe CₙF₂ₙ₊₁ où n est un nombre entier pouvant être compris entre 1 et 8.

Un composé (1) peut se présenter sous la forme de l'isomère Z ou E. L'existence de ces deux isomères est représentée schématiquement, sur la formule (1), par la liaison ondulée figurant entre la double liaison et la fonction carbonyle.

Il a été observé en RMN ¹H que pour un isomère, le proton aldéhydique était couplé avec les atomes de fluor, formant un quadruplet de constante de couplage de ⁵J_{HF} = 1,5 à 2 Hz, et pour l'autre isomère, la constante de couplage est nulle.

Sur l'isomère ne présentant pas de constante de couplage ⁵J_{HF} l'irradiation du proton vinylique entraîne la disparition du doublet correspondant au proton aldéhydique. Il s'agit de l'isomère Z.

Par contre, pour l'autre isomère, l'irradiation du proton vinylique entraîne la disparition de la constante de couplage avec le proton aldéhydique et montre un doublet très fin d'une constante de 1,5 à 2 Hz. Les atomes de fluor sont donc spatialement proches du proton aldéhydique. Il s'agit donc de l'isomère E.

Un composé de formule (1) est très facilement mis en oeuvre, il est stable à une température comprise entre -100°C et 80°C, et il peut être utilisé dans tous les solvants, sans conditions particulières. Le groupement énol-éther protège la fonction carbonyle adjacente à la chaîne perfluoroalkylée et évite la formation d'hydrates ou de cétals qui bloqueraient la réactivité de ces composés.

Un deuxième objet de la présente invention consiste en un procédé de préparation des composés de formule (1).

Pour les composés (1) dans lesquels Z représente un atome d'hydrogène, désignés ci-après par (1a), le procédé selon l'invention consiste à faire réagir un composé de formule (2) 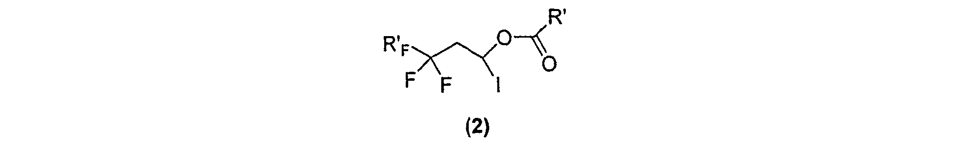 dans laquelle R'_{F} est tel que précédemment défini et R' est un alkyle en C₁ à C₄ ou un aryle (de préférence un méthyle), avec un composé de formule (3) ci-dessous 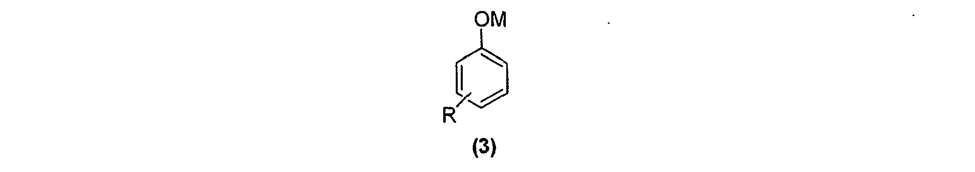 dans laquelle R est tel que précédemment défini et M représente un métal alcalin, de préférence Na, dans les conditions suivantes :
- le rapport molaire entre le composé de formule (3) et le composé de formule (2) est compris entre 3 et 3,5, de préférence égal à 3 ;
- la température peut varier entre 0°C et 70°C ;
- le solvant utilisé est un alcane, ou un mélange d'alcanes liquides ayant une température d'ébullition inférieure à 100°C, par exemple le n-heptane anhydre, ou un hydrocarbure aromatique, ou un éther de pétrole.

On préfère utiliser un solvant comprenant un ou plusieurs alcanes.

Lorsque R qui est un groupe électrodonneur, la température est de préférence de l'ordre de 20°C. Lorsque R est un groupe électroattracteur, la température est de préférence de l'ordre de 70°C.

Ce procédé correspond au schéma réactionnel 1 qui suit.

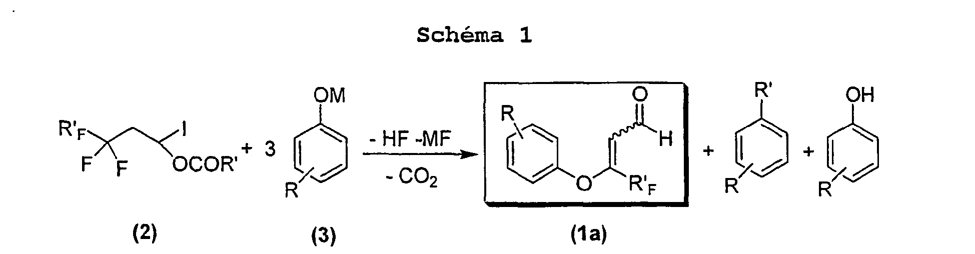

Le composé (2) est préparé par réaction d'un composé de formule R'_{F}-CF₂I, dans laquelle R'_{F} est CₙF₂ₙ₊₁₋ tel que défini ci-dessus, avec un composé CH₂=CH-O-CO-R' dans lequel R' est tel que défini ci-dessus, en présence d'un initiateur radicalaire, par exemple AIBN ou BEt₃.

Le composé R'_{F}-CF₂I est synthétisé à partir de produits commerciaux. Lorsque n est pair, le composé R'_{F}-CF₂I est préparé par télomérisation de CF₃I avec le tétrafluoroéthylène. Lorsque n est impair, le composé R'_{F}-CF₂I est préparé par télomérisation de tétrafluoroéthylène avec IF₅. Les produits cités précédemment sont disponibles notamment chez Dupont de Nemours, et leurs réactivités sont équivalentes.

Pour une description plus détaillée de la synthèse des composés de formule (2), on pourra se reporter aux articles suivants : Ph. Laurent, H. Blancou, A. Commeyras, Tetrahedron lett. 33, 1992, 2489; N.O. Brace, J. Org. Chem, 27, 1962, 3033 et Ph. Laurent, H. Blancou, A. Commeyras, J. Fluorine Chem., 62, 1993, 161 ; M. Napoli, C. Fraccard, L. Conte, G.P. Gambaretto, E. Legnaro, J. Fluorine Chem. 57, 1992, 219.

Les composés de formule (3) sont disponibles commercialement.

Pour les composés (1) dans lesquels Z représente un atome d'halogène, désignés ci-après par (1b), le procédé de préparation consiste à faire subir une halogénation à un composé (1a) obtenu selon le schéma réactionnel (1) ci-dessus, dans le tétrachlorure de carbone et sous rayonnement ultraviolet.

Ce procédé correspond au schéma réactionnel 2 qui suit, dans lequel Z est représenté par Br.

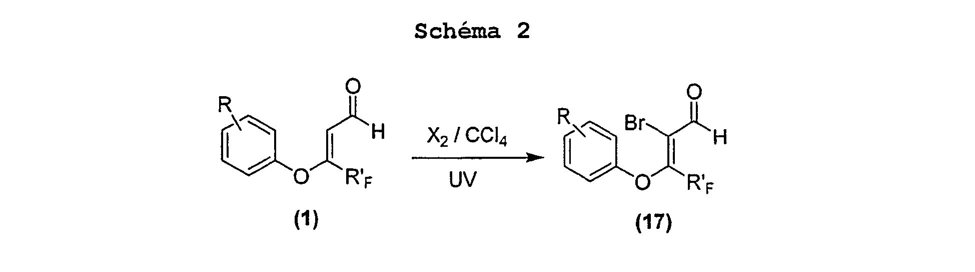

Dans le cadre de la présente invention :
- "groupe aryle" signifie de préférence un système mono- ou polycyclique possédant un ou plusieurs noyaux aromatiques parmi lesquels on peut citer le groupe phényle, le groupe naphtyle, le groupe tétrahydronaphtyle, le groupe indanyle et le groupe binaphtyle. Le groupe aryle peut porter 1 à 3 substituants choisis indépendamment les uns des autres. A titre d'exemple de substituant, on peut citer un groupe hydroxyle, un groupe alkyle linéaire ou ramifié comportant de 1 à 4 atomes de carbone tel que le méthyle, l'éthyle, le propyle ou de préférence le tert-butyle, un groupe nitro, un groupe alcoxy comportant de 1 à 4 atomes de carbone, et un atome d'halogène, tel que le chlore, le brome ou l'iode ;
- "groupe hétérocyclique aromatique" ou "groupe hétéroaryle" signifie un cycle à 5 ou 6 chaînons contenant de 1 à 2 hétéroatomes choisis parmi un atome d'oxygène, un atome d'azote et un atome de soufre, parmi lesquels on peut citer les groupes pyrrolyle, pyrazolyle, imidazolyle, pyridyle, pyrazinyle, pyrimidinyle, pyridazinyle, oxazolyle, isoxazolyle, thiazolyle, isothiazolyle, furyle et thiényle. Le groupe hétérocyclique peut porter un substituant. A titre d'exemple de substituant, on peut citer un groupe hydroxyle, un groupe alkyle linéaire ou ramifié comportant de 1 à 4 atomes de carbone tel que le méthyle, l'éthyle, le propyle ou de préférence le tert-butyle, un groupe nitro, un groupe alcoxy comportant de 1 à 4 atomes de carbone, et un atome d'halogène, tel que le chlore, le brome ou l'iode ;
- Un « groupe aryloxy » signifie un groupe aryle tel que précédemment défini lié à un atome d'oxygène divalent ;
- Un atome d'halogène signifie un atome de chlore, de fluor, de brome ou d'iode. D'une manière générale on préfère l'atome de chlore et de fluor ;
- un groupe alkylène est un groupe divalent dérivé d'un groupe alkyle par élimination d'un atome d'hydrogène ;
- un groupe arylène est un groupe divalent dérivé d'un groupe aryle par élimination d'un atome d'hydrogène ;
- un groupe hétéroarylène est un groupe divalent dérivé d'un groupe hétéroaryle par élimination d'un atome d'hydrogène.

Les composés (1) de la présente invention sont utiles pour la préparation d'un grand nombre de composés hétérocycliques comprenant au moins un groupement perfluoro-alkyle. C'est pourquoi un autre objet de la présente invention est un procédé de préparation de composés hétérocycliques comprenant au moins un groupement perfluoro-alkyle, à partir des composés (1).

En effet, un composé de formule (1) peut réagir de différentes façons :
- La fonction aldéhyde en position β par rapport à la chaîne perfluoro-alkylée est très réactive et réagit facilement avec toutes sortes de composés nucléophiles ;
- Le groupement phénoxy hydrolysable en milieu acide concentré, présente un caractère de groupement partant et réagit facilement avec les composés nucléophiles.

Par exemple, un composé de formule (1) selon la présente invention réagit aisément pour donner les divers hétérocycles perfluoro-alkylés à cinq et à six chaînons suivants : chroménols (par exemple le 2-chroménol et ses dérivés), pyridines (par exemple les 3-acétyl-pyridines et leurs dérivés), pyrimidines, pyrimidones et leurs dérivés, quinoléines, isoxazoles, pyrazoles, quinolones, benzodiazépines, ainsi que les exemples ci-après l'illustrent.

Ces hétérocycles se caractérisent par le fait qu'ils comportent un groupement perfluoro-alkyle greffé régiospécifiquement en position α par rapport à l'hétéroatome. La régiosélectivité de la réaction constitue un autre avantage du procédé selon la présente invention.

La plupart de ces hétérocycles sont eux-mêmes connus, soit comme présentant des propriétés biologiques, soit comme intermédiaires dans la synthèse de molécules bioactives trouvant leurs applications dans les domaines pharmaceutiques et phytosanitaires.

Le schéma 3 ci-après illustre différentes voies de synthèse dans lesquelles les composés (1a) peuvent être mis en oeuvre.

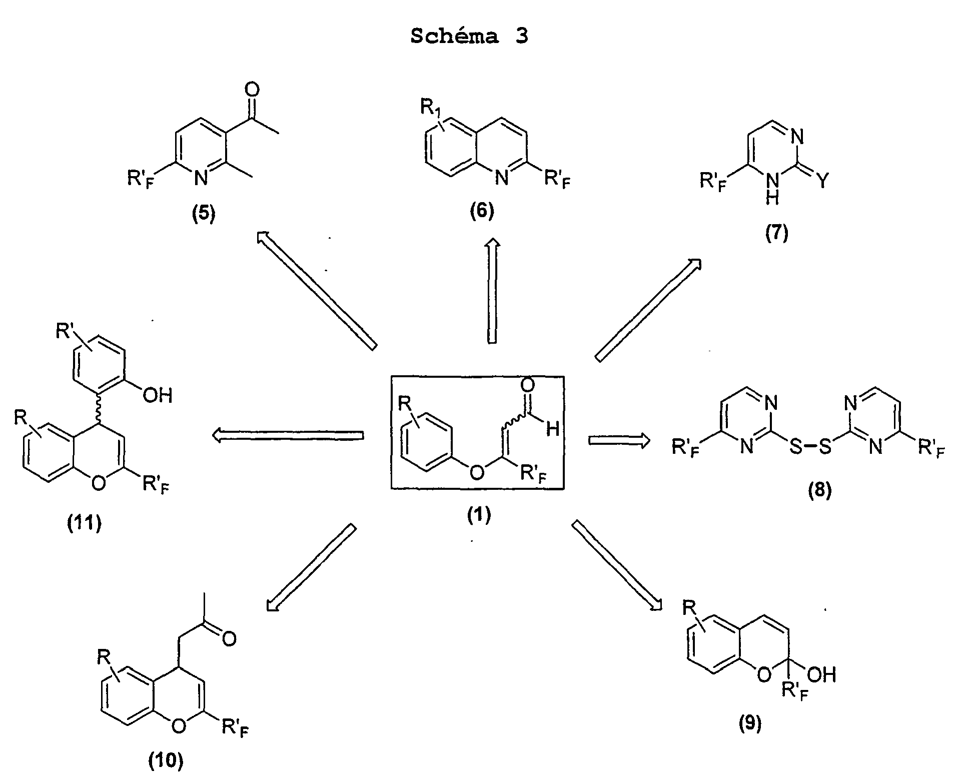

Dans ce schéma R'_{F} et R sont tels que précédemment définis et Y représente un atome d'oxygène ou un atome de soufre.

Le schéma 4 illustre la synthèse de dérivés des 2-perfluoroalkylpyridines, de formule (5).

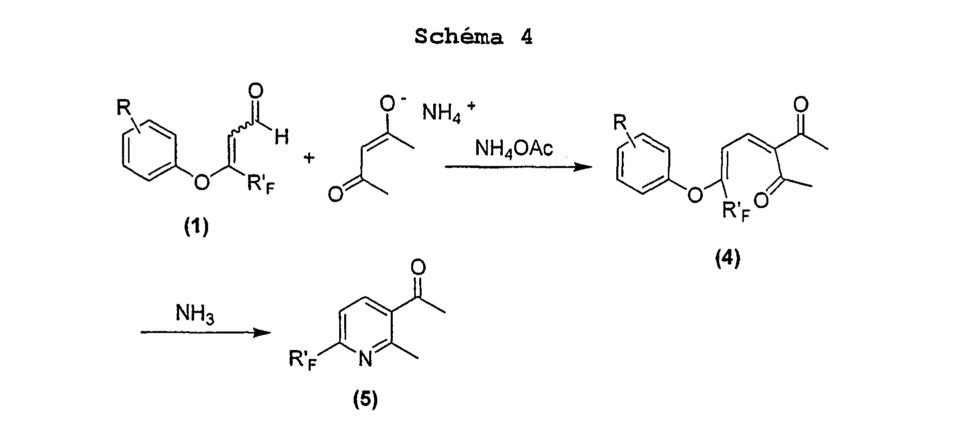

On fait réagir un composé (1) avec l'acétylacétone (4'). Un autre composé béta-difonctionnel possédant un centre activé, notamment un composé béta-dicarbonylé, et par exemple un béta-cétoester peut être utilisé. La réaction a lieu dans l'éthanol, en présence d'acétate d'ammonium à température ambiante, pour obtenir le composé (4). On fait ensuite réagir ce composé (4) avec de l'ammoniac dans de l'éthanol pour obtenir le composé (5).

Cette voie de synthèse est illustrée par l'exemple 2-1.

Le schéma 5 illustre la synthèse d'une 2-perfluoroalkylquinoléine de formule (6).

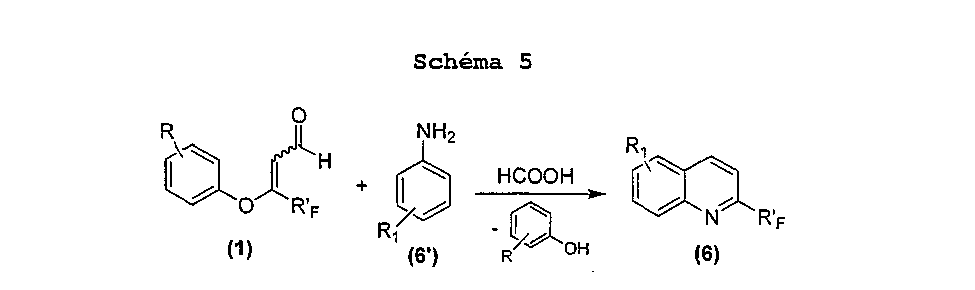

On fait réagir le composé (1) avec l'aniline de formule (6') dans laquelle R₁ est choisi parmi les substituants définis ci-dessus pour R. De préférence, R₁ représente un atome d'hydrogène, un groupe alkyle comportant de 1 à 6 atomes de carbone, un groupe aryle, un groupe hétéroaryle, un groupe alcoxy comportant de 1 à 6 atomes de carbone, un groupe aryloxy, un groupe hydroxyle, un groupe nitro, ou un atome d'halogène, pour obtenir le composé (6).

La réaction a lieu dans le tétrahydrofuranne (THF) ou le dioxanne en présence d'acide formique, à une température comprise entre 60°C et 65°C (préférentiellement à 60°C)

Cette voie de synthèse est illustrée par les exemples 3-1 et 3-2.

Le schéma 6 illustre la synthèse de 2-oxo et 2-thio-6-perfluoroalkylpyrimidines de formule (7).

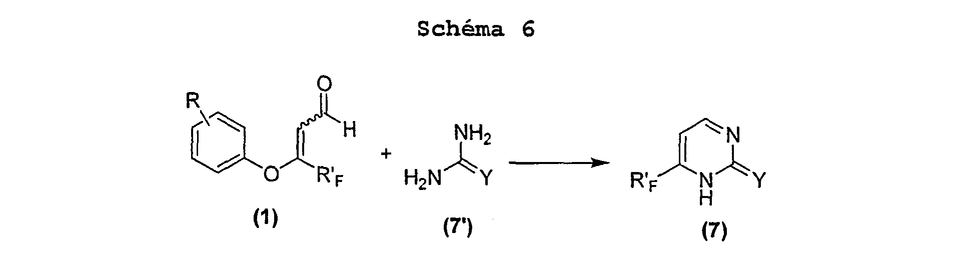

On fait réagir le composé (1) avec un composé de formule (7'), dans laquelle Y représente un atome d'oxygène ou un atome de soufre, pour obtenir le composé (7). La réaction a lieu dans de l'éthanol en présence d'acide sulfurique ou d'acide chlorhydrique de préférence à 1%, à une température comprise entre 78°C et 85°C (préférentiellement à 78°C).

Cette voie de synthèse est illustrée par les exemples 4-1 à 4-4.

Le composé (7), dans lequel Y représente un atome de soufre, dimérise facilement à l'air libre pour donner un composé de formule (8) ci-dessous.

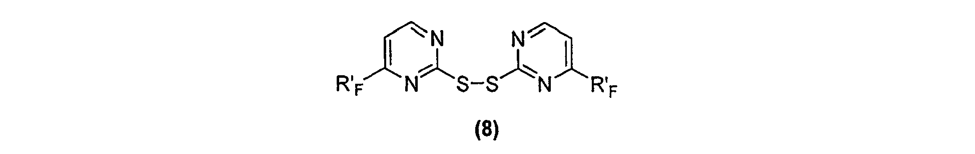

Le schéma 7 illustre la synthèse d'un 2-perfluoroalkylchrom-3-ène-2-ol de formule (9).

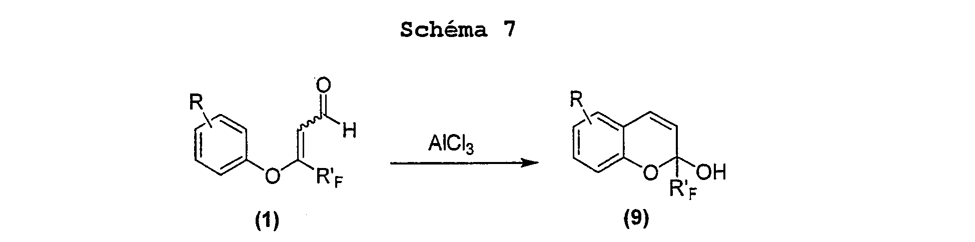

On fait réagir le composé (1) avec un acide de Lewis, préférentiellement du chlorure d'aluminium dans un solvant chloré (par exemple du 1,2-dichloroéthane) à une température comprise entre 85°C et 90°C (préférentiellement à 85°C), pour obtenir le composé (9).

Cette voie de synthèse est illustrée par l'exemple 5-1.

Le schéma 8 illustre la réactivité d'un 2-perfluoro-alkylchromène-2-ol (composé (9)) selon la réaction de Mukayama pour l'obtention du composé de formule (10).

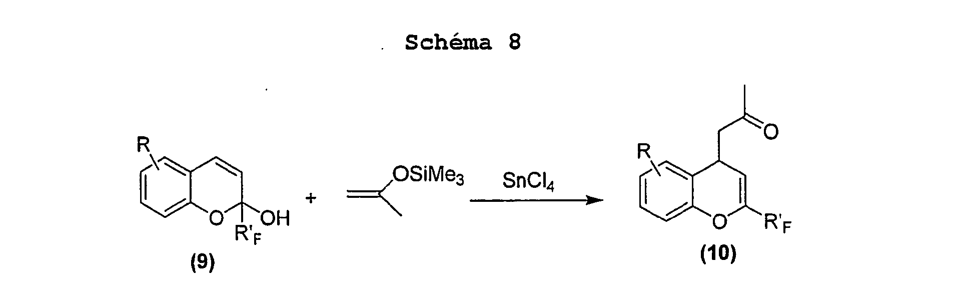

On fait réagir le composé (9) avec du 2-(triméthyl-silyloxy)-propène dans un solvant alcane ou un mélange d'alcanes, (par exemple de l'éther de pétrole), en présence de SnCl₄ à une température comprise entre -30°C et -20°C, (préférentiellement à -20°C), pour obtenir le composé (10).

D'autres réactifs de Mukayama peuvent être utilisés, par exemple le 1-triméthylsilyloxycyclohexène ou le triméthylsilyloxycyclopentène.

Cette voie de synthèse est illustrée par l'exemple 6-1.

Les chromènes de formule (10), qui sont fonctionnels en position 4, sont nouveaux et font partie de l'invention, ainsi que leur procédé de préparation par la réaction de Mukayama. Ces chromènes présentent des propriétés thermochromes.

Le schéma 9 illustre la réaction du 2-perfluoroalkylchromèn-2-ol avec le 4-méthoxyphénol pour obtenir un composé de formule (11).

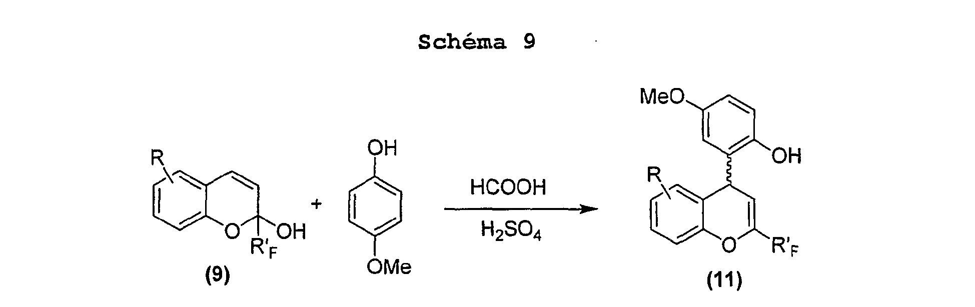

On fait réagir le composé (9) avec un composé aromatique hydroxylé, par exemple le 4-méthoxyphénol. La réaction a lieu dans du tétrahydrofuranne en présence d'acide formique à une température comprise entre 0°C et 20°C (préférentiellement à 0°C), pour obtenir le composé (11).

Cette voie de synthèse est illustrée par les exemples 7-1 et 7-2.

Les composés (11) sont nouveaux et font partie de l'invention. Ces chromènes présentent des propriétés thermochromes : ils se colorent en jaune à - 10°C et en vert à +20°C.

Les schémas 10 et 11 ci-après illustrent d'autres voies de synthèse dans lesquelles les composés de formule (1a) peuvent être mis en oeuvre.

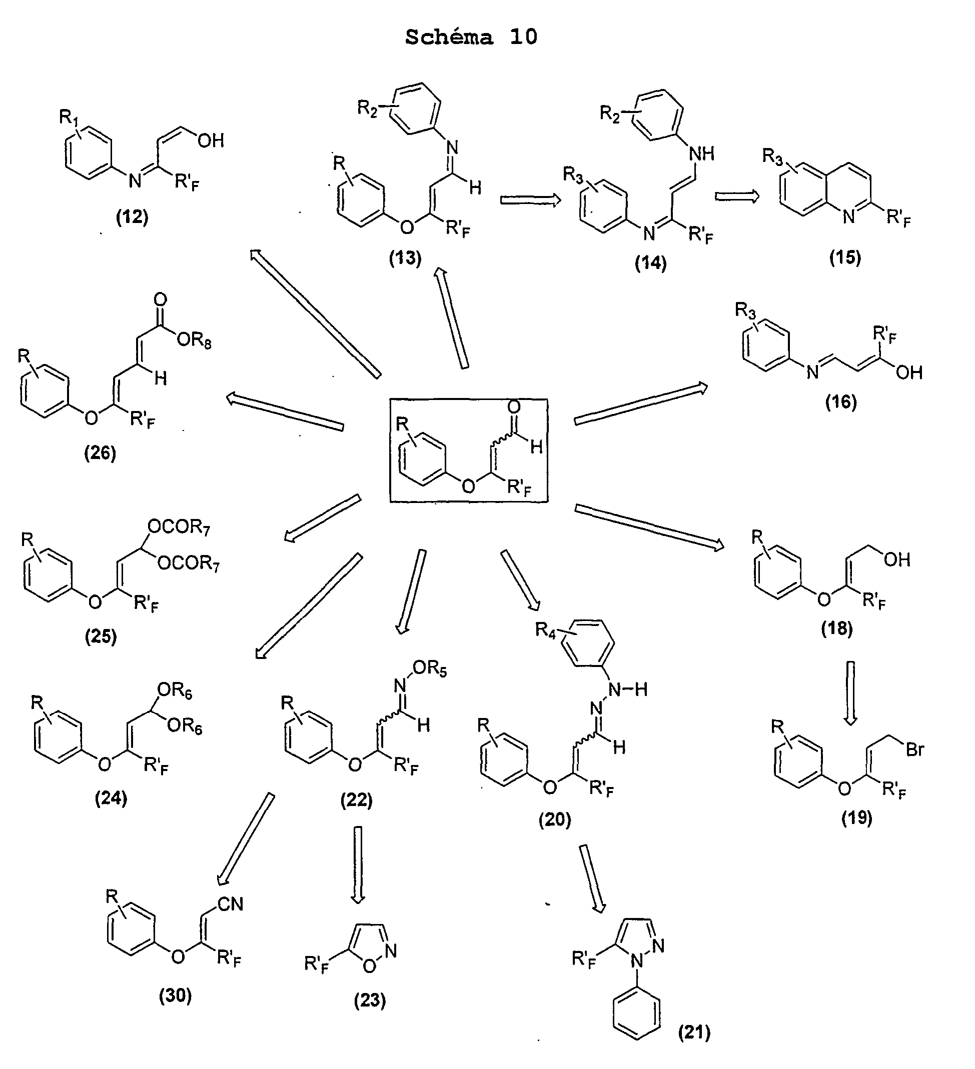

Les substituants R₁ à R₈ apparaissant dans les divers composés du schéma 10 sont choisis indépendamment les uns des autres parmi les substituants définis ci-dessus pour R. De préférence, les substituants R₁ à R₈ sont choisis parmi R₁ représente un atome d'hydrogène, un groupe alkyle comportant de 1 à 6 atomes de carbone, un groupe aryle, un groupe hétéroaryle, un groupe alcoxy comportant de 1 à 6 atomes de carbone, un groupe aryloxy, un groupe hydroxyle, un groupe nitro, ou un atome d'halogène.

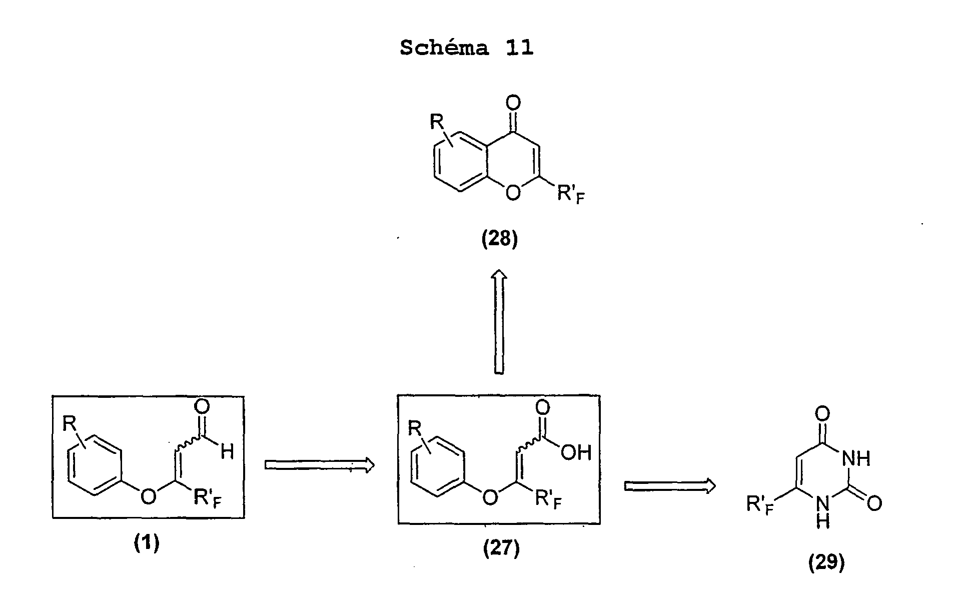

Le schéma 12 illustre la synthèse des composés de formule (12).

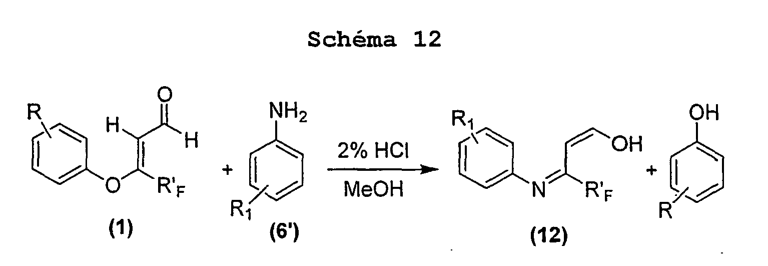

On fait réagir le composé (1) avec l'aniline de formule (6') telle que précédemment définie, pour obtenir le composé (12) La réaction a lieu dans du méthanol, en présence d'acide chlorhydrique.

Cette voie de synthèse est illustrée par les exemples 8-1 à 8-4.

Le schéma 13 illustre la synthèse des composés de formule (13).

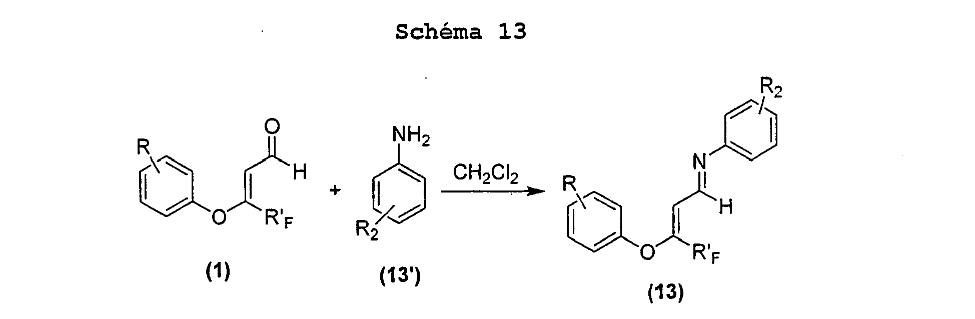

On fait réagir le composé (1) avec l'aniline (13'), dans du dichlorométhane, pour obtenir le composé (13).

Cette voie de synthèse est illustrée par les exemples 9-1 et 9-2.

Les composés (13) peuvent eux-mêmes réagir avec l'aniline de formule générale (14'), dans du dichlorométhane, pour former les diazapentadiènes dissymétriques (14), selon le schéma (14).

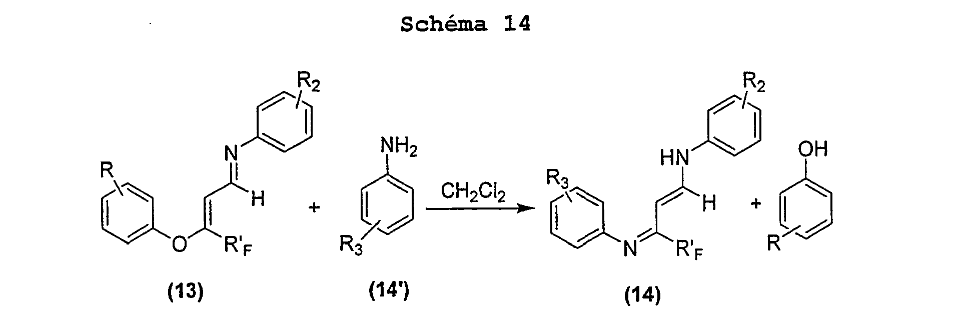

Cette voie de synthèse est illustrée par les exemples 10-1 et 10-2.

Les composés (13) peuvent également réagir avec l'aniline (14') dans du dichlorométhane à reflux, pour former les composés (15), selon le schéma (15).

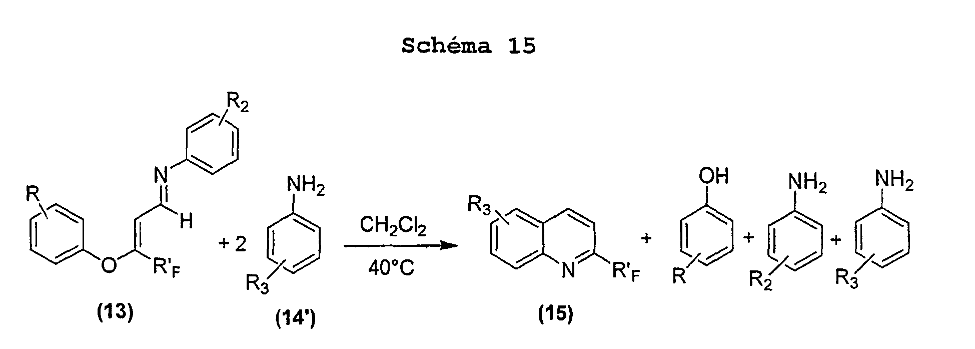

Cette voie de synthèse est illustrée par les exemples 11-1 à 11-8.

Le schéma 16 illustre la synthèse des composés de formule (16).

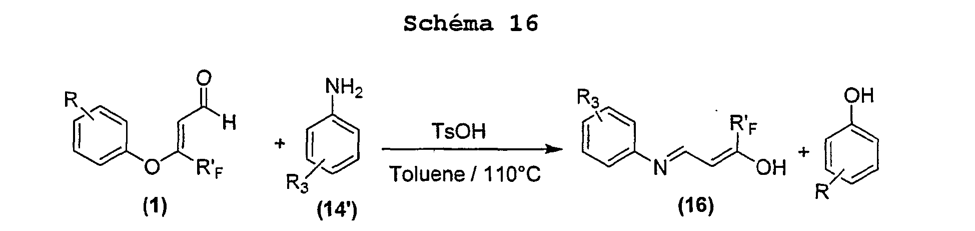

On fait réagir le composé (1) avec l'aniline (14'), en présence d'acide para-toluènesulfonique, pour former l'imino-énol (16). Cette voie de synthèse est illustrée par l'exemple 12-1.

Le schéma 17 illustre la synthèse des composés (18).

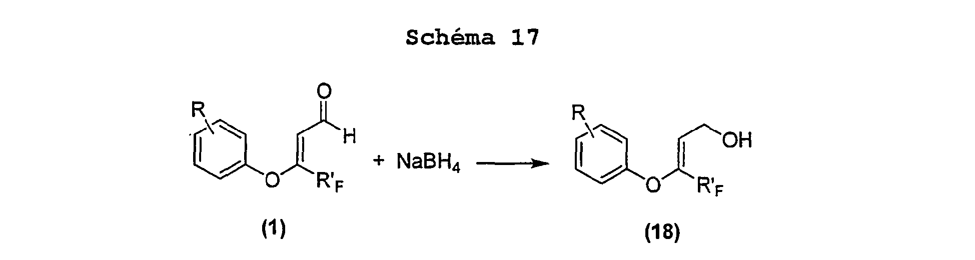

On fait réagir le composé (1) avec du borohydrure de sodium, pour former le composé (18).

Cette voie de synthèse est illustrée par les exemples 13-1 à 13-4.

Le schéma 18 illustre la synthèse des composés (19) à partir des composés (18).

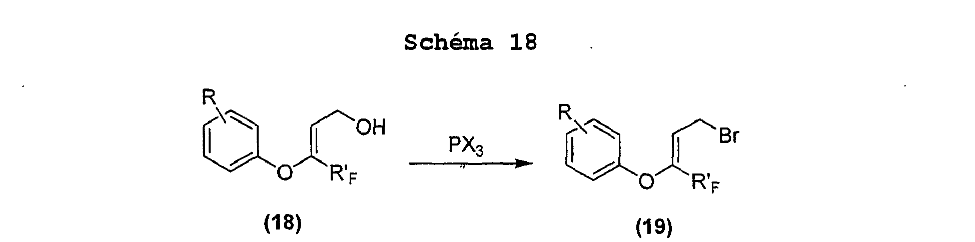

Le composé (18) est mis à réagir avec PX₃ dans lequel X représente un atome d'halogène, en particulier Br ou Cl, de sorte à former les composés (19).

Cette voie de synthèse est illustrée par les exemples 14-1 et 14-2.

Le schéma 19 illustre la synthèse des composés de formule générale (20).

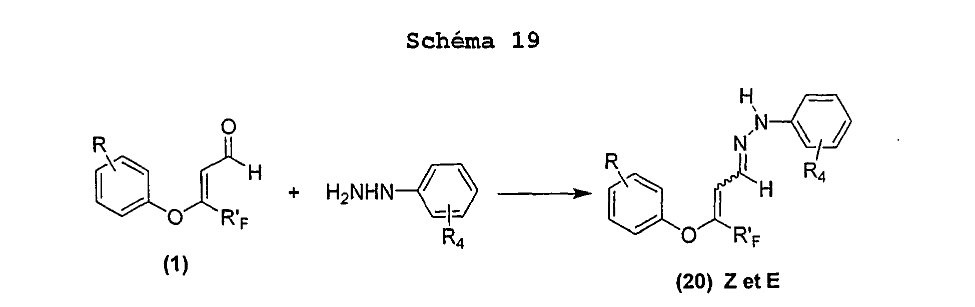

On fait réagir le composé (1) avec une phénylhydrazine, de sorte à former le composé (20).

Cette voie de synthèse est illustrée par l'exemple 15-1.

Le schéma 20 illustre la synthèse des composés de formule générale (21) à partir des composés (20).

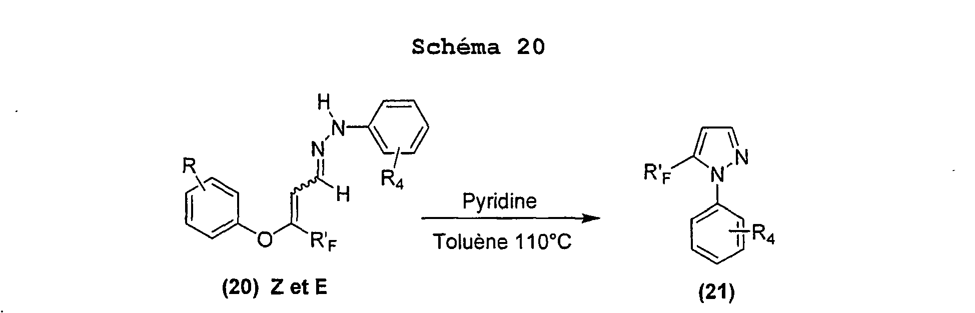

Le composé (20) est cyclisé en présence de pyridine dans le toluène à reflux, pour former le composé (21).

Cette voie de synthèse est illustrée par l'exemple 16-1.

Le schéma 21 illustre la synthèse des composés de formule générale (22).

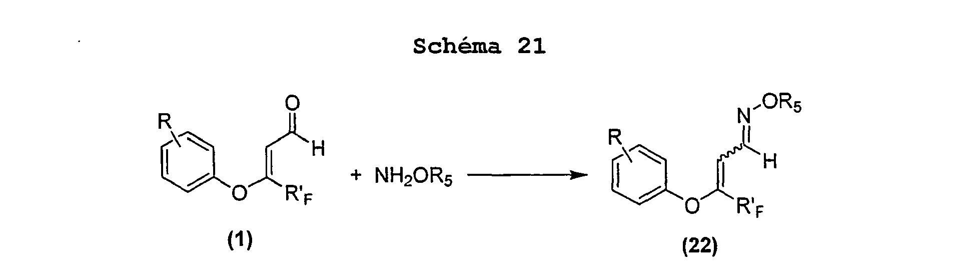

On fait réagir le composé (1) avec un composé de formule générale NH₂OR⁵.

Cette voie de synthèse est illustrée par l'exemple 17-1.

Le schéma 22 illustre la synthèse des composés de formule générale (23) à partir d'un composé (22) dans lequel R₅ est H, désigné ci-après par (22').

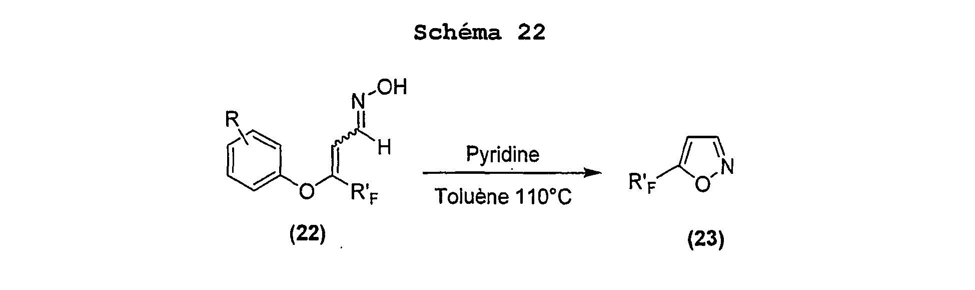

Un composé de formule générale (22') est cyclisé en présence de pyridine dans le toluène à reflux, pour former le composé (23).

Cette voie de synthèse est illustrée par l'exemple 18-1.

Le schéma 23 illustre la synthèse des composés de formule générale (24).

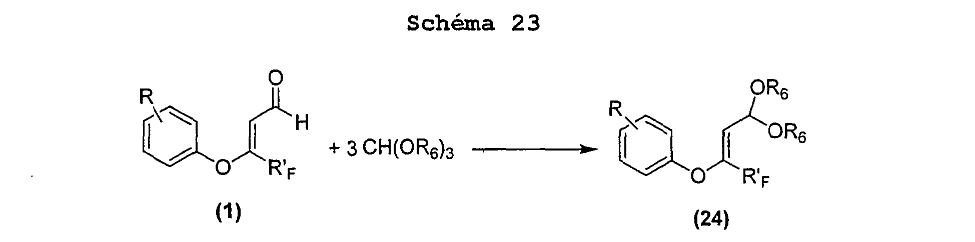

On fait réagir le composé (1) avec un composé de formule générale CH(OR⁶)₃, pour former le composé de formule générale (24).

Cette voie de synthèse est illustrée par l'exemple 19-1.

Le schéma 24 illustre la synthèse des composés de formule générale (25).

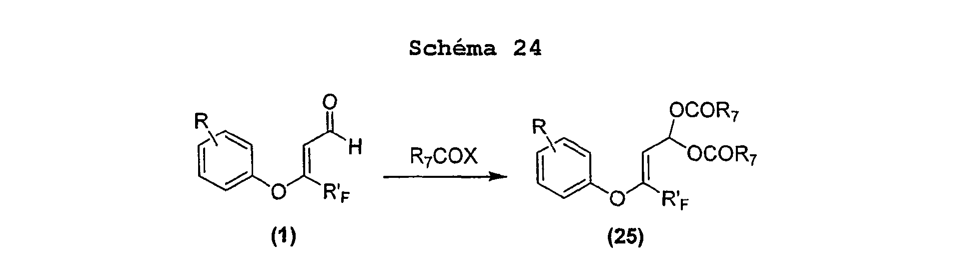

On fait réagir le composé (1) avec un composé de formule générale (R₇COX), et X représente un atome d'halogène, en particulier Cl ou Br, pour former le composé (25).

Cette voie de synthèse est illustrée par l'exemple 20-1.

Le schéma 25 illustre la synthèse des composés de formule générale (26).

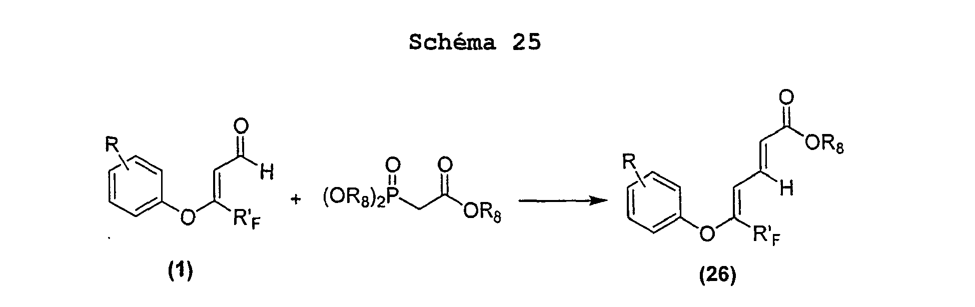

On fait réagir le composé (1) avec un composé de type phosphonoacétate dans lequel R₈ représente de préférence un groupe alkyle ou aryle, pour former le composé de formule générale (26).

Cette voie de synthèse est illustrée par l'exemple 21-1.

Le schéma 26 illustre la synthèse des composés de formule générale (27).

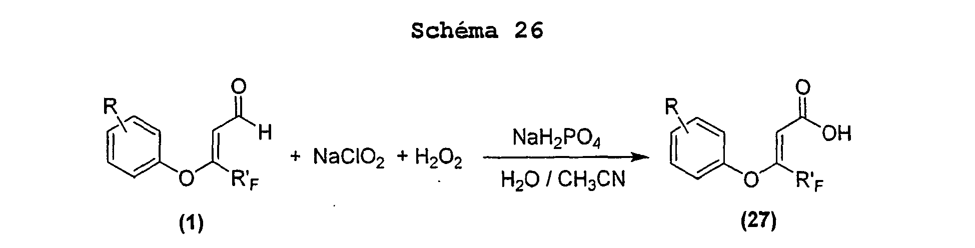

On fait réagir le composé (1) avec du chlorite de sodium et du peroxyde d'hydrogène, pour former le composé (27).

Cette voie de synthèse est illustrée par l'exemple 22-1.

Le schéma 27 illustre la synthèse des composés de formule générale (28) à partir des composés de formule générale (27).

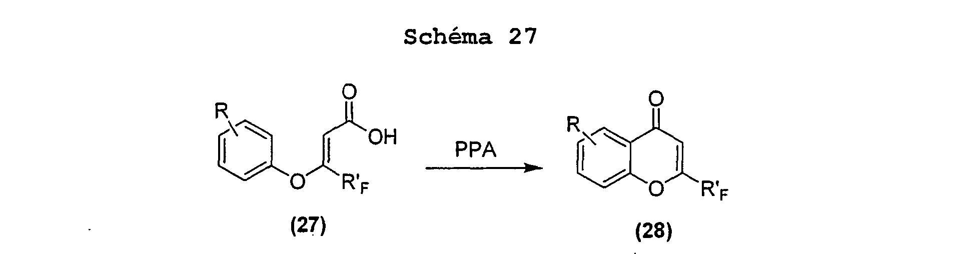

On cyclise le composé (27), en présence d'acide polyphosphorique, pour former le composé de formule générale (28).

Cette voie de synthèse est illustrée par l'exemple 23-1.

Le schéma 28 illustre la synthèse des composés de formule générale (29) à partir des composés de formule générale (27).

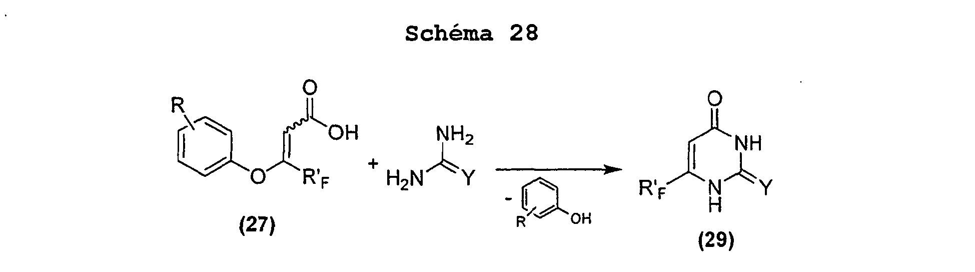

On fait réagir le composé (27) avec l'urée (Y est O), ou la thiourée (Y est S), pour former le composé (29).

Cette voie de synthèse est illustrée par l'exemple 24-1.

Le schéma 29 illustre la synthèse des composés de formule générale (30) à partir des composés de formule générale (22).

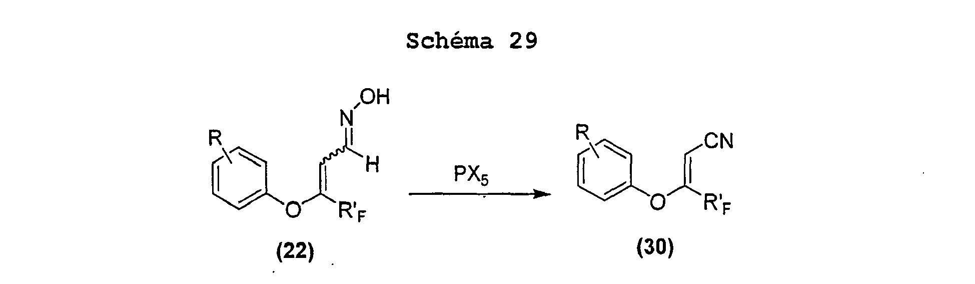

On fait réagir le composé (22) avec un composé de formule générale (PX₅) dans laquelle X représente un atome d'halogène, en particulier Cl ou Br, pour former le composé (30).

Cette voie de synthèse est illustrée par l'exemple 25-1.

Les exemples suivants illustrent le procédé de préparation des composés de l'invention. Les analyses RMN confirment la structure des composés obtenus.

Plus précisément, les exemples **1-1 à 1-10** illustrent la synthèse des composés (1) de l'invention et les exemples **2-1 à 25-1** illustrent des synthèses d'hétérocycles à groupements fluorés greffés régiosélectivement à partir des composés (1) de l'invention.

### Exemple 1-1

### Réaction du 1-acétoxy-1-iodo-perfluorohexyléthane (2) (R'_{F} = C₅F₁₁) avec le 4-méthoxyphénolate de sodium

A une solution contenant 12 g (2,25 10⁻² mole) d'1-acétoxy-1-iodo-perfluorohexyléthane (2) (R'_{F} = C₅F₁₁) dans le n-heptane anhydre (ou de l'éther de pétrole) (25 ml), on ajoute 9,88 g (6,76 10⁻² mole) de 4-méthoxyphénolate de sodium. Le mélange est additionné de 25 ml de n-heptane, puis mis sous agitation magnétique vigoureuse à température ambiante (20°C). L'évolution de la réaction est suivie par chromatographie sur couche mince avec un mélange éluant : éther de pétrole 100/ acétate d'éthyle 12 ; V/V. Après douze heures d'agitation, un précipité brun clair se dépose. Le mélange réactionnel est filtré et le précipité est lavé cinq fois à l'heptane. Les phases organiques sont rassemblées puis concentrées sous pression réduite (20 mmHg).

Le mélange est séparé sur colonne de 5 cm de silice (Si 60, 40-63 µm, Merck®) par élution à l'aide d'un mélange acétate d'éthyle 5/ éther de pétrole 100 ; V/V. Le produit final (1) (R'_{F} = C₅F₁₁) cristallise dans le n-heptane à environ 0°C au bout de 10 heures et est obtenu avec un rendement de 85%.

Le produit final (1) obtenu est chromatographié sur plaque préparative de silice de 2 mm d'épaisseur avec un mélange éluant : acétate d'éthyle 12/ éther de pétrole 100 ; V/V, et montre un seul produit possédant un RF de 0,55.

### Caractéristiques:

RMN ¹H (250 MHZ, CDCl₃): δ 3,8 (s, 3H) ; 5,6 (d, J=7, 3 Hz, 1H) ; 6, 95 (s, 4H) ; 9, 9 (d, J = 7, 3 Hz, 1H).
RMN ¹⁹F (250 MHZ, CDCl₃): δ -126,5 (s, 2F) ; -123 (s, 4F) ; - 112 (s, 2F) ; -81,5 (s, 3F).
MS (FAB⁺, GT): [M+H⁺]⁺ = 447.
Spectroscopie de masse haute résolution : masse calculée = 446,0376 ; masse obtenue = 446,0366

### Exemple 1-2

### Réaction du 1-acétoxy-1-iodo-perfluorohexyléthane (2) (R'_{F} = C₅F₁₁) avec le phénolate de sodium

On procède selon le même mode opératoire que précédemment, en utilisant 10 g (1,87 10⁻² moles) du composé gemiodoacétylé (2) et 6,54 g (5,63 10⁻²mole) de phénolate de sodium dans 20 ml d'éther de pétrole.

La chromatographie est effectuée sur colonne de 5cm de silice (Si 60, 40-63 µm, Merck®) avec le même éluant que précédemment. Le rendement obtenu est de 70%.

### Caractéristiques :

RMN ¹H (250 MHZ, CDCl₃) : δ 5,7 (d, J = 7,09Hz, 1H) ; 7,5 (m, 5H), 9,95 (d, J = 7, 04Hz, 1H).
RMN ¹³C (400 MHz, CDCl₃) : δ 114,9 (s, 1C) ; 121,2 (s, 1C) ; 127,6 (s, 1C) ; 131,1 (s, 1C) ; 152,2 (s, 1C) ; 159,4 (t, 1C) ; 188,6 (t, 1C J=8.95Hz).
RMN ¹⁹F (250 MHz, CDCl₃) : δ -126,5 (t, 2F); -122 (t, 4F); -111,5 (t, 2F); -80,5 (t, 3F).
MS (FAB⁺, GT): [M+H⁺]⁺ = 417.

### Exemple 1-3

### Réaction du 1-acétoxy-1-iodo-perfluorobutyléthane (2) (R'_{F} = C₃F₇) avec le 4-méthoxyphénolate de sodium

On procède selon le même mode opératoire que dans l'exemple 1-1, en utilisant 75 g (0,173mole) du composé gemiodoacétylé (2), 76 g (0,520mole) de 4-méthoxyphénolate de sodium dans 120 ml d'éther de pétrole.

La chromatographie est effectuée sur colonne de 35cm de silice (Si 60, 40-63 µm, Merck®) avec le même éluant que précédemment et le rendement obtenu est de 80%.

### Caractéristiques:

RMN ¹H (250 MHz, CDCl₃) : δ 3,7 (s, 3H) ; 5,8 (d, J = 7Hz, 1H) ; 7,1 (s, 4H) ; 10,1 (d, J = 7 Hz, 1H).
RMN ¹⁹F (250 MHz, CDCl₃): δ -126,6 (s, 2F) ; -124,2 (s, 2F) ; -79,8 (s, 3F).
MS (FAB⁺, GT) : [M+H⁺]⁺ = 347.
Spectroscopie de masse haute résolution : masse calculée = 346,0440 ; masse obtenue = 346,0431.

### Exemple 1-4

### Réaction du 1-acétoxy-1-iodo-perfluorobutyléthane (2) (R'_{F} = C₃F₇) avec le phénolate de sodium

On procède selon le même mode opératoire que pour l'exemple 1-1 en utilisant 25 g (0,057 mole) du composé gemiodoacétylé (2), 20,13 g (0,173 mole) de phénolate de sodium dans 45 ml d'éther de pétrole.

La chromatographie est effectuée sur colonne de 10 cm de silice (Si 60, 40-63 µm, Merck®) avec le même éluant que précédemment. Le rendement obtenu est de 70%.

### Caractéristiques :

RMN ¹H (250 MHz, CDCl₃): δ 5,5 (d, J = 7,2 Hz, 1H) ; 7,3 (m, 5H) ; 10,1 (d, J = 7,2 Hz, 1H).
RMN ¹⁹F (250 MHz, CDCl₃): δ -127,1 (s, 2F) ; -121,3 (s, 2F) ; -79,6 (s, 3F).
MS (FAB⁺, GT): [M+H⁺]⁺ = 317.

### Exemple 1-5

### Réaction du 1-acétoxy-1-iodo-perfluoroéthyléthane (2) (R'_{F} = CF₃) avec le 4-méthoxyphénolate de sodium

A une solution contenant 50g (0,150 mole) d'1-acétoxy-1-iodo-perfluorohexyléthane (2) (**R'_{F} = CF₃**) dans le n-heptane anhydre (ou de l'éther de pétrole) (75 ml), on ajoute 65,9g (0,451 mole) de 4-méthoxyphénolate de sodium. Le mélange est additionné de 25 ml de n-heptane, puis mis sous agitation magnétique vigoureuse à température ambiante (20°C). L'évolution de la réaction est suivie par chromatographie sur couche mince avec un mélange éluant : éther de pétrole 100/ acétate d'éthyle 12; V/V. Après douze heures d'agitation, un précipité brun clair se dépose. Le mélange réactionnel est filtré et le précipité est lavé cinq fois à l'heptane. Les phases organiques sont rassemblées puis concentrées sous pression réduite (20 mmHg).

Le mélange est séparé sur colonne de 20cm de silice (Si 60, 40-63 µm, Merck®) par élution à l'aide d'un mélange acétate d'éthyle 5/ éther de pétrole 100 ; V/V. Le produit final (1) **(R'_{F} = CF₃)** est obtenu sous forme de deux isomères (cis-trans) avec un rendement de 97%.

Le produit final (1) obtenu est chromatographié sur plaque préparative de silice de 2mm d'épaisseur avec un mélange éluant : acétate d'éthyle 12/ éther de pétrole 100 ; V/V, et montre un seul produit possédant un RF de 0,55.

### Caractéristiques:

RMN ¹H (300.13 MHZ, CDCl₃): δ 3,8 (s, 3H); 5,4 (d, J = 7,49Hz, 1H); 6 (d, J = 7,3Hz, 1H); 6,9 (m, 4H); 9,5 (d, J = 7,3Hz, 1H); 10 (dd, J = 7,5Hz et 3,1Hz, 1H).
RMN ¹⁹F (300.13 MHZ, CDCl₃): δ -64,9 (s, 3F) (60%); -71 (s, 3F) (40%).
RMN ¹³C (300.13 MHZ, CDCl₃): 55,5, 111 (q, CH, ⁴J_{CF} = 0,75Hz); 114,5 (q, CH, ⁴J_{CF} = 3Hz); 115,3; 115,4; 118,5; 119,5 (q, CF₃, ¹J_{CF} = 282,3Hz); 121,6; 145; 150; 154,5 (q, C-CF₃, ²J_{CF} = 35,5Hz); 157,2; 157,6; 160,5 (q, C-CF₃, ¹J_{CF} = 37,7Hz); 187,9 (q, CHO, ⁵J_{CF} = 4,5Hz); 188.
MS (FAB⁺, GT): [M+H⁺]⁺ = 247.
Spectroscopie de masse haute résolution : masse calculée = 246,0504 ; masse obtenue = 246,0502.

### Exemple 1-6

### Réaction du 1-acétoxy-1-iodo-perfluorohexyléthane (2) (R'_{F} = C₅F₁₁) avec le para-nitrophénolate de sodium

On procède selon le même mode opératoire que pour l'exemple 1-5, en utilisant 60g (0,112 moles) du composé gemiodoacétylé (2), et 54,4g (0,338 moles) de para-nitrophénolate de sodium dans 85 ml d'éther de pétrole.

Le mélange des deux isomères est séparé sur colonne de 25cm de silice (Si 60, 40-63 µm, Merck®) par élution à l'aide d'un mélange acétate d'éthyle 15/ éther de pétrole 85 ; V/V. Les produits (1) (R'_{F} = C₅F₁₁, R = p-NO₂) sont obtenus avec un rendement de 60/40 (isomères Z/E respectivement).

Les produits finaux (1) obtenus sont chromatographiés sur plaque préparative de silice de 2mm d'épaisseur avec un mélange éluant : acétate d'éthyle 15/ éther de pétrole 85 ; V/V, et montrent un seul produit pour chaque isomère possédant un RF de 0,6 pour l'isomère Z et 0,75 pour l'isomère E.

Chaque stéréo-isomère a été identifié.

### Caractéristiques :

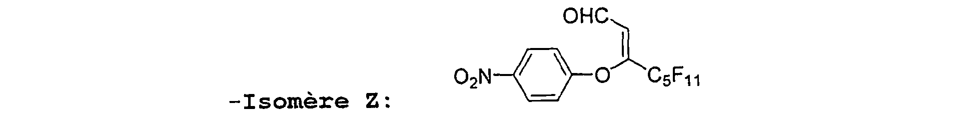

RMN ¹H (250 MHZ, CDCl₃) : δ 6,5 (d, J = 6,8Hz); 7,2 (d, J = 12Hz, 1H); 8,3 (d, J = 12Hz, 1H); 9,9 (d, J = 6,8Hz, 1H). RMN ¹³C (400 MHz, CDCl₃) : δ 115, 6; 122,9 (q, CH, ⁴J_{CF} = 3,7Hz); 126,5; 144,3; 152,6 (q, C-CF₃, ²J_{CF} = 27Hz); 161,4; 186,5.
RMN ¹⁹F (250 MHz, CDCl₃) : δ -126,5 (t, 2F); -123 (t, 2F); -122 (t, 2F); -116 (t, 2F); -81 (t, 3F).
MS (FAB⁺, GT) : [M+H⁺]⁺ = 462.
Spectroscopie de masse haute résolution : masse calculée = 461,0121 ; masse obtenue = 461,0123. 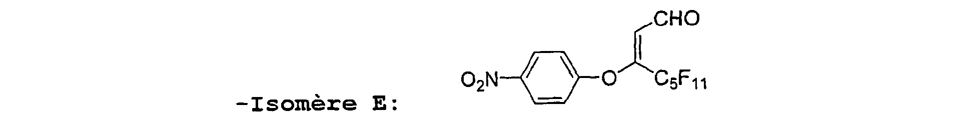 RMN ¹H (250MHZ, CDCl₃) : δ 5,6 (d, J = 6, 9Hz) ; 7,3 (d, J = 12,1Hz, 1H); 8,4 (d, J = 12Hz, 1H); 10 (dd, J = 6,9Hz et 1,5Hz, 1H).
RMN ¹³C (400MHz, CDCl₃) : δ 115,6; 122,9 (q, CH, ⁴J_{CF} = 3,5Hz); 126,8; 144,4; 152,6 (q, C-CF₃, ²J_{CF} = 27,1Hz); 161,2; 185 (q, CHO, ⁵J_{CF} = 3,8Hz).
RMN ¹⁹F (250 MHz, CDCl₃) : δ -126,5 (t, 2F); -123 (t, 4F); -111 (t, 2F); -81 (t, 3F).
MS (FAB⁺, GT): [M+H⁺]⁺ = 4 62.
Spectroscopie de masse haute résolution : masse calculée = 461,0121 ; masse obtenue = 461,0120.

### Exemple 1-7

### Réaction du 1-acétoxy-1-iodo-perfluorohexyléthane (2) (R'_{F} = C₅F₁₁) avec le para-chlorophénolate de sodium

On procède selon le même mode opératoire que pour l'exemple 1-5, en utilisant 55g (0,103 moles) du composé gemiodoacétylé (2) et 46,5g (0,31 moles) de para-chlorophénolate de sodium dans 80 ml d'éther de pétrole.

Le mélange des deux isomères est séparé sur colonne de 25cm de silice (Si 60, 40-63 µm, Merck®) par élution à l'aide d'un mélange acétate d'éthyle 15/ éther de pétrole 85 ; V/V. Les produits (1) (R'_{F} = C₅F₁₁, R = p-Cl) sont obtenus avec un rendement de 68/32 ; Z/E respectivement.

Les produits finaux (1) obtenus sont chromatographiés sur plaque préparative de silice de 2mm d'épaisseur avec un mélange éluant : acétate d'éthyle 15/ éther de pétrole 85 ; V/V, et montrent un seul produit pour chaque isomère possédant un RF de 0,65 pour l'isomère Z et 0,8 pour l'isomère E.

Chaque stéréo-isomère a été identifié.

### Caractéristiques :

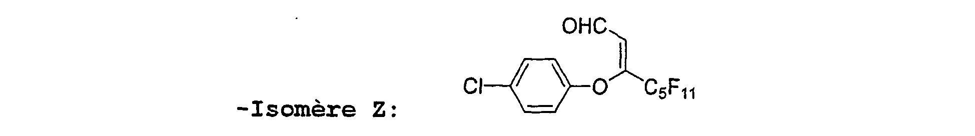

RMN ¹H (250MHZ, CDCl₃) : δ 6,5 (d, J = 7 Hz, 1H); 7,2 (d, J = 12,1Hz, 1H); 8,3 (d, J = 12,2 Hz, 1H); 9,9 .(d, J = 6,9Hz, 1H).
RMN ¹³C (400MHz, CDCl₃) : δ 115, 7; 122,9 (q, CH, ⁴J_{CF} = 3,2 Hz); 126,7; 144,3; 153,8 (q, C-CF₃, ²J_{CF} = 25,6 Hz); 162; 186,2.
RMN ¹⁹F (250 MHz, CDCl₃) : δ -126,5 (t, 2F); -123 (t, 2F); -122,5 (t, 2F); -116 (t, 2F); -81 (t, 3F).
MS (FAB⁺, GT): [M+H⁺]⁺ = 450.
Spectroscopie de masse haute résolution : masse calculée = 449,9881 ; masse obtenue = 449,9901. 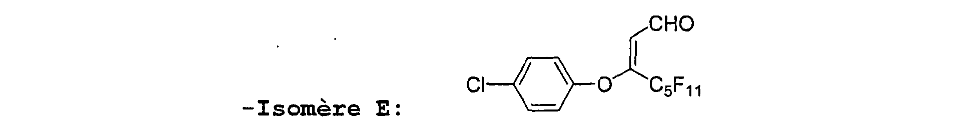 RMN ¹H (250MHZ, CDCl₃) : δ 5,6 (d, J = 7 Hz, 1H); 7,3 (d, J = 12Hz, 1H); 8,4 (d, J = 12Hz, 1H); 10 (dd, J = 7,1 Hz et 1 Hz, 1H).
RMN ¹³C (400MHz, CDCl₃) : δ 115, 6; 122,9 (q, CH, ⁴J_{CF} = 3,6Hz); 127; 144,3; 152,5 (q, C-CF₃, ²J_{CF} = 27 Hz); 161, 2; 185,2 (q, CHO, ⁵J_{CF} = 3,5 Hz).
RMN ¹⁹F (250 MHz, CDCl₃) : δ -126,5 (t, 2F); -123,2 (t, 4F); -111 (t, 2F); -81 (t, 3F).
MS (FAB⁺, GT) : [M+H⁺]⁺ = 450.
Spectroscopie de masse haute résolution : masse calculée = 449,9881 ; masse obtenue = 449,987.

### Exemple 1-8

### Réaction du 1-acétoxy-1-iodo-perfluorohexyléthane (2) (R'_{F} = C₅F₁₁) avec le para-bromophénolate de sodium

On procède selon le même mode opératoire que pour l'exemple 1-5, en utilisant 58g (0,109 moles) du composé gemiodoacétylé (2) et 63,5g (0,33 moles) de para-bromophénolate de sodium dans 70 ml d'éther de pétrole.

Le mélange des deux isomères est séparé sur colonne de 25cm de silice (Si 60, 40-63 µm, Merck®) par élution à l'aide d'un mélange acétate d'éthyle 15/ éther de pétrole 85 ; V/V. Les produits (1) (R'_{F} = C₅F₁₁, R = p-Br) sont obtenus avec un rendement de 62/38 (isomères Z/E respectivement).

Les produits finaux **(1)** obtenus sont chromatographiés sur plaque préparative de silice de 2mm d'épaisseur avec un mélange éluant : acétate d'éthyle 15/ éther de pétrole 85 ; V/V, et montrent un seul produit pour chaque isomère possédant un RF de 0,68 pour l'isomère Z et 0,82 pour l'isomère E.

Chaque stéréo-isomère a été identifié.

### Caractéristiques :

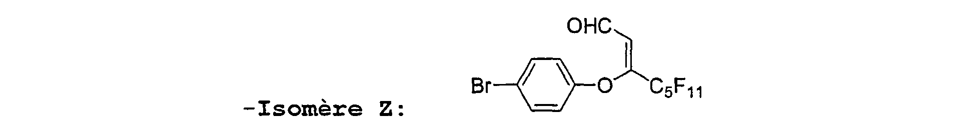

RMN ¹H (250 MHZ, CDCl₃) : δ 6,6 (d, J = 7,2 Hz, 1H); 7 (d, J = 12 )
RMN ¹³C (400 MHz, CDCl₃) : δ 115,5; 123,2 (q, CH, ⁴J_{CF} = 3 Hz); 126,8; 145; 154,1 (q, C-CF₃, ²J_{CF} = 26,1 Hz); 162,2; 186.
RMN ¹⁹F (250 MHz, CDCl₃) : δ -126,5 (t, 2F); -123 (t, 2F); -122,5 (t, 2F); -116 (t, 2F); -81 (t, 3F).
MS (FAB⁺, GT) : [M+H⁺]⁺ = 495.
Spectroscopie de masse haute résolution : masse calculée = 493,9376 ; masse obtenue = 493,9401. 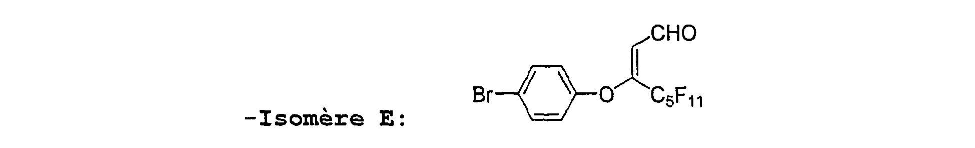 RMN ¹H (250 MHZ, CDCl₃) : δ 5,7 (d, J = 7,3 Hz, 1H); 7,5 (d, J = 12,3 Hz, 1H); 8,4 (d, J = 12,1Hz, 1H); 10,1 (dd, J = 7,2 Hz et 1,5 Hz, 1H).
RMN ¹³C (400 MHz, CDCl₃) : δ 115,6; 123,1 (q, CH, ⁴J_{CF} = 3,2 Hz); 127,2; 144,3; 152,6 (q, C-CF₃, ²J_{CF} = 27,1 Hz) ; 161,4; 186 (q, CHO, ⁵J_{CF} = 3,8 Hz).
RMN ¹⁹F (250 MHz, CDCl₃) : δ -126 (t, 2F); -123 (t, 4F); -111,1 (t, 2F); -81 (t, 3F).
MS (FAB⁺, GT): [M+H⁺]⁺ = 495.
Spectroscopie de masse haute résolution : masse calculée = 493,9376 ; masse obtenue = 493,9402.

### Exemple 1-9

### Réaction du 1-perfluoropentyl-1-phénoxy-propénal (1a) (R'_{F} = C₅F₁₁, R = H) avec le brome.

On dissout 4g (9,61 10⁻³ mole) du composé (1) (R'_{F} = C₅F₁₁, R = H) dans 10 ml de CCl₄. Puis on ajoute 2 g (1,27.10⁻² mole) de brome. Le mélange est mis sous agitation magnétique sous une lampe UV (300 nm) pendant 2 heures. L'évolution de la réaction est suivie par chromatographie sur couche mince avec un mélange éluant : éther de pétrole 70/ acétate d'éthyle 30 ; V/V. En fin de réaction le mélange est lavé avec une solution aqueuse de 1% en masse d'hydogénocarbonate de sodium. Après six heures d'agitation, le mélange réactionnel est additionné d'eau puis extrait cinq fois à l'éther éthylique, les phases organiques sont rassemblées puis concentrées sous pression réduite (20 mmHg, pour obtenir le dérivé bromé (1b) (R'_{F} = C₅F₁₁, R³ = H) avec un rendement de 82%.

Le composé (1b) obtenu est chromatographié sur plaque préparative de silice de 2mm d'épaisseur, avec un mélange éluant : acétate d'éthyle 30/ éther de pétrole 70 ; V/V, pour donner un seul produit sous forme de deux stéréo-isomères (Z-E) non séparables possédant un RF = 0,81.

### Caractéristiques :

RMN ¹⁹F (400.12 MHz, CD₃CN): δ ppm -126,5 (t, 2F), -122 (t, 4F), -115, 5 (t, 2F); -111 (t, 2F); -81 (t, 3F).
RMN ¹H (400,12 MHz, CD₃CN): δ ppm 7,2 (m, 2H); 7,6 (m, 3H); 9,7 (s, 1H); 10,1 (s, 1H).
MS (FAB⁺, GT) : [M+H⁺]⁺ = 494.
Spectroscopie de masse haute résolution : masse calculée = 493,9376 ; masse obtenue = 493,9401.

### Exemple 1-10

### Réaction du 1-acétoxy-1-iodo-perfluoroéthyléthane (2) (R'F = CF3) avec le phénolate de sodium

A une solution contenant 15 g (4,51 10⁻² mole) de 1-acétoxy-1-iodo-perfluoroéthyléthane (2) (R'_{F} = CF₃) dans le n-heptane anhydre (ou de l'éther de pétrole) (20 ml), on ajoute 15,72 g (0,135 mole) de phénolate de sodium. Le mélange est additionné de 15 ml de n-heptane, puis mis sous agitation magnétique vigoureuse à température ambiante (20°C). L'évolution de la réaction est suivie par chromatographie sur couche mince avec un mélange éluant : éther de pétrole 100/ acétate d'éthyle 12 ; V/V. Après douze heures d'agitation, un précipité brun clair se dépose. Le mélange réactionnel est filtré et le précipité est lavé cinq fois à l'heptane. Les phases organiques sont rassemblées puis concentrées sous pression réduite (20 mmHg).

Le mélange est séparé sur colonne de 15 cm de silice (Si 60, 40-63 µm, Merck®) par élution à l'aide d'un mélange acétate d'éthyle 8/ éther de pétrole 100 ; V/V. Le produit final (1) (R'_{F} = CF₃, R = H) est obtenu sous forme de deux isomères (cis-trans) avec un rendement de 98%.

Le produit final (1) obtenu est chromatographié sur plaque préparative de silice de 2mm d'épaisseur avec un mélange éluant : acétate d'éthyle 12/ éther de pétrole 100 ; V/V, et montre un seul produit possédant un RF de 0,78.

### Exemple 2-1

### Réaction du 1-perfluoropropyl-2-(4-méthoxyphénoxy)-propénal (1) (R'_{F} = C₃F₇, R = p-OMe) avec l'acétylacétone.

Dans un ballon on mélange 0,21g (4,7 10⁻⁴ mole) du composé (1) préparé selon l'exemple 1-3 (R'_{F} = C₃F₇) et 0,047g (4,7 10⁻⁴ mole) d'acétylacétone dans l'éthanol (5 ml). Puis on ajoute 0,054 g (7,06 10⁻⁴ mole) d'acétate d'ammonium et on laisse sous agitation à température ambiante (20°C) pendant deux heures. Le mélange réactionnel est ensuite extrait à l'éther éthylique. Le solvant est évaporé sous pression réduite (20mm Hg), et le produit obtenu est utilisé dans l'étape suivante sans purification particulière. Le rendement est supérieur à 95%.

### Caractéristiques:

RMN ¹H (250 MHz, CDCl₃) : δ 2,2 (s, 3H) ; 2,4 (s, 3H) ; 3,8 (s, 3H) ; 5,45 (d, J = 10,31Hz, 1H) ; 6,7 (m, 4H) ; 6,9 (d, J = 12,62Hz, 1H).
RMN ¹³C (400 MHz, CDCl₃): δ 25 (s, 1C); 55 (s, 1C); 105 (s, 1C) ; 113 (s, 1C) ; 115 (s, 1C) ; 118 (s, 1C) ; 119 (s, 1C) ; 145 (s, 1C) ; 157 (s, 1C).
RMN ¹⁹F (250 MHz, CDCl₃): δ -126 (s, 2F); -123,5 (s, 2F); -80 (s, 3F).
MS (FAB⁺, GT): [M+H⁺]⁺ = 429.

Le produit (4) ainsi obtenu est dissous dans de l'éthanol (5 ml), puis on soumet cette solution à un dégagement d'ammoniac. L' éthanol est ensuite évaporé et le résidu est chromatographié sur colonne de 2cm de silice (Si 60, 40-63 µm, Merck®) à l'aide d'un mélange éluant : acétate d'éthyle 3/ éther de pétrole 100 ; V/V. La pyridine (5) (R'_{F} = C₃F₇) est obtenue avec un rendement de 85%.

Le produit final (5) obtenu est chromatographié sur plaque préparative de silice de 2mm d'épaisseur avec un mélange éluant : acétate d'éthyle 10/ éther de pétrole 100 ; V/V., et montre un seul produit avec un RF = 0,68.

### Caractéristiques :

RMN ¹H (250 MHz, CDCl₃) : δ 2,65 (s, 3H) ; 2,75 (s, 3H) ; 7,6 (d, J = 8,04 Hz, 1H) ; 8,1 (d, J = 8,05Hz, 1H).
RMN ¹⁹F (250 MHz, CDCl₃): δ -127 (s, 2F) ; -119,5 (s, 2F) ;-79,5 (s, 3F).
MS (FAB⁺, GT): [M+H⁺]⁺ = 304.
Spectroscopie de masse haute résolution : masse calculée = 303,1861 ; masse obtenue = 303,1868

### Exemple 3-1

### Réaction du 1-perfluoropentyl-1-(4-méthoxyphénoxy)-propénal 2 (R'_{F} = C₅F₁₁, R = p-OMe) avec l'aniline.

Dans un ballon on ajoute 0,20 g (4,48 10⁻⁴ mole) du composé (1) préparé selon l'exemple 1-1 (R'_{F} = C₅F₁₁) à 0,041 g (4,48 10⁻⁴ mole) d'aniline dans du THF (10 ml). On met sous agitation et on ajoute 1,5 ml d'acide formique 99%. Puis on porte le mélange réactionnel à la température de 60°C pendant deux heures. On obtient la quinoléine (6) sans purification particulière avec un rendement de 95%.

Le quinoléine (6) obtenue est chromatographiée sur plaque préparative de silice de 2 mm d'épaisseur, avec un mélange éluant : acétate d'éthyle 10/ éther de pétrole 100 ; V/V, et montre un seul produit possédant un RF = 0,8.

### Caractéristiques :

RMN ¹H (250 MHz, C₆D₆) : δ 7,0 - 7, 3 (m, 4H) ; 7, 5 (d, J = 8,66 Hz, 1H) ; 8,24 (d, J = 8,6 Hz, 1H).
RMN ¹³C (400 MHz, CDCl₃): δ 30 (s, 1C); 118 (s, 1C) ; 126 (s, 1C) ; 128 (s, 1C) ; 130 (s, 1C) ; 131 (s, 1C) ; 138 (s, 1C) ; 147 (s, 1C).
RMN ¹⁹F (250 MHz, C₆D₆) : δ -126 (t, 2F) ; -122 (t, 2F); -121 (t, 2F); -112 (t, 2F); -81 (t, 3F).
MS (FAB⁺, GT): [M+H⁺]⁺ = 398.
Spectroscopie de masse haute résolution : masse calculée = 397,2015 ; masse obtenue = 397,2106

### Exemple 3-2

### Réaction du 1-perfluoropentyl-1-phénoxy-propénal (1) (R'_{F} = C₅F₁₁, R = H) avec l'aniline.

On procède selon le même mode opératoire que pour l'exemple 3-1 en utilisant 0,5 g (1,58 10⁻³ mole) du composé 1 préparé selon l'exemple 1-2 et 0,147 g (1,58 10⁻³ mole) d'aniline dans 15 ml de THF.

On obtient le même produit final (6).

### Exemple 4-1

### Réaction du 1-perfluoropropyl-1-(4-méthoxyphénoxy)-propénal (1) (R'_{F} = C₃F₇, R = OMe) avec l'urée

Dans un ballon contenant 0,63 g (1,82 10⁻³ mole) du composé (1) préparé selon l'exemple 1-3 (R'_{F} = C₃F₇, R = p-OMe) dans une solution de 1% d'acide sulfurique dans l'éthanol (10 ml), on ajoute 0,109 g (1,82 10⁻³ mole) d'urée. Le mélange est porté à reflux pendant deux jours. En fin de réaction le mélange est neutralisé avec une solution éthanolique de 1% en masse d'hydroxyde de sodium. Après extraction à l'éther éthylique et concentration, le mélange réactionnel est chromatographié sur colonne de 2 cm de silice (Si 60, 40-63 µm, Merck®) avec un mélange éluant : acétate d'éthyle 3/ éther de pétrole 100 ; V/V, pour obtenir la pyrimidone (7) (R'_{F} = C₃F₇, Y = O) avec un rendement de 80%.

La pyrimidone (7) obtenue est chromatographiée sur plaque préparative de silice de 2 mm d'épaisseur, avec un mélange éluant : acétate d'éthyle 10/ éther de pétrole 100 ; V/V, pour donner un seul produit possédant un RF = 0,75.

### Caractéristiques :

RMN ¹⁹F (300 MHz, CDCl₃): δ ppm -125,5 (s, 2F), -116,5 (t, 2F), -79 (s, 3F).
RMN ¹H (300 MHz, CDCl₃): δ ppm 2,3 (s, 1H), 7,1 (d, 1H, J= 7.95Hz), 8,65 (d, 1H, J=7.97Hz).
MS (FAB⁺, GT) : [M+H⁺]⁺ = 265.
Spectroscopie de masse haute résolution : masse calculée = 264,0148 ; masse obtenue = 264,0155

### Exemple 4-2

### Réaction du 1-perfluoropropyl-1-phénoxypropénal (1) (R'_{F} = C₃F₇, R = H) avec l'urée.

On procède selon le même mode opératoire que pour l'exemple 4-1 en utilisant 1 g (3,16 10⁻³ mole) du composé (1) préparé selon l'exemple 1-4 et 0,189 g (3,16 10⁻³ mole) d'urée dans 10 ml d'éthanol à 1% d'acide sulfurique.

La chromatographie est effectuée sur colonne de 2 cm de silice avec le même éluant que précédemment. On obtient la même pyrimidone (7) (Y = O) avec un rendement de 76%.

### Exemple 4-3

### Réaction du 1-perfluoropropyl-1-(4-méthoxyphénoxy)-propénal (1) (R'_{F} = C₃F₇, R = p-OMe) avec la thiourée

On procède selon le même mode opératoire que pour l'exemple 4-1, en utilisant 2 g (5,78 10⁻³ mole) du composé (1) préparé selon l'exemple 1-3 et 0,439 g (5,78 10⁻³ mole) de thiourée dans 15 ml d'éthanol à 1% d'acide sulfurique.

La chromatographie est effectuée sur colonne de 2 cm de silice (Si 60, 40-63 µm, Merck®) avec le même mélange éluant que précédemment.

On obtient la 2-thiopyrimidine (7) (R'_{F} = C₃F₇, Y = S), avec un rendement de 85%. La thiopyrimidine obtenue dimérise facilement à l'air libre pour donner le composé (8).

### Caractéristiques du composé (8) :

RMN ¹⁹F (300 MHz, CDCl₃): δ ppm -126,5 (s, 4F), -117,1 (t, 4F), -80,5 (s, 6F).
RMN 1H (300 MHz, CDCl₃): δ ppm 1,6 (s, 2H), 7,42 (d, 2H,J=4.99Hz), 8,83 (d, 2H, J=4.98Hz).
MS (FAB⁺, GT) :[M+H⁺]⁺ = 559.
Spectroscopie de masse haute résolution : masse calculée = 558,9732 ; masse obtenue = 558,9737

### Exemple 4-4

### Réaction du 1-perfluoropropyl-1-phénoxypropénal (1) (R'_{F} = C₃F₇, R = H) avec la thiourée

On procède selon le même mode opératoire que pour l'exemple 4-1, en utilisant 1 g (3,16 10⁻³ mole) du composé (1) préparé selon l'exemple 1-4 et 0,24 g (3,16 10⁻³ mole) de thiourée dans 10ml d'éthanol à 1% d'acide sulfurique.

La chromatographie est effectuée sur colonne de 2 cm de silice (Si 60, 40-63 µm, Merck®) avec le même éluant que précédemment.

On obtient la même 2-thiopyrimidine (8) (Y = S) que dans l'exemple 4-4 avec un rendement de 78%.

### Exemple 5-1

### Réactivité du 1-perfluoropentyl-1-(4-méthoxyphénoxy)propénal (1) (R'_{F} = C₅F₁₁, R = p-OMe) avec AlCl₃

Dans un ballon contenant 3 g (6,72 10⁻³ mole) du composé (1) préparé selon l'exemple 1-1 (R'_{F} = C₅F₁₁) dans du 1,2-dichloroéthane (15 ml), on ajoute 0,89 g (6,72 10⁻³ mole) de chlorure d'aluminium anhydre en poudre. Le milieu réactionnel est soumis à agitation durant 12 heures à 85°C. En fin de réaction le mélange est additionné de 20 ml d'eau puis extrait avec du 1,2-dichloroéthane (3 × 8 ml). La phase organique est concentrée sous pression réduite. Le chroménol (9) (R'_{F} = C₅F₁₁) est obtenu avec un rendement de 85%, et utilisé dans les étapes suivantes sans purification particulière. Le composé (9) final (R'_{F =} C₅F₁₁, R = 6-OMe) après chromatographie sur plaque préparative de silice de 2 mm d'épaisseur avec un mélange éluant : acétate d'éthyle 20/ éther de pétrole 100 ; V/V, apparaît comme un seul produit avec un RF = 0,3.

### Caractéristiques :

RMN ¹H (400 MHz, C₆D₆) : δ 3,25 (s,3H); 5,68 (d, J = 9,88 Hz, 1H); 6,28 (d, J = 9,88 Hz, 1H) ; 6,46 (d, J = 2,95 Hz, 1H) ; 6,66 (dd, J = 8,88 HZ et 2,97 Hz) ; 6,88 (d, J = 8,85 Hz, 1H).
RMN ¹³C (400 MHz, CDCl₃) : δ 56 (s, 1C) ; 113 (s, 1C) ; 116 (s, 1C) ; 117 (s, 1C) ; 118 (s, 1C) ; 119 (s, 1C) ; 129 (s, 1C) ; 144 (s, 1C) ; 155 (s, 1C).
RMN ¹⁹F (250 MHz, CDCl₃) : δ -127 (s, 2F); -123 (s, 2F); - 120,5 (s, 2F); -123,8 (dd, J = 1108,3 Hz et 285 Hz, 2F); -81,3 (s, 3F).
MS (FAB⁺, GT) : [M+H⁺]⁺ = 447.
Spectroscopie de masse haute résolution : masse calculée = 446,0376 ; masse obtenue = 446,0387

### Exemple 6-1

### Réaction du 2-perfluoropentylchromène-2-ol (9) (R'_{F} = C₅F₁₁) avec le 2-(triméthylsilyloxy)-propène

Dans un ballon contenant 0,13 g (2,91 10⁻⁴ mole) du chroménol (9) préparé selon l'exemple 5-1 (R'_{F} = C₅F₁₁) dans l'éther de pétrole (6 ml) on ajoute 0,044 g (2,91 10⁻⁴ mole) de 2-(triméthylsilyloxy)-propène (solution commerciale à 85%). Le mélange réactionnel est maintenu sous agitation magnétique et refroidi à -20°C, ensuite on ajoute 0,075 g (2,91 10⁻⁴ mole) de SnCl₄ et l'agitation est maintenue pendant 15 minutes. On laisse revenir à température ambiante (20°C) et on laisse agiter pendant une heure. On ajoute au mélange réactionnel 10 ml d'eau et on extrait à l'éther éthylique. La phase organique est concentrée sous pression réduite (20 mmHg), et le composé (10) (R'_{F} = C₅F₁₁, R = 6-OMe) est obtenu avec un rendement supérieur à 95% et utilisé sans purification particulière.

Le produit final (10) après chromatographie sur plaque préparative de silice de 2 mm d'épaisseur à l'aide d'un mélange éluant : acétate d'éthyle 6/ éther de pétrole 100 ; V/V, montre un seul produit avec un RF = 0,65.

### Caractéristiques :

RMN ¹H (250 MHz, C₆D₆): δ 1,45 (s, 3H); 2,1 (m, 2H); 3,3 (s, 3H); 3,9 (m, 1H); 5,7 (d, J = 4,97 Hz, 1H); 6,5 (m, 2H); 6,8 (d, J = 8,72 Hz, 1H).
RMN ¹⁹F (250 MHz, C₆D₆): δ -126, 5 (s, 2F); -123 (m, 4F); -116,5 (s, 2F); -81,5 (s, 3F).
MS (FAB⁺, GT) : [M+H⁺]⁺ = 487.
Spectroscopie de masse haute résolution : masse calculée = 486,0689 ; masse obtenue = 486,0688

### Exemple 7-1

### Réaction du 2-perfluoropentylchromèn-2-ol avec le 4-méthoxyphénol.

Dans un bain de glace on mélange 0,46 g (1,03 10⁻³ mole) du composé (9) préparé selon l'exemple 5-1 (R'_{F} = C₅F₁₁, R = 6-OMe) et 0,128g (1,03 10⁻³ mole) de 4-méthoxyphénol dans le THF (2 ml). Puis on ajoute 1ml d'acide formique à 99%, après agitation vigoureuse on ajoute goutte à goutte 0,8 ml d'acide sulfurique concentré. Ensuite on laisse revenir à température ambiante (20°C). Au bout d'une heure, on ajoute 15 ml d'eau au milieu réactionnel qui est par la suite extrait avec de l'éther éthylique. La phase organique est concentrée sous pression réduite. Le résidu est dissous dans l'heptane et le produit cristallise à environ 0°C au bout de 10 heures. Le produit 11 (R'_{F} = -C₅F₁₁) est obtenu avec un rendement supérieur à 95%.

Le produit final 11 obtenu est chromatographié sur plaque préparative de silice de 2 mm d'épaisseur avec un mélange éluant : acétate d'éthyle 15/ éther de pétrole 100 ; V/V, pour donner un seul produit possédant un RF = 0,35.

### Caractéristiques :

RMN ¹H (400 MHz, C₆D₆) : δ 3,1 (s, 1H); 3,25 (s, 3H); 4,1 (m, 1H); 5,2 (m, 1H); 5;6 (d, J = 4,29 Hz, 1H); 6,16 (d, J = 8,7 Hz, 1H) ;6,52 (dd, J= 8,69 Hz et 2,97Hz, 1H) 6,73 (m, 2H) ; 6,88 (d, J = 8,96 Hz, 1H).
RMN ¹⁹F (250 MHz, C₆D₆): δ -127 (t,2F); -123,5 (t, 2F); -122,8 (t, 2F); -117,5 (m, 2F); -81,5 (t, 3F).
MS (FAB⁺, GT): [M+H⁺]⁺ = 553.
Spectroscopie de masse haute résolution : masse calculée = 552,0795 ; masse obtenue = 552,0809

### Exemple 7-2

### Réaction du 2-perfluoropropylchromène-2-ol avec le 4-méthoxyphénol.

On procède selon le même mode opératoire que pour l'exemple 7-1, en utilisant 5 g (1,44 10⁻² mole) du composé 9 correspondant et 1,79 g (1,44 10⁻² mole) de 4-méthoxyphénol dans 20 ml de THF, 4 ml d'acide formique à 99%, 3 ml d'acide sulfurique.

On obtient le composé 11 (R'_{F} = C₃F₇) correspondant.

### Caractéristiques :

RMN ¹⁹F (300 MHz, CDCl₃) : δ ppm -127,1 (s, 2F), -119,1 (t, 2F), -81 (s, 3F).
RMN ¹H (300 MHz, CDCl₃) : δ ppm 3,7 (s, 4H), 4,9 (m, 1H), 5,25 (m, 1H), 5,71 (d, 1H), 6,57 (d, 1H), 6,62 (d, 1H), 6,7 (m, 4H), 6,98 (d, 1H).
MS (FAB⁺, GT) : [M+H⁺]⁺ = 453.
Spectroscopie de masse haute résolution : masse calculée = 452,0859 ; masse obtenue = 452,0769.

### Exemple 8-1

### Réaction du 1-perfluoropentyl-1-(4-méthoxyphénoxy)-propénal (1) (R'_{F} = C₅F₁₁, R = p-OMe) avec l'aniline

Dans un ballon contenant 0,24g (5,76 10⁻⁴ mole) du composé (1) préparé selon l'exemple 1-1 (R'_{F} = C₅F₁₁, R = p-OMe) dans une solution de 2% d'acide chlorhydrique dans le méthanol (2 ml), on ajoute 0,053g (5,76 10⁻⁴ mole) d'aniline. Le mélange est porté à reflux pendant 12 heures. En fin de réaction le mélange est neutralisé avec une solution aqueuse de 2% en masse d'hydrogénocarbonate de sodium. Après extraction à l'éther éthylique et concentration, le mélange réactionnel est chromatographié sur colonne de 2cm de silice (Si 60, 40-63 µm, Merck^{®}) avec un mélange éluant : acétate d'éthyle 10/ éther de pétrole 100 ; V/V, pour obtenir le composé (12) (R'_{F} = C₅F₁₁, R₁ = H) avec un rendement de 78%.

Le composé (12) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,48.

### Caractéristiques :

RMN ¹⁹F (300.13 MHz, CDCl₃): δ ppm -126 (s, 2F), -123 (m, 4F), -120,5 (t, J = 14,1 Hz, 2F); -81 (s, 3F).
RMN ¹H (300.13 MHz, CDCl₃): δ ppm 5,7 (d, J = 7, 5 Hz, 1H); 7,2 (m, 3H); 7,4 (m, 2H); 7,7 (q, J = 7,5 Hz, 1H); 11,9 (1H, NH).
RMN ¹³C (300,13 MHz, CDCl₃): δ ppm 91,5; 117,4; 125,8; 129,9; 138,6; 149,3; 180,4 (q, ³J_{CF} = 24,8Hz).
MS (FAB⁺, GT) : [M+H⁺]⁺ = 416.
Spectroscopie de masse haute résolution : masse calculée = 416,0508 ; masse obtenue = 416,0527.

### Exemple 8-2

### Réaction du 1-trifluorométhyl-1-(4-méthoxyphénoxy)-propénal (1) (R'_{F} = CF₃, R = p-OMe) avec la para-anisidine.

Dans un ballon contenant 0,5 g (2 10⁻³ mole) du composé (1) préparé selon l'exemple 1-5 (R'_{F} = CF₃, R = p-OMe) dans une solution de 2% d'acide chlorhydrique dans le méthanol (3 ml), on ajoute 0,21g (2 10⁻³ mole) de para-anisidine. Le mélange est porté à reflux pendant 12 heures. En fin de réaction le mélange est neutralisé avec une solution aqueuse de 2% en masse d'hydrogénocarbonate de sodium. Après extraction à l'éther éthylique et concentration, le mélange réactionnel est chromatographié sur colonne de 2cm de silice (Si 60, 40-63 µm, Merck^{®}) avec un mélange éluant : acétate d'éthyle 10/ éther de pétrole 100 ; V/V, pour obtenir le composé (12) (R'_{F} = CF₃, R₁ = p-Me) avec un rendement de 84%.

Le composé (12) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,49.

### Caractéristiques :

RMN ¹⁹F (300.13 MHz, CDCl₃): δ ppm - 77,6 (s, 3F).
RMN ¹H (300.13 MHz, CDCl₃): δ ppm 5,6 (d, J = 7,3 Hz, 1H); 7,3 (m, 4H); 8,1 (q, J = 7,4 Hz, 1H); 11,9 (1H, NH).
MS (FAB⁺, GT) : [M+H⁺]⁺ = 230.
Spectroscopie de masse haute résolution : masse calculée = 229,0714 ; masse obtenue = 229,0728.

### Exemple 8-3

### Réaction du 1-trifluorométhyl-1-(4-méthoxyphénoxy)-propénal (1) (R'_{F} = CF₃, R = p-OMe) avec la ortho-anisidine.

Dans un ballon contenant 0,65 g (2,64 10⁻³ mole) du composé (1) préparé selon l'exemple 1-5 (R'_{F} = CF₃, R = p-OMe) dans une solution de 2% d'acide chlorhydrique dans le méthanol (3,5 ml), on ajoute 0,28g (2,64 10⁻³ mole) d'ortho-anisidine. Le mélange est porté à reflux pendant 12 heures. En fin de réaction le mélange est neutralisé avec une solution aqueuse de 2% en masse d'hydrogénocarbonate de sodium. Après extraction à l'éther éthylique et concentration, le mélange réactionnel est chromatographié sur colonne de 2cm de silice (Si 60, 40-63 µm, Merck®) avec un mélange éluant : acétate d'éthyle 10/ éther de pétrole 100 ; V/V, pour obtenir le composé (12) (R'_{F} = CF₃, R₁ = o-Me) avec un rendement de 84%.

Le composé (12) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,65.

### Caractéristiques :

RMN ¹⁹F (300.13 MHz, CDCl₃) : δ ppm - 77,4 (s, 3F).
RMN ¹H (300.13 MHz, CDCl₃) : δ ppm 5,8 (d, J = 7,3 Hz, 1H); 7,1 (t, J = 7,5Hz, 1H); 7,3 (m, 2H); 7,5 (d, J = 8Hz, 1H); 8, 2 (q, J = 7,2 Hz, 1H); 12 (1H, NH).
MS (FAB⁺, GT) : [M+H⁺]⁺ = 230.
Spectroscopie de masse haute résolution : masse calculée = 229,0714 ; masse obtenue = 229,0728.

### Exemple 8-4

### Réaction du 1-trifluorométhyl-1-(4-méthoxyphénoxy)-propénal (1) (R'_{F} = CF₃, R = p-OMe) avec la para-nitroaniline.

Dans un ballon contenant 0,55 g (2,2 10⁻³ mole) du composé (1) préparé selon l'exemple 1-5 (R'_{F} = CF₃, R = p-OMe) dans une solution de 2% d'acide chlorhydrique dans le méthanol (2,5 ml), on ajoute 0,3g (2,2 10⁻³ mole) de para-nitroaniline. Le mélange est porté à reflux pendant 12 heures. En fin de réaction le mélange est neutralisé avec une solution aqueuse de 2% en masse d'hydrogénocarbonate de sodium. Après extraction à l'éther éthylique et concentration, le mélange réactionnel est chromatographié sur colonne de 2cm de silice (Si 60, 40-63 µm, Merck®) avec un mélange éluant : acétate d'éthyle 20/ éther de pétrole 100 ; V/V, pour obtenir le composé (12) (R'_{F} = CF₃, R₁ = p-NO₂) avec un rendement de 72%.

Le composé (12) obtenu est chromatographié sur plaque préparative de silice de 2mm d'épaisseur, avec un mélange éluant : acétate d'éthyle 25/ éther de pétrole 100 ; V/V, pour donner un seul produit possédant un RF = 0,45.

### Caractéristiques :

RMN ¹⁹F (300.13 MHz, CDCl₃) : δ ppm - 76,4 (s, 3F).
RMN ¹H (300.13 MHz, CDCl₃) : δ ppm 6 (d, J = 12,4 Hz, 1H); 7,4 (d, J = 9,1 Hz, 1H); 8,2 (d, J = 9,1 Hz, 1H); 8,4 (t, J = 12,7 Hz, 1H); 11.3 (d, J = 12,9 Hz, 1H).
MS (FAB⁺, GT) : [M+H⁺]⁺ = 261.
Spectroscopie de masse haute résolution : masse calculée = 260,0409 ; masse obtenue = 260,0410.

### Exemple 9-1

### Réaction du 1-perfluoropentyl-1-phénoxy-propénal (1) (R'_{F} = C₅F₁₁, R = H) avec l'aniline dans le dichlorométhane.

Dans un ballon contenant 4,27g (1,02 10⁻² mole) du composé (1) (R'_{F} = C₅F₁₁, R = H) dans le dichlorométhane (10ml), on ajoute 0,95g (1,02 10⁻² mole) d'aniline. Le mélange est mis sous agitation magnétique à température ambiante pendant 12 heures. En fin de réaction on ajoute de l'eau (15ml) et le mélange est extrait à l'éther éthylique. Les phases organiques sont rassemblées puis concentrées sous pression réduite (20 mmHg). Le liquide obtenu est chromatographié sur colonne de 10cm de silice (Si 60, 40-63 µm, Merck®) avec un mélange éluant : acétate d'éthyle 10/ éther de pétrole 100 ; V/V, pour obtenir le composé (13) (R'_{F} = C₅F₁₁, R = R₂ = H) avec un rendement de 94%.

Le composé (13) obtenu est chromatographié sur plaque préparative de silice de 2mm d'épaisseur, avec un mélange éluant : acétate d'éthyle 15/ éther de pétrole 100 ; V/V, pour donner un seul produit sous forme d'un mélange de stéréo-isomères (Z-E) possédant un RF = 0,7.

### Caractéristiques :

RMN ¹⁹F (300.13 MHz, CDCl₃) : δ ppm -126,1 (t, 2F); -122,2 (t, 2F); -114,9 (t, 2F); -111,7 (t, 2F); -80,8 (t, 3F).
RMN ¹H (300.13 MHz, CDCl₃) : δ ppm 6,2 (d, J = 8, 9 Hz, 1H); 6,9 (d, J = 9 Hz, 1H); 7,2 (m, 4H); 7,4 (m, 6H); 8,2 (d, J = 9 Hz, 1H); 8,5 (d, J = 9 Hz, 1H).
RMN ¹³C (300.13 MHz, CDCl₃) : δ ppm 115,1; 115,8; 120,8; 121; 123 (q, J = 4,9 Hz); 123,9; 126,4; 126,6; 127,4; 129,2; 129,3; 130,1; 130,5; 146,8 (q, J = 25,8 Hz); 150,7; 151,4; 152,2 (q, J = 27,3 Hz); 152,7; 153,2; 154,6 (q, J = 5,9 Hz); 157,6.
MS (FAB⁺, GT) : [M+H⁺]⁺ = 492.
Spectroscopie de masse haute résolution : masse calculée = 491,0743 ; masse obtenue = 491,0750.

### Exemple 9-2

### Réaction du 1-trifluorométhyl-1-phénoxy-propénal (1) (R'_{F} = CF₃, R = H) avec l'aniline dans le dichlorométhane.

Dans un ballon contenant 5,5g (2,55 10⁻² mole) du composé (1) préparé selon l'exemple 1-10 (R'_{F} = CF₃, R = H) dans le dichlorométhane (12ml), on ajoute 2,36g (2,55 10⁻² mole) d'aniline. Le mélange est mis sous agitation magnétique à température ambiante pendant 12 heures. En fin de réaction on ajoute de l'eau (15ml) et le mélange est extrait à l'éther éthylique. Les phases organiques sont rassemblées puis concentrées sous pression réduite (20 mmHg). Le liquide obtenu est chromatographié sur colonne de 10cm de silice (Si 60, 40-63 µm, Merck®) avec un mélange éluant : acétate d'éthyle 10/ éther de pétrole 100 ; V/V, pour obtenir le composé (13) (R'_{F} = CF₃, R = R₂ = H) avec un rendement de 95%.

Le composé (13) obtenu est chromatographié sur plaque préparative de silice de 2mm d'épaisseur, avec un mélange éluant : acétate d'éthyle 15/ éther de pétrole 100 ; V/V, pour donner un seul produit sous forme d'un mélange de stéréo-isomères (Z-E) possédant un RF = 0,68.

### Caractéristiques :

RMN ¹⁹F (300.13 MHz, CDCl₃) : δ ppm -66,5 (t, 3F).
RMN ¹H (300.13 MHz, CDCl₃) : δ ppm 6,2 (d, J = 9 Hz, 1H); 6,9 (d, J = 9 Hz, 1H); 7,1 (m, 4H); 7,3 (m, 6H); 8,3 (d, J = 9 Hz, 1H); 8,6 (d, J = 9 Hz, 1H).
RMN ¹³C (300.13 MHz, CDCl₃) : δ ppm 115,2; 115,8; 120,5; 121,2; 123 (q, J = 5 Hz); 123,9; 126,4; 126,7; 127,4; 129,2; 129,3; 130; 130,3; 146,8 (q, J = 25 Hz); 150,8; 151,4; 152,3 (q, J = 27,5 Hz); 152,7; 153,1; 154,6 (q, J = 6 Hz); 157.
MS (FAB⁺, GT) : [M+H⁺]⁺ = 292.
Spectroscopie de masse haute résolution : masse calculée = 291,0871 ; masse obtenue = 291,0860.

### Exemple 10-1

### Réaction du composé (13) (R'_{F} = CF₃, R = R² = H) avec la para-anisidine dans le dichlorométhane.

Dans un ballon contenant 1g (3,43 10⁻³ mole) du composé (13) (R'_{F} = CF₃, R = R² = H) dans le dichlorométhane (5mal), on ajoute 1,1g (1,03 10⁻² mole) de para-anisidine. Le mélange est mis sous agitation magnétique à température ambiante pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 8cm de silice (Si 60, 40-63µm, Merck®) avec un mélange éluant : acétate d'éthyle 10/ éther de pétrole 100 ; V/V, pour obtenir le composé (14) (R'_{F} = CF₃, R₃ = p-Me et R₂ = H) avec un rendement de 90%.

Le composé (14) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,75.

### Caractéristiques :

RMN ¹⁹F (300.13 MHz, CDCl₃) : δ ppm -65,7 (t, 3F).
RMN ¹H (300.13 MHz, CDCl₃) : δ ppm 2,2 (s, 3H); 5,4 (d, J = 13,8 Hz, 1H); 6,6 (d, J = 8 Hz, 1H); 6,8 (m, 4H); 7,2 (m, 4H); 7,5 (t, J = 12,8 Hz, 1H); 9,7 (d, J = 12,4 Hz, 1H).
RMN ¹³C (300.13 MHz, CDCl₃) : δ ppm 20,2, 90,4, 115, 119,1, 120, 6 (q, CF₃, ¹J_{CF} = 279,5 Hz), 129,1, 129,7, 129,9, 130,8, 131,1, 132,5, 138,2, 140,8 (q, CH, ³J_{CF} = 3,1 Hz), 147, 153,4 (q, C-CF₃, ²J_{CF} = 30,8 Hz).
MS (FAB⁺, GT) : [M+H⁺]⁺ = 292.
Spectroscopie de masse haute résolution : masse calculée = 291,0871 ; masse obtenue = 291,0860.

### Exemple 10-2

### Réaction du composé (13) (R'_{F} = CF₃, R = R² = H) avec la para-chloroaniline dans le dichlorométhane.

Dans un ballon contenant 1,5 g (5,15 10⁻³ mole) du composé (13) préparé selon l'exemple 9-2 (R'_{F} = CF₃, R = R² = H) dans le dichlorométhane (6ml), on ajoute 1,97g (1,54 10⁻² mole) de para-chloroaniline. Le mélange est mis sous agitation magnétique à température ambiante pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 8 cm de silice (Si 60, 40-63 µm, Merck®) avec un mélange éluant : acétate d'éthyle 10/ éther de pétrole 100 ; V/V, pour obtenir le composé (14) (R'_{F} = CF₃, R₃ = p-Cl et R₂ = H) avec un rendement de 85%.

Le composé (14) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,72.

### Caractéristiques :

RMN ¹⁹F (300.13 MHz, CDCl₃) : δ ppm -65,8 (t, 3F).
RMN ¹H (300.13 MHz, CDCl₃) : δ ppm 5,4 (d, J = 13,6 Hz, 1H); 6,7 (m, 3H); 6,8 (m, 4H); 7,2 (m, 2H); 7,5 (t, J = 12,9 Hz, 1H); 9,7 (d, J = 12,6 Hz, 1H).
RMN ¹³C (300.13 MHz, CDCl₃) : δ ppm 90,8, 116, 117,7, 119,7 (q, CF₃, ¹J_{CF} = 279,3 Hz) 120,5, 125,3, 127,2, 128,6, 128,8, 138,8, 140,2, 147,7, 153,3 (q, C-CF₃, ²J_{CF} = 32 Hz).
MS (FAB⁺, GT) : [M+H⁺]⁺ = 325.
Spectroscopie de masse haute résolution : masse calculée = 324,0641 ; masse obtenue = 324,0623.

### Exemple 11-1

### Réaction du composé (13) (R'_{F} = CF₃, R = R² = H) avec la para-anisidine dans le dichlorométhane à reflux.

Dans un ballon contenant 1,5g (5,15 10⁻³ mole) du composé (13) préparé selon l'exemple 9-2 (R'_{F} = CF₃, R = R² = H) dans le dichlorométhane (3 ml), on ajoute 1,65 g (1,54 10⁻² mole) de para-anisidine. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 8/ éther de pétrole 100 ; V/V, pour obtenir la quinoléine (15) (R'_{F} = CF₃, R₃ = 6-Me) avec un rendement de 92%.

Le composé (15) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,9.

### Caractéristiques :

RMN ¹⁹F (300.13 MHz, CDCl₃) : δ ppm -67,8 (s, 3F).
RMN ¹H (300.13 MHz, CDCl₃) : δ ppm 2,52 (s, 3H), 7,56 (d, J = 8,6Hz, 1H), 7,8 (m, 2H), 8,2 (d, J = 8,6Hz, 1H), 8,51 (d, J = 8,5Hz, 1H).
RMN ¹³C (300.13 MHz, CDCl₃) : δ ppm 21,65, 117,63 (q, CH, ³J_{CF} = 2,2Hz), 122,8 (q, CF₃, ¹J_{CF} = 273,9Hz), 127, 6, 130, 130, 2, 134,2, 138,8, 140, 146,6, 147,4 (q, C-CF₃, ²J_{CF} = 34,7 Hz).
MS (FAB⁺, GT) : [M+H⁺]⁺ = 212.
Spectroscopie de masse haute résolution : masse calculée = 212,0687 ; masse obtenue = 212,0707.

### Exemple 11-2

### Réaction du composé (13) (R'_{F} = CF₃, R = R² = H) avec la para-chloroaniline dans le dichlorométhane à reflux.

Dans un ballon contenant 1,5g (5,15 10⁻³ mole) du composé (13) préparé selon l'exemple 9-2 (R'_{F} = CF₃, R = R² = H) dans le dichlorométhane (3ml), on ajoute 1,97 g (1,54 10⁻² mole) de para-chloroaniline. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 8/ éther de pétrole 100 ; V/V, pour obtenir la quinoléine (15) (R'_{F} = CF₃, R₃ = 6-Cl) avec un rendement de 88%.

Le composé (15) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,88.

### Caractéristiques :

RMN ¹⁹F (300.13 MHz, CDCl₃) : δ ppm -66,6 (s, 3F).
RMN ¹H (300.13 MHz, CDCl₃) : δ ppm 7,48 (dd, J = 2,3 et 9 Hz, 1H), 7,59 (d, J = 8,6 Hz, 1H), 7,74 (d, J = 9 Hz, 1H), 7,88 (d, J = 2,3 Hz, 1H) 8,24 (d, J = 8,6 Hz, 1H).
RMN ¹³C (300.13 MHz, CDCl₃) : δ ppm 118,8 (q, CH, ³J_{CF} = 2,1 Hz), 122,3 (q, CF₃, ¹J_{CF} = 275,4 Hz), 127,8, 130,3, 132,2, 132,8, 134,3, 139,4, 145,7, 147,8 (q, C-CF₃, ²J_{CF} = 34 Hz).
MS (FAB⁺, GT) : [M+H⁺]⁺ = 232.
Spectroscopie de masse haute résolution : masse calculée = 232,0204 ; masse obtenue = 232,0200.

### Exemple 11-3

### Réaction du composé (13) (R'_{F} = CF₃, R = R² = H) avec la para-nitroaniline dans le dichlorométhane à reflux.

Dans un ballon contenant 1,5g (5,15 10⁻³ mole) du composé (13) (R'_{F} = CF₃, R = R² = H) dans le dichlorométhane (3ml), on ajoute 2,13g (1,54 10⁻² mole) de para-nitroaniline. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 20/ éther de pétrole 100 ; V/V, pour obtenir la quinoléine (15) (R'_{F} = CF₃, R³ = 6-NO₂) avec un rendement de 85%.

Le composé (15) obtenu est chromatographié sur plaque préparative de silice de 2mm d'épaisseur, avec un mélange éluant : acétate d'éthyle 25/ éther de pétrole 100 ; V/V, pour donner un seul produit possédant un RF = 0,56.

### Caractéristiques :

RMN ¹⁹F (300.13 MHz, CDCl₃) : δ ppm -68,5 (s, 3F).
RMN ¹H (300. 13 MHz, CDCl₃) : δ ppm 8,2 (d, J = 8, 6 Hz, 1H), 8,4 (d, J = 9,3 Hz, 1H), 8,6 (dd, J = 2,5 et 9,3 Hz, 1H), 9,1 (d, J = 8,6 Hz, 1H) 9,19 (d, J = 2,5 Hz, 1H).
RMN ¹³C (300.13 MHz, CDCl₃) : δ ppm 119,5 (q, CH, ³J_{CF} = 2,2 Hz), 122 (q, CF_{3,} ¹J_{CF} = 275, 4 Hz), 127, 5, 130, 1, 132, 132,8, 134,5, 140,1, 145,2, 148 (q, C-CF₃, ²J_{CF} = 34 Hz).
MS (FAB⁺, GT) : [M+H⁺]⁺ = 243.
Spectroscopie de masse haute résolution : masse calculée = 243,0381 ; masse obtenue = 243,0408.

### Exemple 11-4

### Réaction du composé (13) (R'_{F} = CF₃, R = R² = H) avec la méta-anisidine dans le dichlorométhane à reflux.

Dans un ballon contenant 1, 5g (5,15 10⁻³ mole) du composé (13) (R'_{F} = CF₃, R = R² = H) dans le dichlorométhane (3ml), on ajoute 1,65g (1,54 10⁻² mole) de méta-anisidine. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 20/ éther de pétrole 100 ; V/V, pour obtenir la quinoléine (15) (R'_{F} = CF₃, R³ = 7-Me) avec un rendement de 85%.

Le composé (15) obtenu est chromatographié sur plaque préparative de silice de 2mm d'épaisseur, avec un mélange éluant : acétate d'éthyle 25/ éther de pétrole 100 ; V/V, pour donner un seul produit possédant un RF = 0,56.

### Caractéristiques :

RMN ¹⁹F (300.13 MHz, CDCl₃) : δ ppm -67,9 (s, 3F).
RMN ¹H (300.13 MHz, CDCl₃) : δ ppm 2,6 (s, 3H), 7, 52 (d, J = 8,4 Hz, 1H), 7,8 (d, J = 8,5 Hz, 1H), 8 (m, 2H), 8,51 (d, J = 8,5 Hz, 1H).
RMN ¹³C (300.13 MHz, CDCl₃) : δ ppm 21,81, 116,7 (q, CH, ³J_{CF} = 2,2 Hz), 122,8 (q, CF₃, ¹J_{CF} = 274,3 Hz), 128,05, 128,5, 129,3, 131,9, 136,2, 139,2, 142,5, 148,4 (q, C-CF₃, ²J_{CF} = 33,9 Hz).
MS (FAB⁺, GT) : [M+H⁺]⁺ = 212.
Spectroscopie de masse haute résolution : masse calculée = 212,0687 ; masse obtenue = 212,0696.

### Exemple 11-5

### Réaction du composé (13) (R'_{F} = CF₃, R = R² = H) avec l'ortho-hydroxyaniline dans le dichlorométhane à reflux.

Dans un ballon contenant 1,5g (5,15 10⁻³ mole) du composé (13) (R'_{F} = CF₃, R = R² = H) dans le dichlorométhane (3ml), on ajoute 1,68g (1,54 10⁻² mole) d'ortho-hydroxyaniline. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 20/ éther de pétrole 100 ; V/V, pour obtenir la quinoléine (15) (R'_{F} = CF₃, R³ = 8-OH) avec un rendement de 95%.

Le composé (15) obtenu est chromatographié sur plaque préparative de silice de 2mm d'épaisseur, avec un mélange éluant : acétate d'éthyle 25/ éther de pétrole 100 ; V/V, pour donner un seul produit possédant un RF = 0,6.

### Caractéristiques :

RMN ¹⁹F (300.13 MHz, CDCl₃) : δ ppm -65,7 (s, 3F).
RMN ¹H (300.13 MHz, CDCl₃) : δ ppm 7,25 (d, J = 7,4 Hz, 1H), 7,51 (m, 2H), 7,9 (d, J = 8,5 Hz, 1H), 8,51 (d, J = 8,5 Hz, 1H), 10,25 (s, 1H, OH)
RMN ¹³C (300.13 MHz, CDCl₃) : δ ppm 112,9, 116,8 (q, CH, ³J_{CF} = 2 Hz), 117,9, 121, 5 (q, CF₃, ¹J_{CF} = 275 Hz), 129,7, 129,8, 137,3, 138,7, 144,5 (q, C-CF₃, ²J_{CF} = 34,2 Hz), 153.
MS (FAB⁺, GT) : [M+H⁺]⁺ = 214.
Spectroscopie de masse haute résolution : masse calculée = 213,0401 ; masse obtenue = 213,0391.

### Exemple 11-6

### Réaction du composé (13) (R'F = CF₃, R = R² = H) avec l'acide méta-aminobenzoïque dans le dichlorométhane à reflux.

Dans un ballon contenant 1,5g (5,15 10⁻³ mole) du composé (13) (R'_{F} = CF₃, R = R² = H) dans le dichlorométhane (3ml), on ajoute 2,12g (1,54 10⁻² mole) d'acide méta-aminobenzoïque. Le mélange est mis à reflux et sous agitation magnétique pendant 24 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 50/ éther de pétrole 50 ; V/V, pour obtenir la quinoléine (15) (R'_{F} = CF₃, R³ = 7-COOH) avec un rendement de 95%.

Le composé (15) obtenu est chromatographié sur plaque préparative de silice de 2mm d'épaisseur, avec un mélange éluant : acétate d'éthyle 80/ éther de pétrole 20 ; V/V, pour donner un seul produit possédant un RF = 0,5.

### Caractéristiques :

RMN ¹⁹F (300.13 MHz, CDCl₃) : δ ppm -66,2 (s, 3F).
RMN ¹H (300.13 MHz, CDCl₃) : δ ppm 8 (m, 1H), 8,15 (d, J = 9Hz, 1H), 8,4 (m, 2H), 9,6 (d, J = 8,9 Hz, 1H), 13,6 (1H, OH).
RMN ¹³C (300,13 MHz, CDCl₃) : δ ppm 118,1, 127,1, 127,8, 130,3, 132,7, 134,3, 137,4, 146,5, 167.
MS (FAB⁺, GT) : [M+H⁺]⁺ = 242.
Spectroscopie de masse haute résolution : masse calculée = 242,0429 ; masse obtenue = 242,0431.

### Exemple 11-7

### Réaction du composé (13) (R'_{F} = CF₃, R = R² = H) avec l'ortho-anisidine dans le dichlorométhane à reflux.

Dans un ballon contenant 1,5g (5,15 10⁻³ mole) du composé (13) (R'_{F} = CF₃, R = R² = H) dans le dichlorométhane (3ml), on ajoute 1,65g (1,54 10⁻² mole) d'ortho-anisidine. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 10/ éther de pétrole 100 ; V/V, pour obtenir la quinoléine (15) (R'_{F} = CF₃, R³ = 8-Me) avec un rendement de 86%.

Le composé (15) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,85.

### Caractéristiques :

RMN ¹⁹F (300.13 MHz, CDCl₃) : δ ppm -67,8 (s, 3F).
RMN ¹H (300.13 MHz, CDCl₃) : δ ppm 2,8 (s, 3H), 7,65 (m, 1H), 7,75 (d, J = 6,8 Hz, 1H), 7,92 (m, 2H), 8,61 (d, J = 8,5 Hz, 1H).
RMN ¹³C (300.13 MHz, CDCl₃) : δ ppm 17,6, 117,3 (q, CH, ³J_{CF} = 2,1 Hz), 122,8 (q, CF₃, ¹J_{CF} = 274, 2 Hz), 126,8, 129,5, 130, 131,8, 138,6, 139,8, 146,9, 147,1 (q, C-CF₃, ²J_{CF} = 29 Hz).
MS (FAB⁺, GT) : [M+H⁺]⁺ = 212.
Spectroscopie de masse haute résolution : masse calculée = 212,0687 ; masse obtenue = 212,0684.

### Exemple 11-8

### Réaction du composé (13) (R'_{F} = CF₃, R = R² = H) avec la para-cyanoaniline dans le dichlorométhane à reflux.

Dans un ballon contenant 1,5g (5,15 10⁻³mole) du composé (13) (R'_{F} = CF₃, R = R² = H) dans le dichlorométhane (3ml), on ajoute 1,82g (1,54 10⁻² mole) de para-cyanoaniline. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 10/ éther de pétrole 100 ; V/V, pour obtenir la quinoléine (15) (R'_{F} = CF₃, R³ = 6-CN) avec un rendement de 86%.

Le composé (15) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,7.

### Caractéristiques :

RMN ¹⁹F (300.13 MHz, CDCl₃) : δ ppm -65,9 (s, 3F).
RMN ¹H (300.13 MHz, CDCl₃) : δ ppm 8 (d, J = 8,6 Hz, 1H), 8,3 (d, J = 9, 3 Hz, 1H), 8, 5 (dd, J = 9, 3 et 2, 5 Hz, 1H), 8, 9 (d, J = 8,6 Hz, 1H), 9,2 (d, J = 2, 5 Hz, 1H)
RMN ¹³C (300.13 MHz, CDCl₃) : δ ppm 119,5, 125,1, 125,8, 129,1, 132,6, 142,4, 147,9, 149.
MS (FAB⁺, GT) : [M+H⁺]⁺ = 223.
Spectroscopie de masse haute résolution : masse calculée = 222,0405 ; masse obtenue = 222,0407.

### Exemple 12-1

### Réaction du 1-perfluoropentyl-1-(4-méthoxyphénoxy)-propénal (1) (R'_{F} = C₅F₁₁, R = p-OMe) avec l'aniline en présence d'acide para-toluènesulfonique.

On dissout 2,5g (5,6 10⁻³ mole) du composé (1) préparé selon l'exemple 1-1 (R'_{F} = C₅F₁₁, R = p-OMe) dans une solution de 1% d'acide para-toluènesulfonique dans le toluène (8 ml). On ajoute 0,52 g (5,6 10⁻³ mole) d'aniline. Le mélange est porté à reflux pendant 12 heures. En fin de réaction le mélange est neutralisé avec une solution aqueuse de 1% en masse d'hydroxyde de sodium. Après extraction à l'éther éthylique et concentration, le mélange réactionnel est chromatographié sur colonne de 15 cm de silice (Si 60, 40-63 µm, Merck®) avec un mélange éluant : acétate d'éthyle 20/ éther de pétrole 80 ; V/V, pour obtenir l'imino-énol (16) (R'_{F} = C₅F₁₁, R³ = H) avec un rendement de 67%.

Le composé (16) obtenu est chromatographié sur plaque préparative de silice de 2mm d'épaisseur, avec un mélange éluant : acétate d'éthyle 25/ éther de pétrole 75 ; V/V, pour donner un seul produit possédant un RF = 0,6.

### Caractéristiques :

RMN ¹⁹F (400.12 MHz, C₆D₆) : δ ppm -126,5 (t, 2F), -123 (t, 4F), -120 (t, 2F); -81 (t, 3F).
RMN ¹H (400.12 MHz, C₆D₆) : δ ppm 5,4 (d, J = 7 Hz, 1H); 6,6 (d, J = 8 Hz, 1H); 6,9 (q, J = 7,2 Hz, 1H); 7,2 (m, 3H); 12,1 (1H, OH).
RMN ¹³C (400.12 MHz, C₆D₆) : δ ppm 91,4; 117,4; 125,4; 129.8; 138,8; 149,1; 180,5 (q, ²J_{CF} = 24,8 Hz).
MS (FAB⁺, GT) : [M+H⁺]⁺ = 416.
Spectroscopie de masse haute résolution : masse calculée = 415,043 ; masse obtenue = 415,0421.

### Exemple 13-1

### Réduction du 1-perfluoropentyl-1-(4-méthoxyphénoxy)-propénal (1) (R'_{F} = C₅F₁₁, R = p-OMe) par le borohydrure de sodium.

A une solution contenant 10g (2,24 10⁻² mole) de 1-perfluoropentyl-1-(4-méthoxyphénoxy)-propénal (1) préparé selon l'exemple 1-1 (R'_{F} = C₅F₁₁, R = p-OMe) dans l'éther éthylique anhydre (30 ml), on ajoute 0,42g (1,12 10⁻² mole) de borohydrure de sodium. Le mélange est mis sous agitation magnétique vigoureuse à température ambiante (20°C). L'évolution de la réaction est suivie par chromatographie sur couche mince avec un mélange éluant : éther de pétrole 70/ acétate d'éthyle 30 ; V/V. Après six heures d'agitation, le mélange réactionnel est additionné d'eau. Après extraction cinq fois à l'éther éthylique, les phases organiques sont rassemblées puis concentrées sous pression réduite (20 mmHg).

Le produit final (18) (R'_{F} = C₅F₁₁, R = p-OMe) est obtenu avec un rendement de 95%.

Le produit final (18) obtenu est chromatographié sur plaque préparative de silice de 2mm d'épaisseur avec un mélange éluant : acétate d'éthyle 30/ éther de pétrole 70 ; V/V, et montre un seul produit possédant un RF de 0,55.

### Caractéristiques:

RMN ¹H (300.13 MHZ, CDCl₃) : δ ppm 1,8 (1H, OH) ; 3,8 (s, 3H); 4,3 (m, 2H); 5,3 (t, J = 7,1 Hz, 1H); 6,9 (m, 4H).
RMN ¹⁹F (300.13 MHZ, CDCl₃) : δ ppm -126 (t, 2F); -123 (m, 4F); -113 (t, 2F); -81 (t, 3F).
MS (FAB⁺, GT): [M+H⁺]⁺ = 449.
Spectroscopie de masse haute résolution : masse calculée = 448,0533 ; masse obtenue = 448,0531.

### Exemple 13-2

### Réduction du 1-perfluoropentyl-1-phénoxy-propénal (1) (R'_{F} = C₅F₁₁, R = H) par le borohydrure de sodium.

A une solution contenant 10,5g (2,52 10⁻² mole) de 1-perfluoropentyl-1-phénoxy-propénal (1) préparé selon l'exemple 1-2 (R'_{F} = C₅F₁₁, R = H) dans l'éther éthylique anhydre (30 ml), on ajoute 0,47g (1,26 10⁻² mole) de borohydrure de sodium. Le mélange est mis sous agitation magnétique vigoureuse à température ambiante (20°C). L'évolution de la réaction est suivie par chromatographie sur couche mince avec un mélange éluant : éther de pétrole 70/ acétate d'éthyle 30 ; V/V. Après six heures d'agitation, le mélange réactionnel est additionné d'eau. Après extraction cinq fois à l'éther éthylique, les phases organiques sont rassemblées puis concentrées sous pression réduite (20 mmHg).

Le produit final (18) (R'_{F} = C₅F₁₁, R = H) est obtenu avec un rendement de 93%.

Le produit final (18) obtenu est chromatographié sur plaque préparative de silice de 2mm d'épaisseur avec un mélange éluant : acétate d'éthyle 30/ éther de pétrole 70 ; V/V, et montre un seul produit possédant un RF de 0,5.

### Caractéristiques:

RMN ¹H (300.13 MHZ, CDCl₃) : δ ppm 1,8 (1H, OH); 4,2 (m, 2H); 5,3 (t, J = 7,1 Hz, 1H); 6,9 (m, 5H).
RMN ¹⁹F (300.13 MHZ, CDCl₃) : δ ppm -126.2 (t, 2F); -123 (m, 4F); -114.1 (t, 2F); -81 (t, 3F).
MS (FAB⁺, GT) : [M+H⁺]⁺ = 419.
Spectroscopie de masse haute résolution : masse calculée = 418,0427 ; masse obtenue = 418,0431.

### Exemple 13-3

### Réduction du 1-trifluorométhyl-1-(4-méthoxyphénoxy)-propénal (1) (R'_{F} = CF₃, R = p-OMe) par le borohydrure de sodium.

A une solution contenant 10g (4 10⁻² mole) de 1-trifluorométhyl-1-(4-méthoxyphénoxy)-propénal (1) préparé selon l'exemple 1-5 (R'_{F} = CF₃, R = p-OMe) dans l'éther éthylique anhydre (30 ml), on ajoute 0,77g (2 10⁻² mole) de borohydrure de sodium. Le mélange est mis sous agitation magnétique vigoureuse à température ambiante (20°C). L'évolution de la réaction est suivie par chromatographie sur couche mince avec un mélange éluant : éther de pétrole 70/ acétate d'éthyle 30 ; V/V. Après six heures d'agitation, le mélange réactionnel est additionné d'eau. Après extraction cinq fois à l'éther éthylique, les phases organiques sont rassemblées puis concentrées sous pression réduite (20 mmHg).

Le produit final (18) (R'_{F} = C₅F₁₁, R = H) est obtenu avec un rendement de 95%.

Le produit final (18) obtenu est chromatographié sur plaque préparative de silice de 2mm d'épaisseur avec un mélange éluant : acétate d'éthyle 30/ éther de pétrole 70 ; V/V, et montre un seul produit possédant un RF de 0,52.

### Caractéristiques:

RMN ¹H (300.13 MHZ, CDCl₃): δ ppm 1,8 (1H, OH); 3,7 (s, 3H); 4,5 (m, 2H); 5,4 (t, J = 7,1 Hz, 1H); 7,1 (m, 4H).
RMN ¹⁹F (300.13 MHZ, CDCl₃) : δ ppm -70,2 (t, 3F)
MS (FAB⁺, GT) : [M+H⁺]⁺ = 249.
Spectroscopie de masse haute résolution : masse calculée = 248,066 ; masse obtenue = 248,0599.

### Exemple 13-4

### Réduction du 1-perfluoropentyl-1-(4-nitrophénoxy)-propénal (1) (R'_{F} = C₅F₁₁, R = p-NO₂) par le borohydrure de sodium.

A une solution contenant 10g (2,16 10⁻² mole) de 1-trifluorométhyl-1-(4-nitrophénoxy)-propénal (1) préparé selon l'exemple 1-6 (R'_{F} = C₅F₁₁, R = p-NO₂) dans l'éther éthylique anhydre (30 ml), on ajoute 0,41g (1 10⁻² mole) de borohydrure de sodium. Le mélange est mis sous agitation magnétique vigoureuse à température ambiante (20°C). L'évolution de la réaction est suivie par chromatographie sur couche mince avec un mélange éluant : éther de pétrole 70/ acétate d'éthyle 30 ; V/V. Après six heures d'agitation, le mélange réactionnel est additionné d'eau. Après extraction cinq fois à l'éther éthylique, les phases organiques sont rassemblées puis concentrées sous pression réduite (20 mmHg).
Le produit final (18) (R'_{F} = C₅F₁₁, R = p-NO₂) est obtenu avec un rendement de 98%.

Le produit final (18) obtenu est chromatographié sur plaque préparative de silice de 2mm d'épaisseur avec un mélange éluant : acétate d'éthyle 30/ éther de pétrole 70 ; V/V, et montre un seul produit possédant un RF de 0,51.

### Caractéristiques:

RMN ¹H (300.13 MHZ, CDCl₃) : δ ppm 2,5 (1H, OH); 4,6 (m, 2H); 5,3 (t, J = 7 Hz, 1H); 6,9 (d, J = 9,1 Hz, 2H) 7,1 (d, J = 9,1 Hz, 2H).
RMN ¹⁹F (300.13 MHZ, CDCl₃) : δ ppm -68,2 (t, 3F)
MS (FAB⁺, GT) : [M+H⁺]⁺ = 464.
Spectroscopie de masse haute résolution : masse calculée = 463,0278 ; masse obtenue = 463,0268.

### Exemple 14-1

### Réaction de l'alcool (18) (R'_{F} = C₅F₁₁, R = p-OMe) avec le PBr₃.

A une solution contenant 5,2g (1,16 10⁻² mole) du composé (18) préparé selon l'exemple 13-1 (R'_{F} = C₅F₁₁, R = p-OMe) dans l'éther éthylique anhydre (10 ml), on ajoute 3,1 g (1,16 10⁻² mole) de bromure de phosphore (III). Le mélange est mis sous agitation magnétique vigoureuse à température ambiante (20°C). L'évolution de la réaction est suivie par chromatographie sur couche mince avec un mélange éluant : éther de pétrole 85/ acétate d'éthyle 15 ; V/V. Après six heures d'agitation, le mélange réactionnel est additionné d'eau. Après extraction cinq fois à l'éther éthylique, les phases organiques sont rassemblées puis concentrées sous pression réduite (20 mmHg).
Le produit final (19) (R'_{F} = C₅F₁₁, R = p-OMe) est obtenu avec un rendement de 97%.
Le produit final (19) obtenu est chromatographié sur plaque préparative de silice de 2mm d'épaisseur avec un mélange éluant : acétate d'éthyle 20/ éther de pétrole 80 ; V/V, et montre un seul produit possédant un RF de 0,8.

### Caractéristiques:

RMN ¹H (300.13 MHZ, CDCl₃) : δ ppm 3,7 (s, 3H); 4,2 (m, 2H); 5,3 (t, J = 7 Hz, 1H); 6,9 (m, 4H).
RMN ¹⁹F (300.13 MHZ, CDCl₃) : δ ppm -126, 2 (t, 2F); -123 (m, 4F); -113,1 (t, 2F); -80 (t, 3F).
MS (FAB⁺, GT) : [M+H⁺]⁺ = 511.
Spectroscopie de masse haute résolution : masse calculée = 509,9689 ; masse obtenue = 509,9679.

### Exemple 14-2

### Réaction de l'alcool (18) (R'_{F} = CF₃, R = p-OMe) avec le PBr₃.

A une solution contenant 5g (2 10⁻² mole) du composé (18) (R'_{F} = CF₃, R = p-OMe) dans l'éther éthylique anhydre (10 ml), on ajoute 5,4g (2 10⁻² mole) de bromure de phosphore (III). Le mélange est mis sous agitation magnétique vigoureuse à température ambiante (20°C). L'évolution de la réaction est suivie par chromatographie sur couche mince avec un mélange éluant : éther de pétrole 85/ acétate d'éthyle 15 ; V/V. Après six heures d'agitation, le mélange réactionnel est additionné d'eau. Après extraction cinq fois à l'éther éthylique, les phases organiques sont rassemblées puis concentrées sous pression réduite (20 mmHg).
Le produit final (19) (R'_{F} = CF₃, R = p-OMe) est obtenu avec un rendement de 95%.

Le produit final (19) obtenu est chromatographié sur plaque préparative de silice de 2mm d'épaisseur avec un mélange éluant : acétate d'éthyle 20/ éther de pétrole 80 ; V/V, et montre un seul produit possédant un RF de 0,81.

### Caractéristiques:

RMN ¹H (300.13 MHZ, CDCl₃): δ ppm 3, .7 (s, 3H); 4,4 (m, 2H); 5,4 (t, J = 7,2 Hz, 1H); 7 (m, 4H).
RMN ¹⁹F (300.13 MHZ, CDCl₃) : δ ppm -68,7 (t, 3F)
MS (FAB⁺, GT) : [M+H⁺]⁺ = 311.
Spectroscopie de masse haute résolution : masse calculée = 309,9816 ; masse obtenue = 309,9821.

### Exemple 15-1

### Réaction du 1-perfluoropentyl-1-phénoxy-propénal (1) (R'_{F} = C₅F₁₁, R = H) avec la phénylhydrazine.

A une solution contenant 10g (2,4 10⁻² mole) de 1-perfluoropentyl-1-phénoxy-propénal (1) préparé selon l'exemple 1-2 (R'_{F} = C₅F₁₁, R = H) dans le dichlorométhane (20 ml), on ajoute 3,5g (2,4 10⁻² mole) d'hydrochlorure de phénylhydrazine. Le mélange est additionné de 15 ml d'une solution aqueuse de 5% d'hydrogénocarbonate de sodium, puis mis sous agitation magnétique vigoureuse à température ambiante (20°C). L'évolution de la réaction est suivie par chromatographie sur couche mince avec un mélange éluant : éther de pétrole 70/ acétate d'éthyle 30 ; V/V. Après deux heures d'agitation le mélange réactionnel est extrait cinq fois à l'éther éthylique. Les phases organiques sont rassemblées puis concentrées sous pression réduite (20 mmHg).

Le mélange des deux isomères est séparé sur colonne de 25cm de silice (Si 60, 40-63 µm, Merck®) par élution à l'aide d'un mélange acétate d'éthyle 15/ éther de pétrole 85 ; V/V. Les produits (20) (R'_{F} = C₅F₁₁, R = R⁴ = H) sont obtenus avec un rendement de 70/30 (isomères Z/E respectivement).

Les produits finaux (20) obtenus sont chromatographiés sur plaque préparative de silice de 2mm d'épaisseur avec un mélange éluant : acétate d'éthyle 20/ éther de pétrole 80 ; V/V, et montrent un seul produit pour chaque isomère possédant un RF de 0,5 pour l'isomère Z et 0,62 pour l'isomère E.

### Caractéristiques:

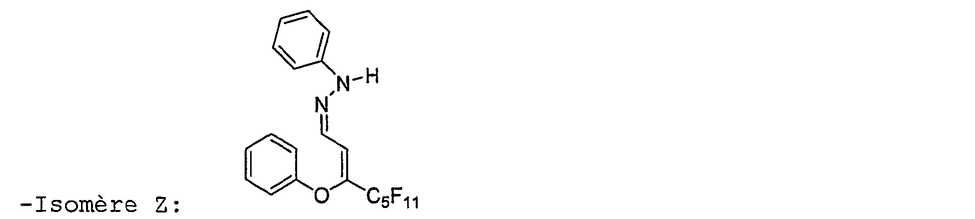

RMN ¹H (300.13 MHZ, CDCl₃): δ 6,8 (d, J = 9, 34 Hz, 1H); 7,1 (m, 6H); 7,3 (m, 5H); 7,9 (s, 1H, NH).
RMN ¹⁹F (300.13 MHZ, CDCl₃) : δ -126,2 (t, 2F); -123 (t, 2F); -122 (t, 2F); -114 (t, 2F); -81 (t, 3F).
RMN ¹³C (300.13 MHZ, CDCl₃) : 113; 115, 3; 121,4; 123,1; 129,4; 129,7; 130,2; 137,9; 143; 157,5.
MS (FAB⁺, GT) : [M+H⁺]⁺ = 507.
Spectroscopie de masse haute résolution : masse calculée = 506,0852 ; masse obtenue = 506,0851.

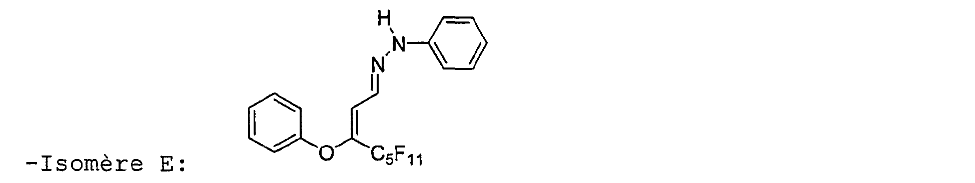

RMN ¹H (300.13 MHZ, CDCl₃): δ 6,2 (d, J = 9,74 Hz, 1H); 6,9 (m, 3H); 7,1 (m, 5H); 7,4 (m, 2H); 7,65 (d, J = 9,7 Hz, 1H); 7,8 (s, 1H, NH).
RMN ¹⁹F (300.13 MHZ, CDCl₃): δ -126,1 (t, 2F); -122,2 (t, 4F); -112 (t, 2F); -81 (t, 3F).
RMN ¹³C (300.13 MHZ, CDCl₃): 113; 115, 5; 121,4; 122,3 123,1; 129,4; 129,7; 130; 137,9; 143,5; 154,6.
MS (FAB⁺, GT): [M+H⁺]⁺ = 507.
Spectroscopie de masse haute résolution : masse calculée = 506,0852 ; masse obtenue = 506,0861.

### Exemple 16-1

### Cyclisation du composé (20) (R'_{F} = C₅F₁₁, R = H) en présence de pyridine dans le toluène à reflux.

Dans un ballon contenant 3g (5,9 10⁻³ mole) du composé (20) préparé selon l'exemple 15-1 (R'_{F} = C₅F₁₁, R = H) dans le toluène (5ml), on ajoute 2ml de pyridine. Le mélange est mis à reflux et sous agitation magnétique pendant 12 heures. En fin de réaction de l'eau est additionnée (10 ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40863 µm, Merck®) avec un mélange éluant : acétate d'éthyle 15/ éther de pétrole 85 ; V/V, pour obtenir le phénylpyrazole (21) (R'_{F} = C₅F₁₁, R⁴ = H) avec un rendement de 85%.

Le composé (21) obtenu est chromatographié sur plaque préparative de silice de 2mm d'épaisseur, avec un mélange éluant : acétate d'éthyle 20/ éther de pétrole 80 ; V/V, pour donner un seul produit possédant un RF = 0,78.

### Caractéristiques :

RMN ¹⁹F (300.13 MHz, CDCl₃) δ ppm -127 (t, 2F); -123 (t, 4F); -113,1 (t, 2F); -81 (t, 3F).
RMN ¹H (300.13 MHz, CDCl₃): δ ppm 6,5 (d, J = 9,4 Hz, 1H); 6,9 (m, 3H); 7,1 (m, 2H); 7,8 (d, J = 9,4 Hz, 1H).
MS (FAB⁺, GT) : [M+H⁺]⁺ = 413.
Spectroscopie de masse haute résolution : masse calculée = 412,0434 ; masse obtenue = 412,0440.

### Exemple 17-1

### Réaction du 1-perfluoropentyl-1-(4-methoxyphénoxy)-propénal (1) (R'_{F} = C₅F₁₁, R = p-OMe) avec l'hydroxylamine (R⁵ = H) .

Dans un ballon contenant 10,5 g (2,35 10⁻² mole) du composé (1) préparé selon l'exemple 1-1 (R'_{F} = C₅F₁₁, R = p-OMe) dans une solution de 1% d'acide chlorhydrique dans l'éthanol (20 ml), on ajoute 1,63 g (2,35 10⁻² mole) d'hydrochlorure d'hydroxylamine. Le mélange est porté à reflux pendant six heures. En fin de réaction le mélange est neutralisé avec une solution éthanolique de 1% en masse d'hydrogénocarbonate de sodium. Après extraction à l'éther éthylique et concentration, le mélange des deux isomères est séparé sur colonne de 25cm de silice (Si 60, 40-63 µm, Merck®) par élution à l'aide d'un mélange acétate d'éthyle 10/ éther de pétrole 90 ; V/V. Les produits (22) (R'_{F} = C₅F₁₁, R = p-OMe, R⁵ = H) sont obtenus avec un rendement de 68/32 (isomères Z/E respectivement).

Les produits finaux (22) obtenus sont chromatographiés sur plaque préparative de silice de 2mm d'épaisseur avec un mélange éluant : acétate d'éthyle 20/ éther de pétrole 80 ; V/V, et montrent un seul produit pour chaque isomère possédant un RF de 0,6 pour l'isomère Z et 0,71 pour l'isomère E.

### Caractéristiques:

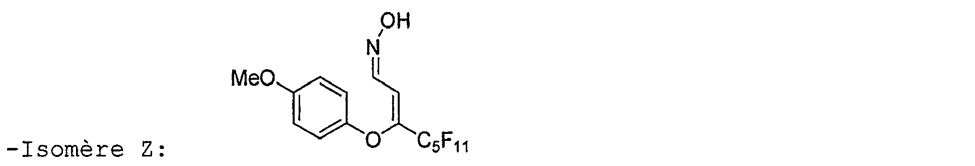

RMN ¹H (300. 13 MHZ, CDCl₃) : δ 3,8 (s, 3H); 5,8 (d, J = 10,5 Hz, 1H); 6,9 (m, 2H); 7 (m, 2H); 8,1 (d, J = 10,5 Hz, 1H); 8,5 (s, 1H, OH).
RMN ¹⁹F (300.13 MHZ, CDCl₃): δ -127 (t, 2F); -123 (m, 4F); -115 (m, 2F); -81 (t, 3F).
MS (FAB⁺, GT): [M+H⁺]⁺ = 462.
Spectroscopie de masse haute résolution : masse calculée = 461,0485 ; masse obtenue = 461,0487. 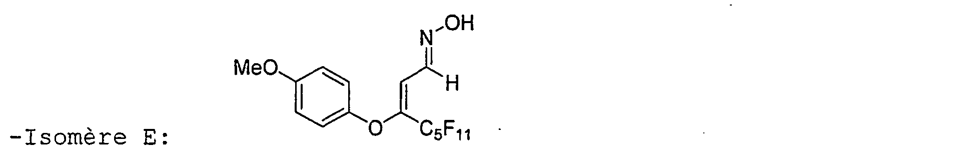 RMN ¹H (300.13 MHZ, CDCl₃): δ 3,8 (s, 3H); 6,6 (d, J = 10 Hz, 1H); 7,1 (d, J = 9,45 Hz, 1H); 7,2 (d, J = 9,5 Hz, 1H); 7,8 (d, J = 10 Hz, 1H); 8,4 (s, 1H, OH).
RMN ¹⁹F (300.13 MHZ, CDCl₃): δ -127 (t, 2F); -123,3 (m, 4F); -115,1 (m, 2F); -81,5 (t, 3F).
MS (FAB⁺, GT): [M+H⁺]⁺ = 462.
Spectroscopie de masse haute résolution : masse calculée = 46.

### Exemple 18-1

### Cyclisation du composé (22) (R'_{F} = C₅F₁₁, R = p-OMe) en présence de pyridine dans le toluène à reflux.

Dans un ballon contenant 2g (4,33 10⁻³ mole) du composé (22) préparé selon l'exemple 17-1 (R'_{F} = C₅F₁₁, R = p-OMe) dans le toluène (4ml), on ajoute 3ml de pyridine. Le mélange est mis à reflux et sous agitation magnétique pendant 12 heures. En fin de réaction de l'eau est additionnée (10ml) et le mélange est extrait à l'éther éthylique. Les phases organiques sont rassemblées puis concentrées sous pression réduite (20 mmHg). Le liquide obtenu est chromatographié sur colonne de 5cm de silice (Si 60, 40-63 µm, Merck^{®}) avec un mélange éluant : acétate d'éthyle 15/ éther de pétrole 85 ; V/V, pour obtenir l'oxazole (23) (R'_{F} = C₅F₁₁) avec un rendement de 80%.

Le composé (23) obtenu est chromatographié sur plaque préparative de silice de 2mm d'épaisseur, avec un mélange éluant : acétate d'éthyle 20/ éther de pétrole 80 ; V/V, pour donner un seul produit possédant un RF = 0,8.

### Caractéristiques :

RMN ¹⁹F (300.13 MHz, CDCl₃): δ ppm -126,5 (t, 2F); -122,1 (m, 4F); -114 (t, 2F); -81 (t, 3F).
RMN ¹H (300.13 MHz, CDCl₃): δ ppm 6,9 (d, J = 9 Hz, 1H); 8,3 (d, J = 9,1 Hz, 1H).
MS (FAB⁺, GT) : [M+H⁺]⁺ = 337.
Spectroscopie de masse haute résolution : masse calculée = 336,9961 ; masse obtenue = 337,0102.

### Exemple 19-1

### Réaction du 1-perfluoropentyl-1-(4-methoxyphénoxy)-propénal (1) (R'_{F} = C₅F₁₁, R = p-OMe) avec le triéthyl-orthoformiate (R⁶ = Et).

Dans un ballon on mélange 3,6 g (8 10⁻³ mole) du composé (1) préparé selon l'exemple 1-1 (R'_{F} = C₅F₁₁, R = p-OMe) et 3,6 g (2,4 10⁻² mole) de triéthyl-orthoformiate dans l'éthanol (7 ml). Puis on ajoute 1,39 g (8.0⁻³ mole) d'acide para-toluènesulfonique. Le mélange est porté à reflux pendant six heures. En fin de réaction le mélange est neutralisé avec une solution éthanolique de 2% en masse d'hydrogénocarbonate de sodium. Après extraction à l'éther éthylique le solvant est évaporé sous pression réduite (20mm Hg), et le produit obtenu (24) (R'_{F} = C₅F₁₁, R = p-OMe, R⁶ = Et) est utilisé sans purification particulière. Le rendement est supérieur à 95%.

### Caractéristiques:

RMN ¹H (300.13 MHZ, CDCl₃): δ ppm 1,1 (t, J = 7,05 Hz, 6H); 3,4 (m, 2H); 3,6 (m, 2H); 3,7 (s, 3H); 5,1 (d, J = 7,5 Hz, 1H); 6,1 (d, J = 7,5 Hz, 1H); 6,8 (d, J = 12,8 Hz, 2H); 7 (d, J = 12,8 Hz, 1H).
RMN ¹⁹F (300.13 MHZ, CDCl₃): δ ppm -126,5 (t, 2F); -123 (t, 2F); -123,5 (t, 2F); -115 (t, 2F); -81 (t, 3F).
RMN ¹³C (300.13 MHZ, CDCl₃): δ ppm 16; 57,1; 62,1; 62,2; 97,3; 115,1; 116,9; 123,4.
MS (FAB⁺, GT): [M+H⁺]⁺ = 521.
Spectroscopie de masse haute résolution : masse calculée = 520,1108 ; masse obtenue = 520,1110.

### Exemple 20-1 :

### Réaction du 1-perfluoropropyl-1-(4-methoxyphénoxy)-propénal (1) (R'_{F} = C₃F₇, R = p-OMe) avec le bromure d'acyle (R⁶ = CH₃, X = Br).

Dans un ballon on dissout 6 g (1,73 10⁻² mole) du composé (1) préparé selon l'exemple 1-3 (R'_{F} = C₃F₇, R = p-OMe) dans le bromure d'acyle (5 ml). Le mélange est porté à 60°C pendant six heures. En fin de réaction le mélange est neutralisé avec une solution éthanolique de 2% en masse d'hydrogénocarbonate de sodium. Après extraction à l'éther éthylique, le solvant est évaporé sous pression réduite (20 mm Hg), et le produit obtenu (25) (R'_{F} = C₃F₇, R = p-OMe, R⁷ = Me) est utilisé sans purification particulière. Le rendement est supérieur à 95%.

### Caractéristiques:

RMN ¹H (300.13 MHZ, CDCl₃): δ ppm 2 (s, 6H); 3,8 (s, 3H); 6 (d, J = 7,2 Hz, 1H); 6,8 (d, J = 9,2 Hz, 1H); 7 (d, J = 9,2 Hz, 1H); 7,3 (dd, J = 7,2 Hz et 1,5 Hz).
RMN ¹⁹F (300.13 MHZ, CDCl₃): δ ppm -126.5 (t, 2F); -116 (q, 2F); -81 (t, 3F).
MS (FAB⁺, GT) : [M+H⁺]⁺ = 449.
Spectroscopie de masse haute résolution : masse calculée = 448,0757 ; masse obtenue = 448,0759.

### Exemple 21-1

### Réaction du 1-perfluoropentyl-1-(4-méthoxyphénoxy)-propénal (1) (R'_{F} = C₅F₁₁, R = p-OMe) avec le triméthyl-phosphonoacétate (R⁸ = Me).

Dans un ballon on mélange 2,25 g (1,23 10⁻² mole) de triméthyl-phosphonoacétate (R⁸ = Me) et 0,35 g d'hydrure de sodium (1,47.10⁻² mole) dans le méthanol (8 ml). Puis on ajoute 5,5 g (1,23 10⁻² mole) du composé (1) préparé selon l'exemple 1-1 (R'_{F} = C₅F₁₁, R = p-OMe) et on laisse sous agitation à température ambiante (20°C) pendant deux heures. Le mélange réactionnel est ensuite extrait à l'éther éthylique. Le solvant est évaporé sous pression réduite (20mm Hg), et le produit final (26) (R'_{F} = C₅F₁₁, R = p-OMe, R⁸ = Me) est obtenu sans purification particulière. Le rendement est supérieur à 95%.

### Caractéristiques:

RMN ¹H (400.13 MHz, CDCl₃): δ ppm 3,6 (s, 3H); 3,8 (s, 3H); 5,68 (d, J = 15 Hz, 1H); 5,7 (d, J = 12 Hz, 1H); 6,9 (m, 4H); 7,5 (dd, J = 14,9 Hz et 12,3 Hz, 1H).
RMN ¹³C (400 MHz, CDCl₃): δ ppm 52; 55,9; 112,6; 115,6; 122,1; 124,5; 135,9 (q, CH, ⁵J_{CF} = 6,2 Hz); 146,7; 149,7 (q, C-CF₂, ³J_{CF} = 25,8 Hz); 157,9; 166,8.
RMN ¹⁹F (250 MHz, CDCl₃): δ -126,5 (s, 2F); -123 (m, 4F); -113,1 (t, 2F); -81 (t, 3F).
MS (FAB⁺, GT) : [M+H⁺]⁺ = 503.
Spectroscopie de masse haute résolution : masse calculée = 502,0638 ; masse obtenue = 502,0642.

### Exemple 22-1

### Oxydation du 1-perfluoropentyl-1-(4-methoxyphénoxy)-propénal (1) (R'_{F} = C₅F₁₁, R = p-OMe) avec le chlorite de sodium et le peroxyde d'hydrogène.

On dissout 10,7 g (2,4 10⁻² mole) du composé (1) préparé selon l'exemple 1-1 (R'_{F} = C₅F₁₁, R = p-OMe) dans un mélange acétonitrile/eau ; 80/20 ; V/V (15ml), puis on ajoute 0,42g d'hydrogénophosphate de sodium et ensuite de 2,2 g (2,4 10⁻² mole) de chlorite de sodium. Le mélange est mis sous agitation magnétique vigoureuse à température ambiante (20°C) puis additionné de 1,5ml de peroxyde d'hydrogène. L'évolution de la réaction est suivie par chromatographie sur couche mince avec un mélange éluant : éther de pétrole 50/ acétate d'éthyle 50 ; V/V. Après douze heures d'agitation, de l'eau est additionnée (20ml) et le mélange est extrait 3 fois à l'éther éthylique. Les phases organiques sont rassemblées puis concentrées sous pression réduite (20 mmHg).

Le produit final (27) (R'_{F} = C₅F₁₁, R = p-OMe) est obtenu sous forme de deux isomères (Z et E) avec un rendement supérieur à 95%.

Le produit final (27) obtenu est chromatographié sur plaque préparative de silice de 2mm d'épaisseur avec un mélange éluant : acétate d'éthyle 60/ éther de pétrole 40 ; V/V, et montre un seul produit possédant un RF de 0,5.

### Caractéristiques:

RMN ¹H (300.13 MHZ, CDCl₃): δ 3,8 (s, 3H); 5,4 (s, 1H); 6,2 (s, 1H); 6,8(d, J = 9,3 Hz, 1H); 7 (d, J = 9,3 Hz, 1H) 8,5 (s, 1H, COOH).
RMN ¹⁹F (300.13 MHZ, CDCl₃): δ -127 (t, 2F); -123 (m, 2F); -122,1 (t, 2F); -116 (t, 2F) (65%); -113,2 (s, 2F) (35%); -81 (t, 3F).
RMN ¹³C (300.13 MHZ, CDCl₃): 55,6; 103,6; 110,3 (q, CH, ³J_{CF} = 5,3 Hz); 114,5; 115,4; 117,2; 121,8; 145,5; 150,2; 152 (q, C-CF₃, ²J_{CF} = 24,9 Hz); 155 (q, C-CF3, ²J_{CF} = 27,9 Hz); 157,9; 166,9; 168,7; 209.
MS (FAB⁺, GT): [M+H⁺]⁺ = 463.
Spectroscopie de masse haute résolution : masse calculée = 462,0325 ; masse obtenue = 462,0319.

### Exemple 23-1

### Cyclisation du composé (27) (R'_{F} = C₅F₁₁, R = p-OMe) en présence d'acide polyphosphorique

Dans un ballon contenant 2 g (4,33 10⁻³ mole) du composé (27) préparé selon l'exemple 22-1 (R'_{F} = C₅F₁₁, R = p-OMe) on ajoute 0,5 g d'acide polyphosphorique. Le mélange est mis sous agitation magnétique pendant 12 heures à 80°C. En fin de réaction de l'eau est additionnée (10ml) et le mélange est extrait à l'éther éthylique. Les phases organiques sont rassemblées puis concentrées sous pression réduite (20 mmHg). On obtient la chromone (28) (R'_{F} = C₅F₁₁, R = 6-OMe) avec un rendement de 92%.

Le composé (28) obtenu est chromatographié sur plaque préparative de silice de 2mm d'épaisseur, avec un mélange éluant : acétate d'éthyle 15/ éther de pétrole 85 ; V/V, pour donner un seul produit possédant un RF = 0,8.

### Caractéristiques :

RMN ¹⁹F (300.13 MHz, CDCl₃): δ ppm -126,5 (t, 2F); -123 (t, 2F); -122,2 (t, 2F); -118 (t, 2F); -81 (t, 3F).
RMN ¹H (300.13 MHz, CDCl₃): δ ppm 3,9 (s, 3H); 6,8 (s, 1H); 7,4 (dd, J = 9,2 Hz et 3 Hz, 1H); 7,5 (d, J = 9,2 Hz, 1H); 7,6 (d, J = 3 Hz, 1H).
MS (FAB⁺, GT) : [M+H⁺]⁺ = 445.
Spectroscopie de masse haute résolution : masse calculée = 444,022 ; masse obtenue = 444,0221.

### Exemple 24-1

### Réaction de l'acide 1-perfluoropentyl-1-(4-méthoxyphénoxy)-propénoïque (27) (R'_{F} = C₅F₁₁, R = p-OMe) avec l'urée.

Dans un ballon contenant 1,5g (3,24 10⁻³ mole) du composé (27) (R'_{F} = C₅F₁₁, R = p-OMe) dans une solution de 1% d'acide sulfurique dans l'éthanol (3 ml), on ajoute 0,2g (3,24 10⁻³ mole) d'urée. Le mélange est porté à reflux pendant deux jours. En fin de réaction le mélange est neutralisé avec une solution éthanolique de 1% en masse d'hydroxyde de sodium. Après extraction à l'éther éthylique et concentration, le mélange réactionnel est chromatographié sur colonne de 2cm de silice (Si 60, 40-63 µm, Merck®) avec un mélange éluant : acétate d'éthyle 15/ éther de pétrole 85 ; V/V, pour obtenir la 2,4-dioxopyrimidine (29) (R'_{F} = C₅F₁₁, Y = O) avec un rendement de 75%.

Le composé (29) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,62.

### Caractéristiques :

RMN ¹⁹F (300 MHz, CDCl₃): δ ppm -126 (t, 2F); -123 (t, 2F); -122,2 (t, 2F); -117,8 (t, 2F); -81 (t, 3F).
RMN ¹H (300 MHz, CDCl₃): δ ppm 5,8 (s, 1H); 6,5 (s, 1H, NH); 11,5 (s, 1H, NH).
MS (FAB⁺, GT) : [M+H⁺]⁺ = 381.
Spectroscopie de masse haute résolution : masse calculée = 380,0019 ; masse obtenue = 380,0101.

### Exemple 25-1

### Réaction de l'oxime (22) (R'_{F} = C₅F₁₁, R = p-OMe) avec le chlorure de phosphore (v).

Dans un ballon contenant 2 g (4,33 10⁻³ mole) du composé (22) préparé selon l'exemple 17-1 (R'_{F} = C₅F₁₁, R = p-OMe) dans l'éther éthylique (3 ml), on ajoute 0,89g (4,33 10⁻³ mole) de chlorure de phosphore (v). Le mélange est mis sous agitation magnétique pendant deux heures. En fin de réaction le mélange est lavé 3 fois à l'eau (10ml). Après extraction à l'éther éthylique et concentration, on obtient le phénoxy-acrylonitrile (30) (R'_{F =} C₅F₁₁, R = p-OMe) avec un rendement supérieur à 98%.

Le composé (30) obtenu est chromatographié sur plaque préparative de silice de 2mm d'épaisseur, avec un mélange éluant : acétate d'éthyle 10/ éther de pétrole 100 ; V/V, pour donner un seul produit possédant un RF = 0,85.

### Caractéristiques :

RMN ¹⁹F (300 MHz, CDCl₃): δ ppm -126,1 (t, 2F); -123 (t, 2F); -122,2 (t, 2F); -117,5 (t, 2F); -81 (t, 3F).
RMN ¹H (300 MHz, CDCl₃): δ ppm 3,5 (s, 3H); 5,6 (s, 1H); 7 (s, 4H).
MS (FAB⁺, GT) : [M+H⁺]⁺ = 444.
Spectroscopie de masse haute résolution : masse calculée = 443,0379 ; masse obtenue = 443,0382.

## Revendications

1. Composé de formule (1) 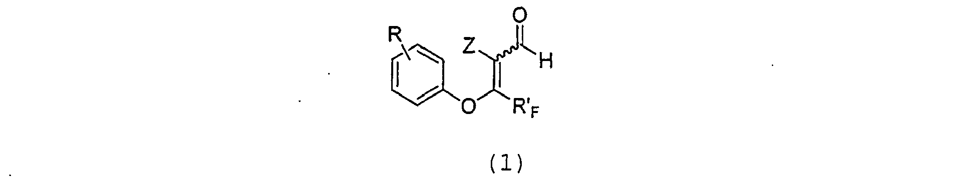 dans laquelle
- Z représente un atome d'hydrogène ou un atome d'halogène;
- R'_{F} représente un groupe perfluoroalkyle CₙF₂ₙ₊₁ où n est un nombre entier pouvant être compris entre 1 et 12 ;
- R est en position ortho, méta ou para, et il représente un groupe électrodonneur, un groupe électroattracteur, un groupe aryle, ou un groupe hétéroaryle, étant entendu que si R est un aryle ou un hétéroaryle, il peut former un groupe aromatique à noyaux condensés avec le groupe phényle qui le porte, ou bien R est un atome d'hydrogène.

2. Composé selon la revendication 1, **caractérisé en ce que** R est un groupe électrodonneur choisi parmi les groupes alkyle comprenant de 1 à 6 atomes de carbone ou les groupes alcoxy comprenant de 1 à 6 atomes de carbone, ou le groupe OH.

3. Composé selon la revendication 1 , **caractérisé en ce que** R représente le groupe méthoxy.

4. Composé selon la revendication 1, **caractérisé en ce que** R est un groupe électroattracteur choisi parmi :
- les groupes NO₂ et nitrile,
- les atomes d'halogène,
- un groupe carbonyle -C(=O)-R₉ dans lequel R₉ est un atome d'hydrogène, un groupe OH, un groupe alkyle, un groupe alkoxy, un groupe aryle, un groupe aryloxy, un groupe hétéroaryle, ou un groupe hétéroaryloxy, ledit groupe carbonyle étant fixé sur le groupe qui le porte soit directement, soit par l'intermédiaire d'un groupe alkylène, d'un groupe arylène, d'un groupe hétéroarylène ;
- un groupe -O-C(=O)-R₁₀ dans lequel R₁₀ est un atome d'hydrogène, un groupe alkyle, un groupe aryle, un groupe hétéroaryle, ledit groupe -O-C(=O)-R₁₀ étant fixé sur le groupe qui le porte soit directement, soit par l'intermédiaire d'un groupe alkylène, d'un groupe arylène, d'un groupe hétéroarylène.

5. Composé selon l'une quelconque des revendications 1 à 4, **caractérisé en ce que** R'_{F} représente un groupe CₙF₂ₙ₊₁ où n est un nombre entier compris entre 1 et 8.

6. Composé selon l'une quelconque des revendications 1 à 5, **caractérisé en ce qu'**il correspond à l'isomère Z.

7. Composé selon l'une quelconque des revendications 1 à 5, **caractérisé en ce qu'**il correspond à l'isomère E.

8. Composé selon l'une quelconque des revendications 1 à 7, **caractérisé en ce que** Z représente un atome d'hydrogène (composé 1a).

9. Composé selon l'une quelconque des revendications 1 à 7, **caractérisé en ce que** Z représente un atome d'halogène (Composé 1b).

10. Procédé de préparation d'un composé 1a selon la revendication 8, **caractérisé en ce qu'**il consiste à faire réagir dans un solvant, un composé de formule (2) 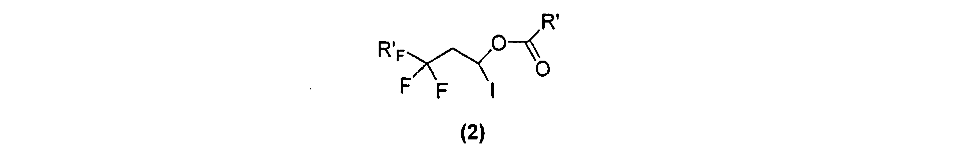 dans laquelle R'_{F} est tel que défini dans la revendication 1 et R' est un alkyle en C1 à C4 ou un aryle, avec un composé de formule (3) 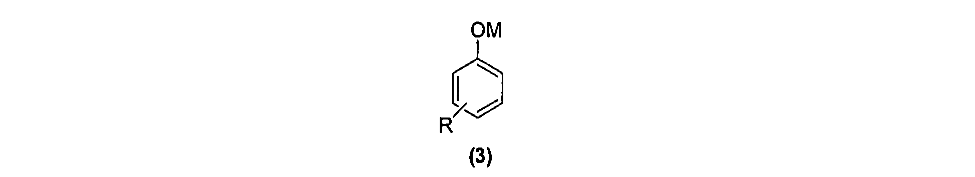 dans laquelle R est tel que défini dans la revendication 1 et M représente un métal alcalin.

11. Procédé selon la revendication 10, **caractérisé en ce que** R' représente un groupe méthyle, et M représente Na.

12. Procédé selon la revendication 10, **caractérisé en ce que** la réaction est mise en oeuvre dans les conditions suivantes :
- le rapport molaire entre le composé de formule (3) et le composé de formule (2) est compris entre 3 et 3,5,
- la température est comprise entre 0°C et 70°C ;
- le solvant est un alcane, ou un mélange d'alcanes liquides ayant une température d'ébullition inférieure à 100°C, ou un hydrocarbure aromatique, ou un éther de pétrole.

13. Procédé de préparation selon la revendication 12, **caractérisé en ce que** le rapport molaire entre le composé de formule (3) et le composé de formule (2) est égal à 3.

14. Procédé selon la revendication 12, pour la préparation d'un composé (1a) dans lequel R est un groupe électrodonneur, **caractérisé en ce que** la température de réaction est de l'ordre de 20°C.

15. Procédé selon la revendication 12, pour la préparation d'un composé (1a) dans lequel R est un groupe électroattracteur, **caractérisé en ce que** la température de réaction est de l'ordre de 70°C.

16. Procédé selon la revendication 10, **caractérisé en ce que** le composé (2) est préparé par réaction d'un composé de formule R'_{F}-CF₂I, dans laquelle R'_{F} est CₙF₂ₙ₊₁₋, n étant un nombre entier de 1 à 12, avec un composé CH₂=CH-O-CO-R' , en présence d'un initiateur radicalaire.

17. Procédé selon la revendication 16, **caractérisé en ce que** n est un nombre pair, et le composé R'_{F}-CF₂I est préparé par télomérisation de CF₃I avec le tétrafluoroéthylène.

18. Procédé selon la revendication 16, **caractérisé en ce que** n est impair et le composé R'_{F}-CF₂I est préparé par télomérisation de tétrafluoroéthylène avec IF₅.

19. Procédé de préparation d'un composé (1b) selon la revendication 9, **caractérisé en ce qu'**il consiste à faire subir une halogénation à un composé (1a) selon la revendication 8, dans le tétrachlorure de carbone et sous rayonnement ultraviolet.

20. Procédé de préparation d'une 2-perfluoro-alkylpyridine de formule (5) 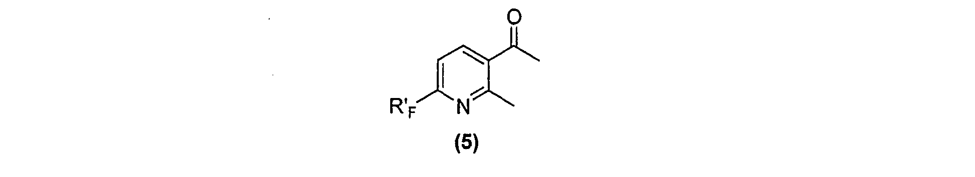 dans laquelle R'_{F} est tel que défini dans la revendication 1, à partir d'un composé de formule (1).

21. Procédé selon la revendication 20, **caractérisé en ce qu'**il consiste à faire réagir un composé (1) avec l'acétylacétone dans l'éthanol, en présence d'acétate d'ammonium et à température ambiante, pour obtenir le composé de formule (4) ci-dessous 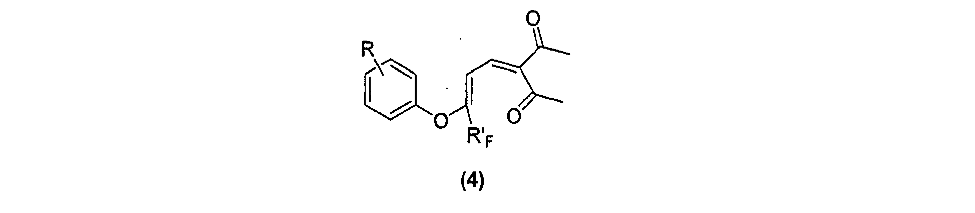 et **en ce qu'**on fait ensuite réagir ledit composé de formule (4) avec de l'ammoniac dans de l'éthanol.

22. Procédé de préparation d'une 2-perfluoroalkylquinoléine de formule (6) 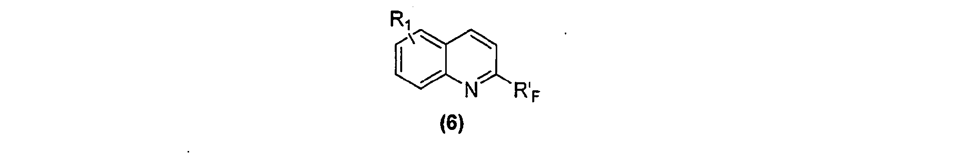 dans laquelle R'_{F} est tel que défini dans la revendication 1, et dans laquelle R₁ est tel que défini pour R dans la revendications 1, à partir d'un composé de formule (1).

23. Procédé selon la revendication 22, **caractérisé en ce que** R₁ représente un atome d'hydrogène, un groupe alkyle comportant de 1 à 6 atomes de carbone, un groupe aryle, un groupe hétéroaryle, un groupe alcoxy comportant de 1 à 6 atomes de carbone, un groupe aryloxy, un groupe hydroxyle, un groupe nitro, ou un atome d'halogène.

24. Procédé selon la revendication 22, **caractérisé en ce qu'**il consiste à faire réagir un composé de formule (1) avec une aniline dans du tétrahydrofuranne en présence d'acide formique, à une température comprise entre 60°C et 65°C.

25. Procédé de préparation d'un composé de formule (7) 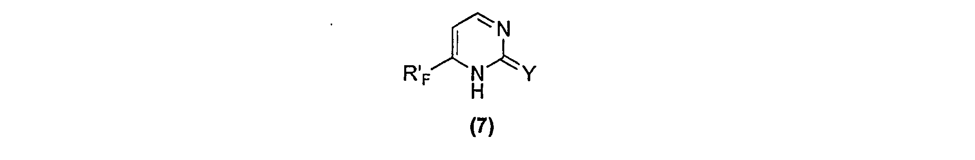 dans laquelle R'_{F} est tel que défini dans la revendication 1 et dans laquelle Y représente un atome d'oxygène ou un atome de soufre, à partir d'un composé de formule (1).

26. Procédé selon la revendication 25, **caractérisé en ce qu'**il consiste à faire réagir un composé de formule (1) avec un composé de formule (7') 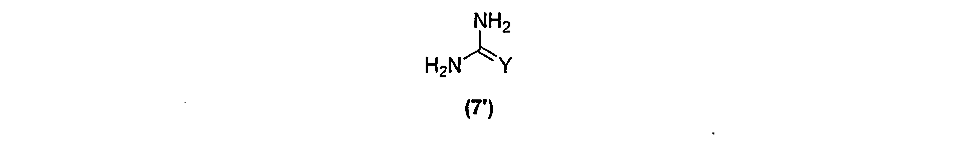 dans de l'éthanol, à une température comprise entre 78°C et 85°C, et en présence d'acide sulfurique ou d'acide chlorhydrique).

27. Procédé de préparation d'un composé de formule (8) 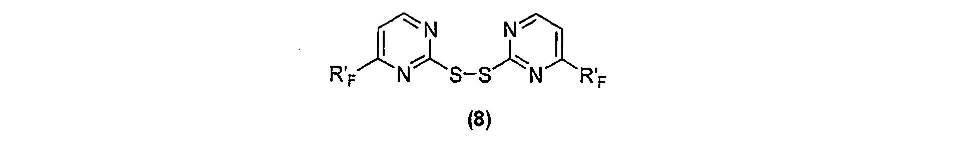 dans laquelle R'_{F} est tel que défini dans la revendication 1, par dimérisation à l'air libre d'un composé de formule (7) obtenu selon le procédé de la revendication 25, dans lequel Y représente un atome de soufre.

28. Procédé de préparation d'un 2-perfluoroalkylchrom-3-ène-2-ol de formule (9) 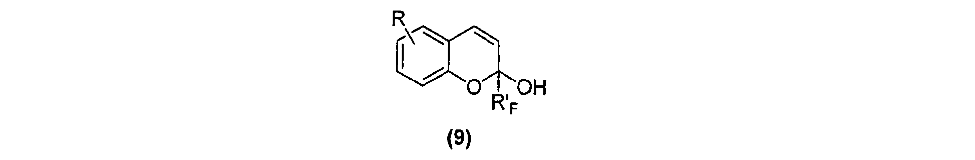 dans laquelle R'_{F} et R sont tels que définis dans la revendication 1, à partir d'un composé (1.

29. Procédé selon la revendication 28, **caractérisé en ce qu'**il consiste à faire réagir le composé de formule (1) avec un acide de Lewis dans du 1,2-dichloroéthane ou du dichlorométhane à une température comprise entre 85°C et 90°C.

30. Procédé de préparation d'un composé de formule (10) 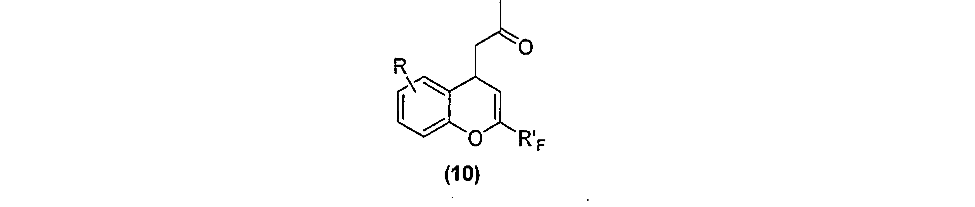 dans laquelle R'_{F} et R sont tels que définis dans la revendication 1, à partir d'un composé de formule (9) obtenu par le procédé selon la revendication 28.

31. Procédé selon la revendication 30, **caractérisé en ce qu'**il consiste à faire réagir le composé de formule (9) avec du 2-(triméthylsilyloxy)-propène dans un solvant alcane ou un mélange d'alcane, en présence de SnCl₄ à une température comprise entre -30°C et -20°C.

32. Composé de formule (10) 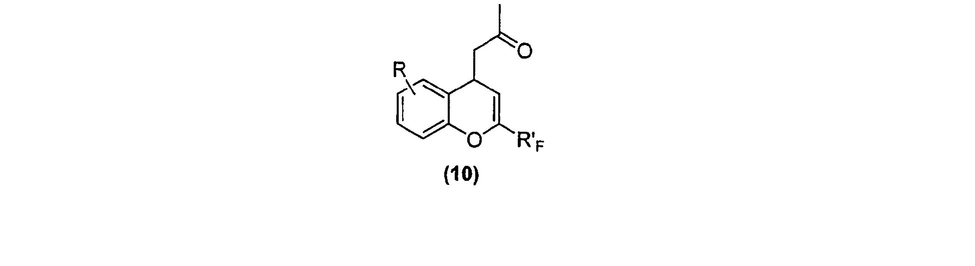 dans laquelle R et R'_{F} sont tels que définis dans la revendication 1.

33. Procédé de préparation d'un composé de formule (11) 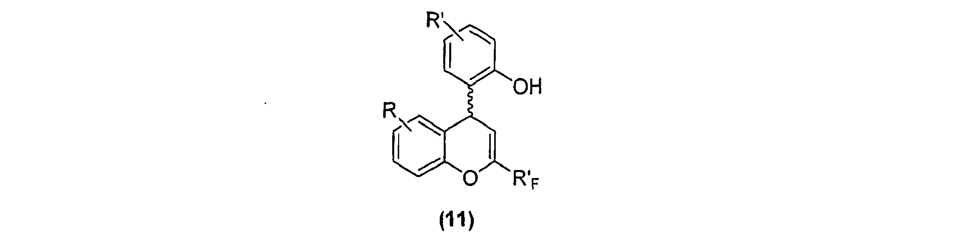 dans laquelle R et R'_{F} sont tels que définis dans la revendication 1, et dans laquelle R' est tel que défini dans la revendication 10, à partir d'un composé de formule (9) obtenu par un procédé selon la revendication 28.

34. Procédé selon la revendication 33, **caractérisé en ce qu'**il consiste à faire réagir le composé de formule (9) avec du 4-méthoxyphénol dans du tétrahydrofuranne en présence d'acide formique, à une température comprise entre 0°C et 20°C.

35. Composé de formule (11) 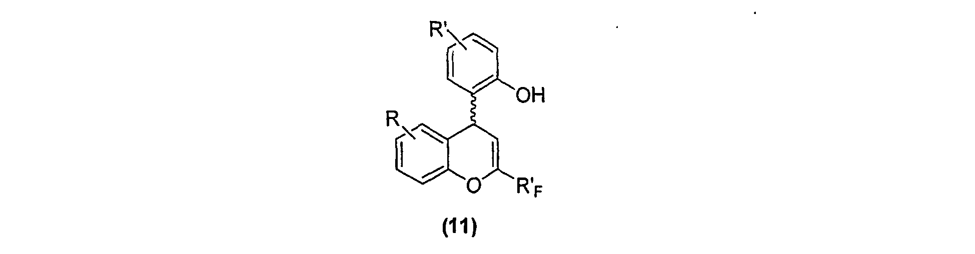 dans laquelle R et R'_{F} sont tels que définis dans la revendication 1 et dans laquelle R' est tel que défini dans la revendication 10.

36. Procédé de préparation d'un composé de formule (12) 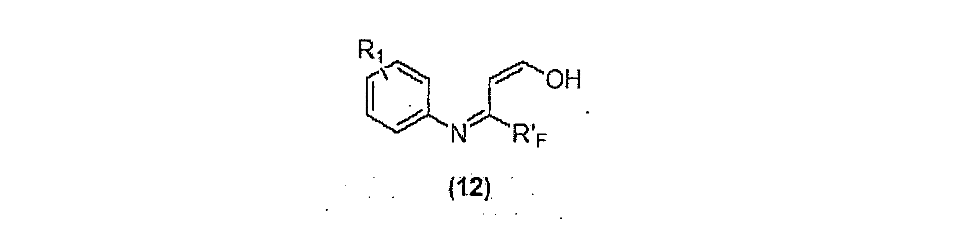 dans laquelle R'_{F} est tel que défini dans la revendication 1, et R₁ est tel que défini dans la revendication 23, à partir d'un composé (1) .

37. Procédé selon la revendication 36, **caractérisé en ce qu'**il consiste à faire réagir le composé (1) avec une aniline dans du méthanol, en présence d'acide chlorhydrique.

38. Procédé de préparation d'un composé de formule (13) 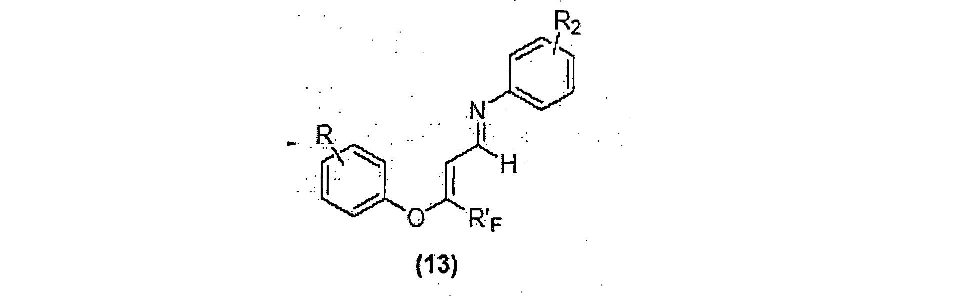 dans laquelle R'_{F} est tel que défini dans la revendication 1, et dans laquelle R₂ est tel que défini pour R₁ dans la revendication 22, à partir d'un composé (1).

39. Procédé selon la revendication 38, **caractérisé en ce qu'**il consiste à faire réagir le composé (1) avec une aniline dans du dichlorométhane.

40. Procédé de préparation d'un diazapentadiène dissymétrique de formule (14) 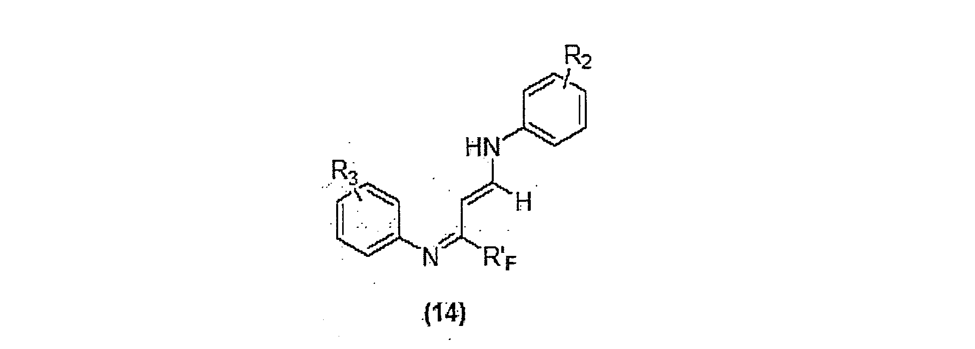 dans laquelle R'_{F} est tel que défini dans la revendication 1, et dans laquelle R₂ et R₃, indépendamment l'un de l'autre, sont tels queR₁ défini dans la revendication 22, à partir d'un composé (13) obtenu par le procédé selon la revendication 38.

41. Procédé selon la revendication 40, **caractérisé en ce qu'**il consiste à faire réagir le composé (13) avec une aniline dans du dichlorométhane.

42. Procédé de préparation d'un composé de formule (15) 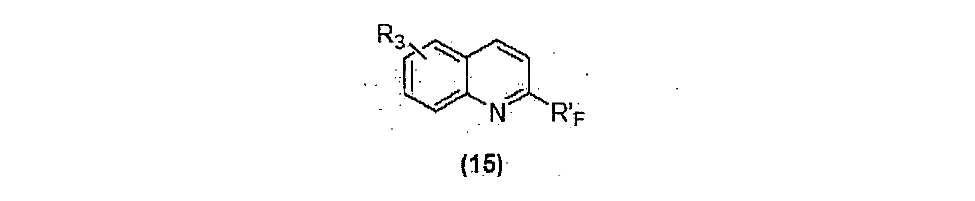 dans laquelle R'_{F} est tel que défini dans la revendication 1, et dans laquelle R₃ est tel que R₁ défini selon la revendications 22, à partir d'un composé (13) obtenu par le procédé selon la revendication 38.

43. Procédé selon la revendication 42, **caractérisé en ce qu'**il consiste à faire réagir le composé (13) avec une aniline dans du dichlorométhane à reflux.

44. Procédé de préparation d'un imino-énol de formule (16) 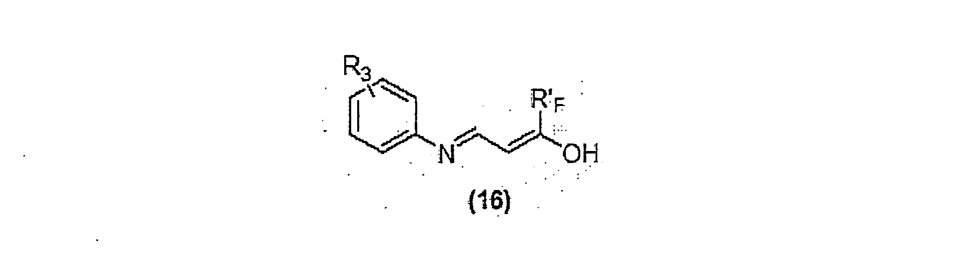 dans laquelle R'_{F} est tel que défini dans la revendication 1, et dans laquelle R₃ est tel que R₁ défini selon la revendications 22, à partir d'un composé (1.

45. Procédé selon la revendication 44, **caractérisé en ce qu'**il consiste à faire réagir le composé (1) avec une aniline en présence d'acide para-toluènesulfonique).

46. Procédé de préparation d'un composé de formule (18) 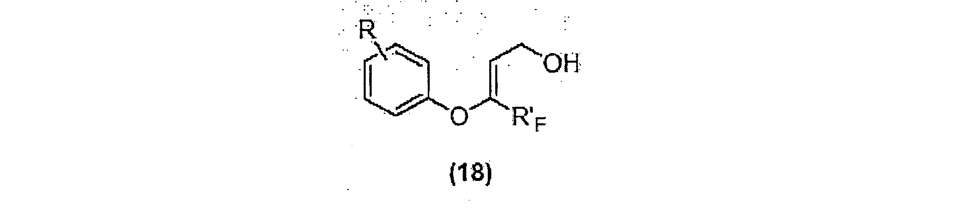 dans laquelle R et R'_{F} sont tels que définis dans la revendication 1, à partir d'un composé (1).

47. Procédé selon la revendication 46, **caractérisé en ce qu'**il consiste à faire réagir le composé (1) avec du borohydrure de sodium.

48. Procédé de préparation d'un composé de formule (19) 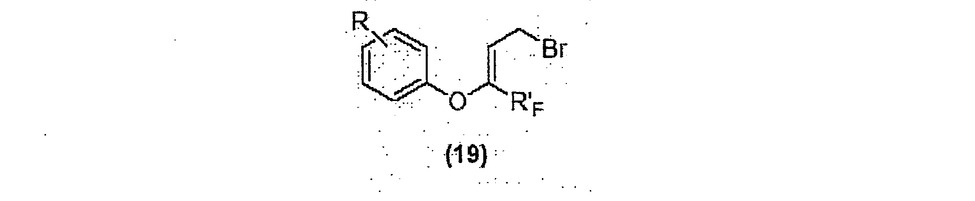 dans laquelle R et R'_{F} sont tels que définis dans la revendication 1, à partir d'un composé (18) obtenu par le procédé selon la revendication 46.

49. Procédé selon la revendication 48, **caractérisé en ce qu'**il consiste à faire réagir le composé (18) avec PX₃, dans lequel X représente un atome d'halogène.

50. Procédé de préparation d'un composé de formule (20) 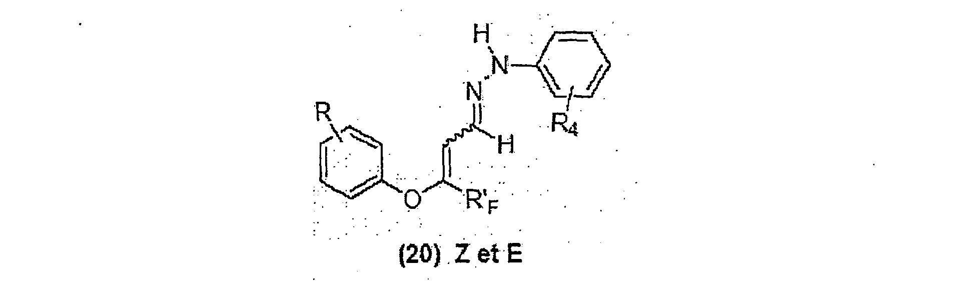 dans laquelle R et R'_{F} sont tels que définis dans la revendication 1, et dans laquelle R₄ est tel que R₁ défini dans la revendication 22, à partir d'un composé (1).

51. Procédé selon la revendication 50, **caractérisé en ce qu'**il consiste à faire réagir le composé (1) avec une phénylhydrazine.

52. Procédé de préparation d'un composé de formule (21) 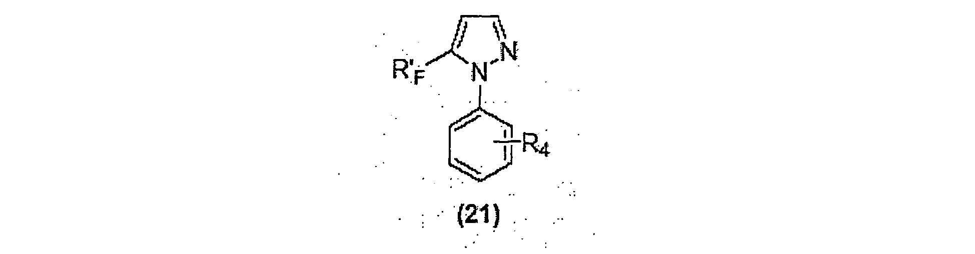 dans laquelle R'_{F} est tel que défini dans la revendication 1, et dans laquelle R₄ est tel R₁ défini dans la revendication 22, à partir d'un composé (20) obtenu par un procédé selon la revendication 50.

53. Procédé selon la revendication 52, **caractérisé en ce qu'**il consiste à cycliser le composé (20) en présence de pyridine dans le toluène à reflux.

54. Procédé de préparation d'un composé de formule (22) 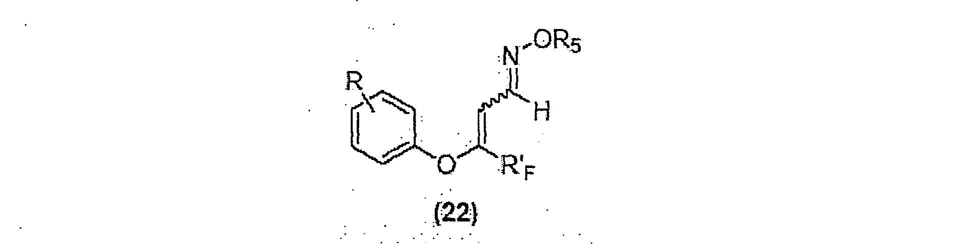 dans laquelle R et R'_{F} sont tels que définis dans la revendication 1, et dans laquelle R₅ est tel que R₁ défini selon la revendication 22, à partir d'un composé (1).

55. Procédé selon la revendication 54, **caractérisé en ce qu'**il consiste à faire réagir le composé (1) avec un composé de formule générale NH₂OR₅.

56. Procédé de préparation d'un composé de formule (23) 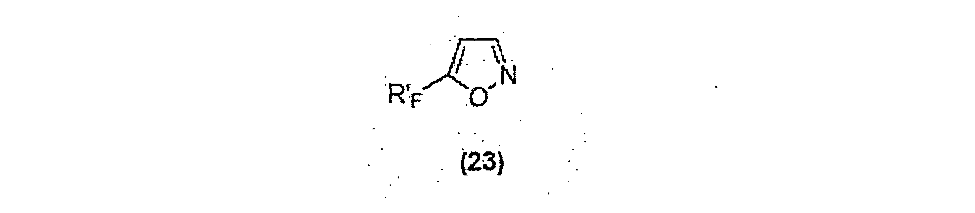 dans laquelle R'_{F} est tel que défini dans la revendication 1, à partir d'un composé (22) obtenu par le procédé selon la revendication 54, dans lequel R₅ est un atome d'hydrogène.

57. Procédé de préparation selon la revendication 56, **caractérisé en ce qu'**il consiste à cycliser un composé (22) dans lequel R₅ est un atome d'hydrogène, en présence de pyridine dans le toluène à reflux.

58. Procédé de préparation d'un composé de formule (24) 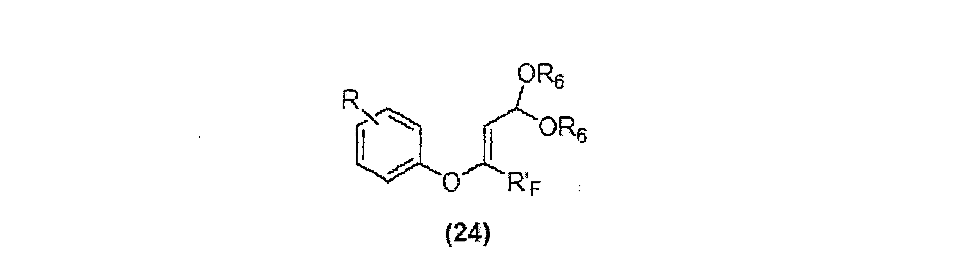 dans laquelle R et R'_{F} sont tels que définis dans la revendication 1, et dans laquelle R₆ est tel que R₁ défini selon la revendication 22, à partir d'un composé (1).

59. Procédé selon la revendication 58, **caractérisé en ce qu'**il consiste à faire réagir le composé (1) avec un composé de formule générale CH(OR₆)₃.

60. Procédé de préparation d'un composé de formule (25) 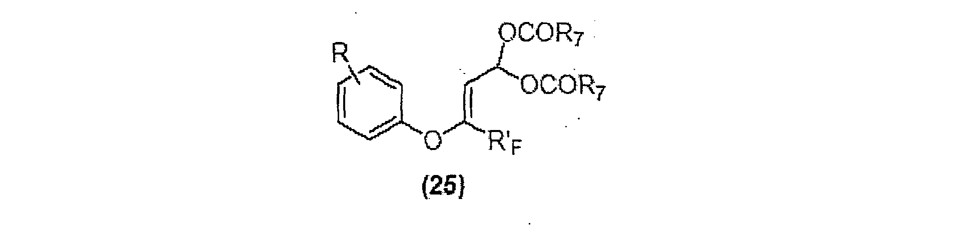 dans laquelle R et R'_{F} sont tels que définis dans la revendication 1, et dans laquelle R₇ est tel que R₁ défini selon la revendication 22, à partir d'un composé (1).

61. Procédé selon la revendication 60, **caractérisé en ce qu'**il consiste à faire réagir le composé (1) avec un composé de formule générale (R₇COX), dans laquelle X représente un atome d'halogène.

62. Procédé de préparation d'un composé de formule (26) 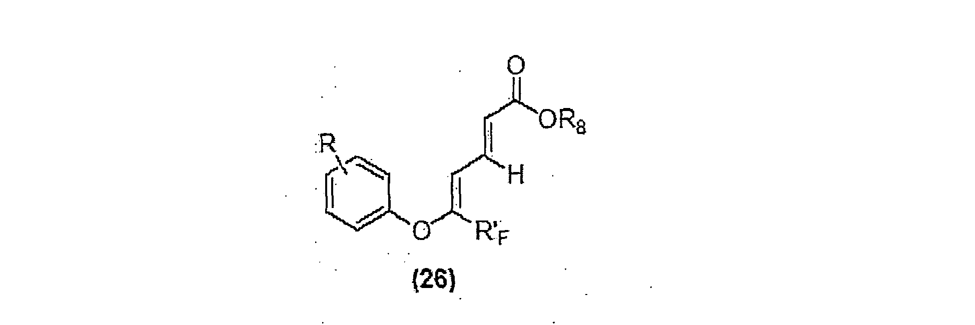 dans laquelle R et R'_{F} sont tels que définis dans la revendication 1, et dans laquelle R₈ est tel que R₁ défini selon la revendication 22, à partir d'un composé (1).

63. Procédé de préparation selon la revendication 62, **caractérisé en ce qu'**il consiste à faire réagir le composé (1) avec un composé de type phosphonoacétate.

64. Procédé de préparation d'un composé de formule (27) 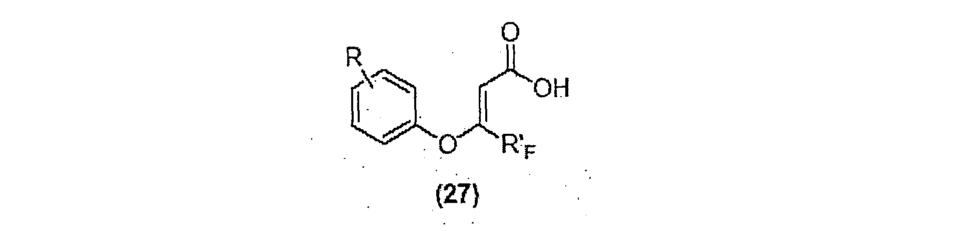 dans laquelle R et R'_{F} sont tels que définis dans la revendication 1, à partir d'un composé (1).

65. Procédé selon la revendication 64, **caractérisé en ce qu'**il consiste à faire réagir le composé (1) avec du chlorite de sodium et du peroxyde d'hydrogène.

66. Procédé de préparation d'un composé de formule (28) 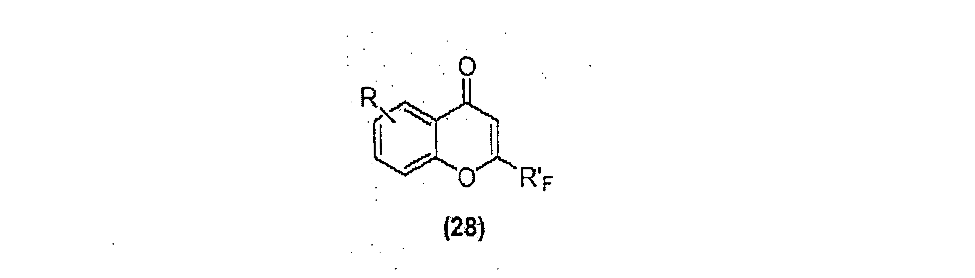 dans laquelle R et R'_{F} sont tels que définis dans la revendication 1, à partir d'un composé (27) obtenu par un procédé selon la revendication 64.

67. Procédé de préparation selon la revendication 66, **caractérisé en ce qu'**il consiste à cycliser le composé (27) en présence d'acide polyphosphorique.

68. Procédé de préparation d'un composé de formule (29) 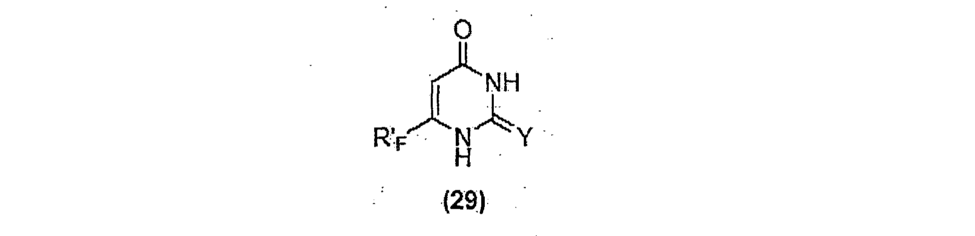 dans laquelle R'_{F} est tel que défini dans la revendication 1, et dans laquelle Y représente O ou S, à partir d'un composé (27) obtenu selon le procédé de la revendication 64.

69. Procédé selon la revendication 68, **caractérisé en ce qu'**il consiste à faire réagir le composé (27) avec l'urée lorsque Y est O, ou avec la thiourée lorsque Y est S.

70. Procédé de préparation d'un composé de formule (30) 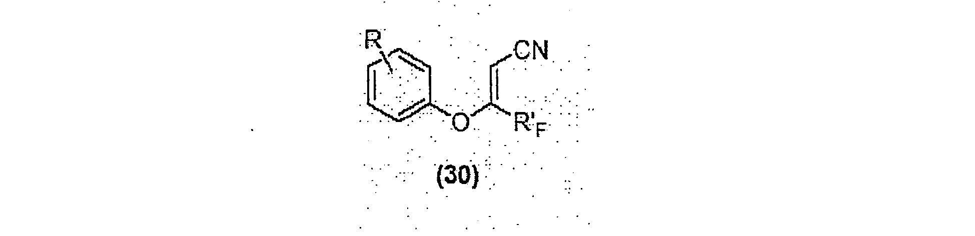 dans laquelle R et R'_{F} sont tels que définis dans la revendication 1, et dans laquelle Y est O ou S, à partir d'un composé (22) obtenu par un procédé selon la revendication 54.

71. Procédé selon la revendication 70, **caractérisé en ce qu'**il consiste à faire réagir le composé (22) avec un composé de formule (PX₅) dans laquelle X représente un atome d'halogène.

## Claims

1. Compound having formula (1) 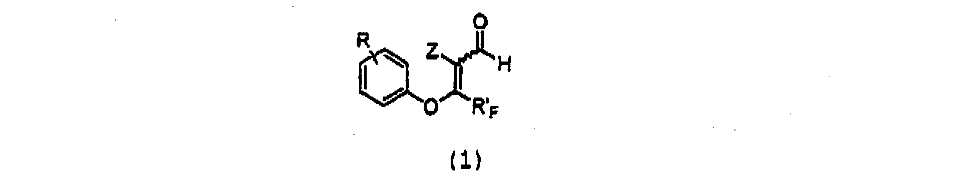 in which
- Z represents a hydrogen atom or a halogen atom;
- R'_{F} represents a perfluoroalkyl group CₙF₂ₙ₊₁ where n is a whole number between 1 and 12;
- R is in ortho, meta or para position and represents an electron donor group, an electron attractor group, an aryl group or a heteroaryl group, it being understood that if R is an aryl or a heteroaryl it can form an aromatic group having condensed nodes with the phenyl group that bears it, or R is a hydrogen atom.

2. Compound according to claim 1, **characterised in that** R is an electron donor group chosen from alkyl groups containing 1 to 6 carbon atoms or alkoxy groups containing 1 to 6 carbon atoms or the OH group.

3. Compound according to claim 1, **characterised in that** R represents the methoxy group.

4. Compound according to claim 1, **characterised in that** R is an electron attractor group chosen from:
- NO₂ and nitrile groups,
- halogen atoms,
- a carbonyl group -C(=O)-R₉ in which R₉ is a hydrogen atom, an OH group, an alkyl group, an alkoxy group, an aryl group, an aryloxy group, a heteroaryl group or a heteroaryloxy group, said carbonyl group being fixed to the group that bears it either directly or by means of an alkylene group, an arylene group, a heteroarylene group;
- an -O-C(=O)-R₁₀ group in which R₁₀ is a hydrogen atom, an alkyl group, an aryl group, a heteroaryl group, said -O-C(=O)-R₁₀ group being fixed to the group that bears it either directly or by means of an alkylene group, an arylene group, a heteroarylene group.

5. Compound according to one of claims 1 to 4,
**characterised in that** R'_{F} represents a CₙF₂ₙ₊₁ group where n is a whole number between 1 and 8.

6. Compound according to one of claims 1 to 5,
**characterised in that** it corresponds to isomer Z.

7. Compound according to one of claims 1 to 5,
**characterised in that** it corresponds to isomer E.

8. Compound according to one of claims 1 to 7,
**characterised in that** Z represents a hydrogen atom (compound 1a).

9. Compound according to one of claims 1 to 7,
**characterised in that** Z represents a halogen atom (compound 1b).

10. Process for preparing a compound la according to claim 8, **characterised in that** it involves reacting in a solvent a compound having formula (2) 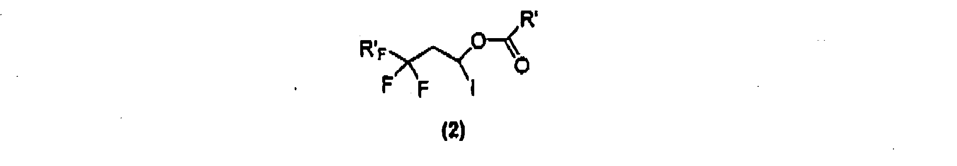 in which R'_{F} is as defined in claim 1 and R' is a C₁ to C₄ alkyl or an aryl, with a compound having formula (3) 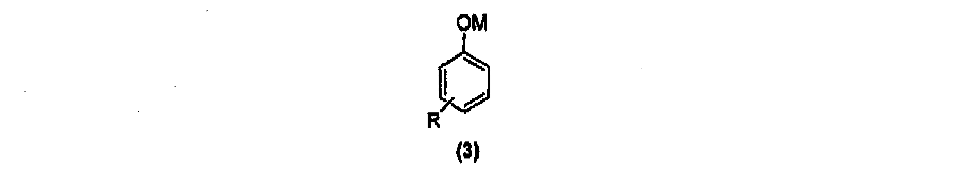 in which R is as defined in claim 1 and M represents an alkali metal.

11. Process according to claim 10, **characterised in that** R' represents a methyl group and M represents Na.

12. Process according to claim 10, **characterised in that** the reaction is performed in the following conditions:
- the molar ratio between the compound having formula (3) and the compound having formula (2) is between 3 and 3.5,
- the temperature is between 0°C and 70°C;
- the solvent is an alkane or a blend of liquid alkanes having a boiling temperature below 100°C or an aromatic hydrocarbon or a petroleum ether.

13. Preparation process according to claim 12,
**characterised in that** the molar ratio between the compound having formula (3) and the compound having formula (2) is equal to 3.

14. Process according to claim 12 for preparing a compound (1a) in which R is an electron donor group,
**characterised in that** the reaction temperature is in the region of 20°C.

15. Process according to claim 12 for preparing a compound (1a) in which R is an electron attractor group,
**characterised in that** the reaction temperature is in the region of 70°C.

16. Process according to claim 10, **characterised in that** the compound (2) is prepared by reacting a compound having formula R'_{F}-CF₂I, in which R'_{F} is CₙF₂ₙ₊₁₋, n being a whole number from 1 to 12, with a compound CH₂=CH-O-CO-R' in the presence of a radical initiator.

17. Process according to claim 16, **characterised in that** n is an even number and the compound R'_{F}-CF₂I is prepared by telomerising CF₃I with tetrafluoroethylene.

18. Process according to claim 16, **characterised in that** n is an odd number and the compound R'_{F}-CF₂I is prepared by telomerising tetrafluoroethylene with IF₅.

19. Process for preparing a compound (1b) according to claim 9, **characterised in that** it involves halogenating a compound (1a) according to claim 8 in carbon tetrachloride under ultraviolet radiation.

20. Process for preparing a 2-perfluoroalkyl pyridine having formula (5) 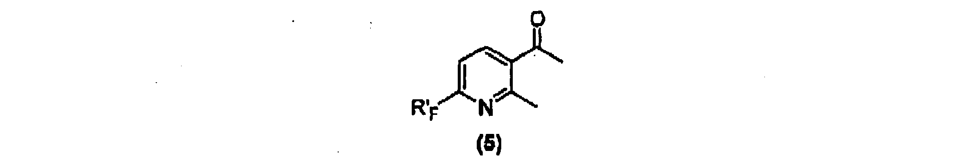 in which R'_{F} is as defined in claim 1, starting from a compound having formula (1).

21. Process according to claim 20, **characterised in that** it involves reacting a compound (1) with acetyl acetone in ethanol, in the presence of ammonium acetate and at ambient temperature, to obtain the compound having formula (4) below 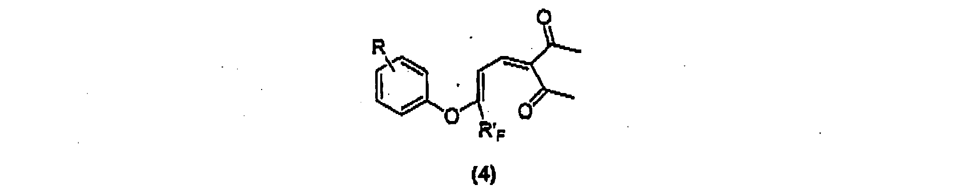 and **in that** said compound having formula (4) is then reacted with ammonia in ethanol.

22. Process for preparing a 2-perfluoroalkyl quinoline having formula (6) 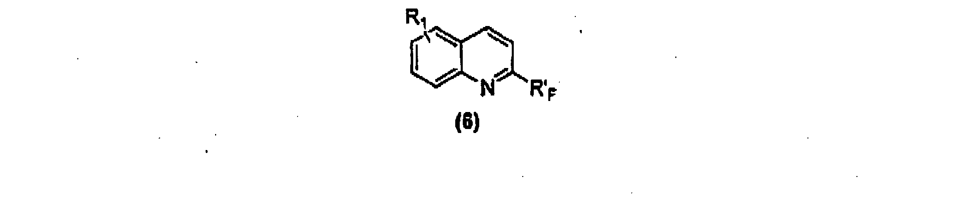 in which R'_{F} is as defined in claim 1, and in which R₁ is as defined for R in claim 1, starting from a compound having formula (1).

23. Process according to claim 22, **characterised in that** R₁ represents a hydrogen atom, an alkyl group having 1 to 6 carbon atoms, an aryl group, a heteroaryl group, an alkoxy group containing 1 to 6 carbon atoms, an aryloxy group, a hydroxyl group, a nitro group or a halogen atom.

24. Process according to claim 22, **characterised in that** it involves reacting a compound having formula (1) with an aniline in tetrahydrofuran in the presence of formic acid, at a temperature of between 60°C and 65°C.

25. Process for preparing a compound having formula (7) 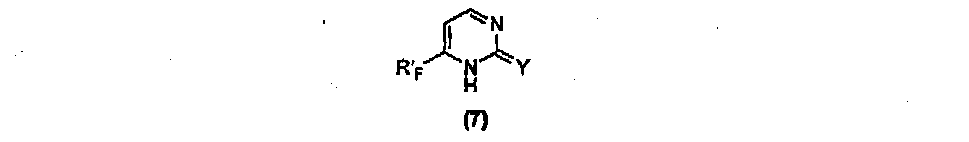 in which R'_{F} is as defined in claim 1 and in which Y represents an oxygen atom or a sulfur atom, starting from a compound having formula (1).

26. Process according to claim 25, **characterised in that** it involves reacting a compound having formula (1) with a compound having formula (7') 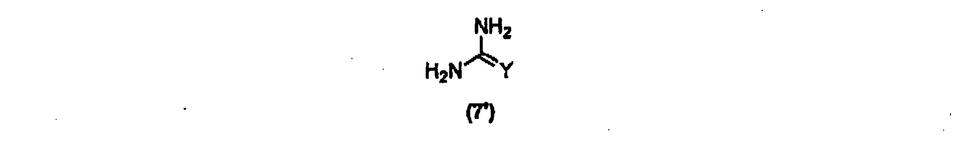 in ethanol, at a temperature of between 78°C and 85°C and in the presence of sulfuric acid or hydrochloric acid.

27. Process for preparing a compound having formula (8) 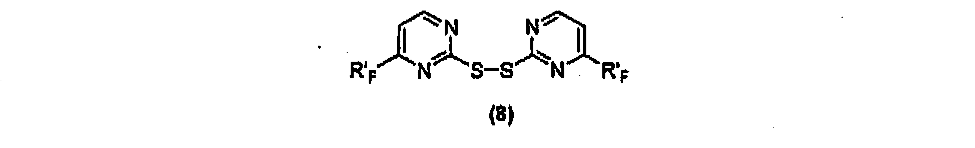 in which R'_{F} is as defined in claim 1, by dimerisation in the open air of a compound having formula (7) obtained according to the process of claim 25, in which Y represents a sulfur atom.

28. Process for preparing a 2-perfluoroalkyl chrom-3-en-2-ol having formula (9) 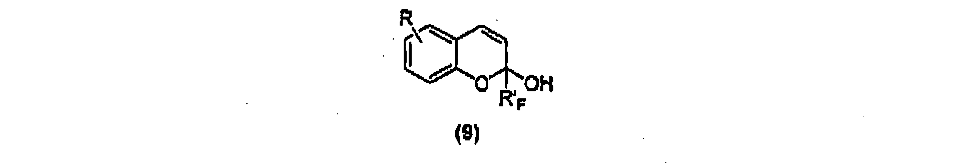 in which R'_{F} and R are as defined in claim 1, starting from a compound (1).

29. Process according to claim 28, **characterised in that** it involves reacting the compound having formula (1) with a Lewis acid in 1,2-dichloroethane or dichloromethane at a temperature of between 85°C and 90°C.

30. Process for preparing a compound having formula (10) 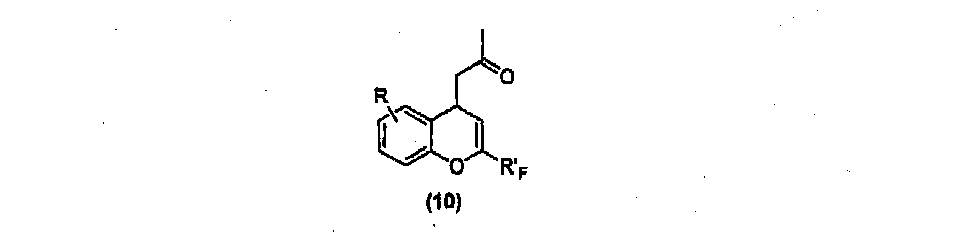 in which R'_{F} and R are as defined in claim 1, starting from a compound having formula (9) obtained by the process according to claim 28.

31. Process according to claim 30, **characterised in that** it involves reacting the compound having formula (9) with 2-(trimethyl silyloxy)propene in an alkane solvent or an alkane blend, in the presence of SnCl₄, at a temperature of between -30°C and -20°C.

32. Compound having formula (10) 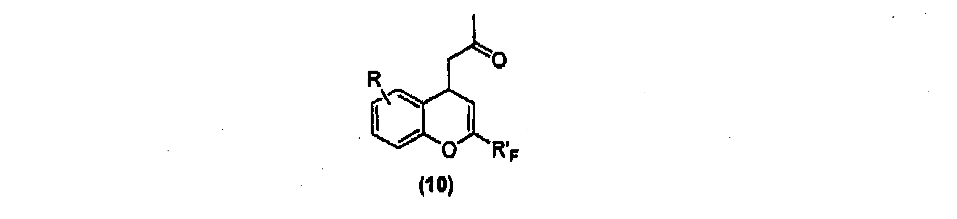 in which R and R'_{F} are as defined in claim 1.

33. Process for preparing a compound having formula (11) 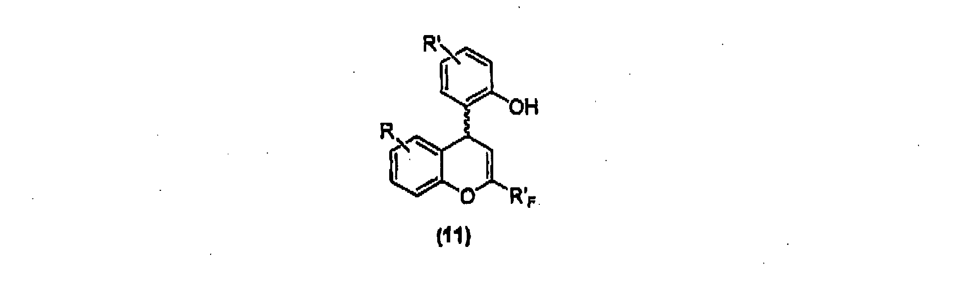 in which R and R'_{F} are as defined in claim 1 and in which R' is as defined in claim 10, starting from a compound having formula (9) obtained by a process according to claim 28.

34. Process according to claim 33, **characterised in that** it involves reacting the compound having formula (9) with 4-methoxyphenol in tetrahydrofuran in the presence of formic acid, at a temperature of between 0°C and 20°C.

35. Compound having formula (11) 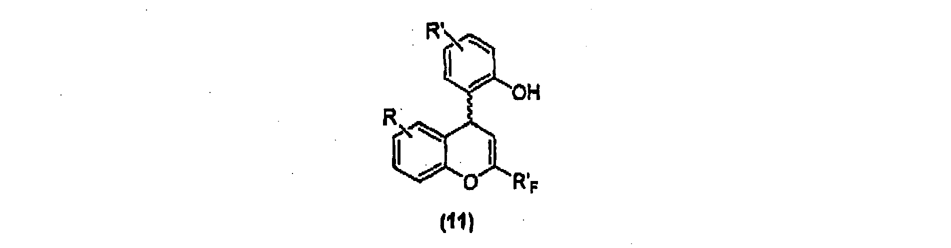 in which R and R'_{F} are as defined in claim 1 and in which R' is as defined in claim 10.

36. Process for preparing a compound having formula (12) 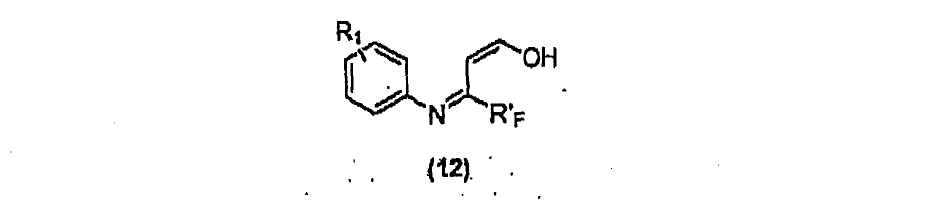 in which R'_{F} is as defined in claim 1 and R₁ is as defined in claim 23, starting from a compound (1).

37. Process according to claim 36, **characterised in that** it involves reacting the compound (1) with an aniline in methanol in the presence of hydrochloric acid.

38. Process for preparing a compound having formula (13) 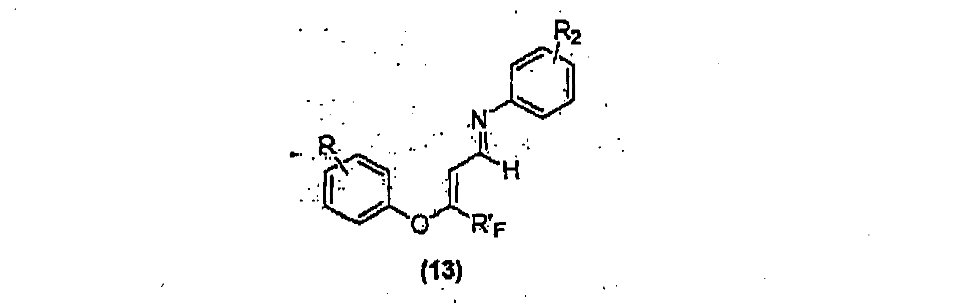 in which R'_{F} is as defined in claim 1 and in which R₂ is as defined for R₁ in claim 22, starting from a compound (1).

39. Process according to claim 38, **characterised in that** it involves reacting the compound (1) with an aniline in dichloromethane.

40. Process for preparing an asymmetrical diazapentadiene having formula (14) 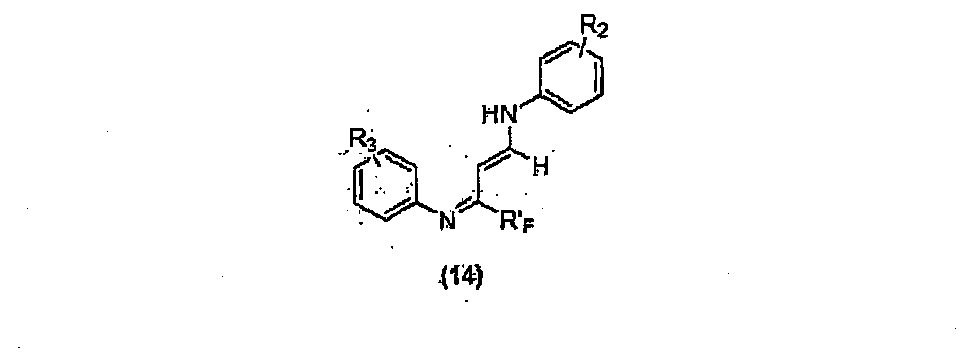 in which R'_{F} is as defined in claim 1 and in which R₂ and R₃ are mutually independently the same as R₁ defined in claim 22, starting from a compound (13) obtained by the process according to claim 38.

41. Process according to claim 40, **characterised in that** it involves reacting the compound (13) with an aniline in dichloromethane.

42. Process for preparing a compound having formula (15) 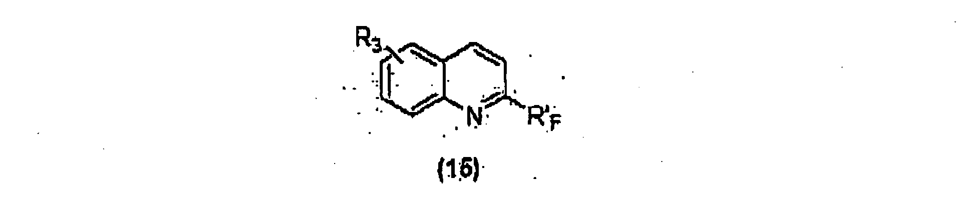 in which R'_{F} is as defined in claim 1 and in which R₃ is the same as R₁ defined according to claim 22, starting from a compound (13) obtained by the process according to claim 38.

43. Process according to claim 42, **characterised in that** it involves reacting the compound (13) with an aniline in refluxing dichloromethane.

44. Process for preparing an imino-enol having formula (16) 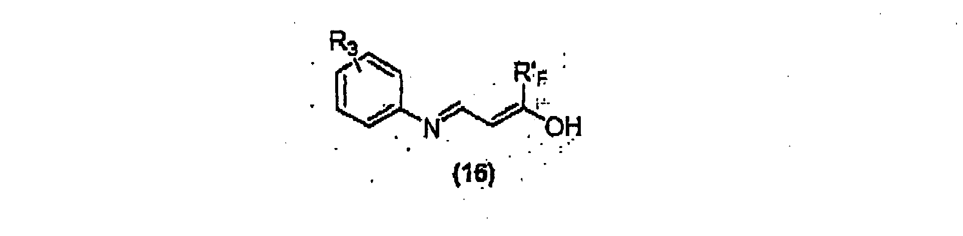 in which R'_{F} is as defined in claim 1 and in which R₃ is the same as R₁ defined according to claim 22, starting from a compound (1).

45. Process according to claim 44, **characterised in that** it involves reacting the compound (1) with an aniline in the presence of para-toluene sulfonic acid.

46. Process for preparing a compound having formula (18) 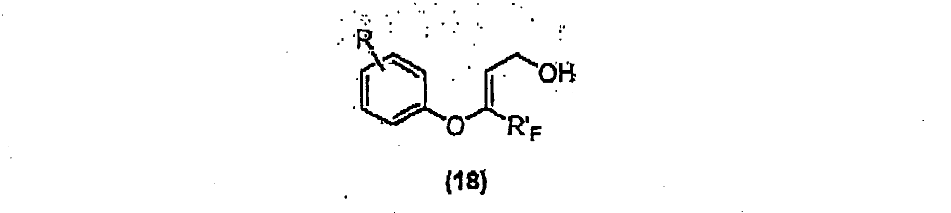 in which R and R'_{F} are as defined in claim 1, starting from a compound (1).

47. Process according to claim 46, **characterised in that** it involves reacting the compound (1) with sodium borohydride.

48. Process for preparing a compound having formula (19) 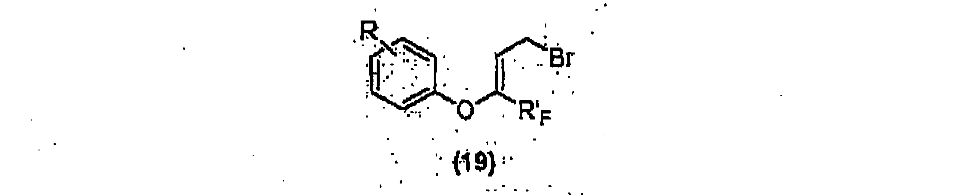 in which R and R'_{F} are as defined in claim 1, starting from a compound (18) obtained by the process according to claim 46.

49. Process according to claim 48, **characterised in that** it involves reacting the compound (18) with PX₃, in which X represents a halogen atom.

50. Process for preparing a compound having formula (20) 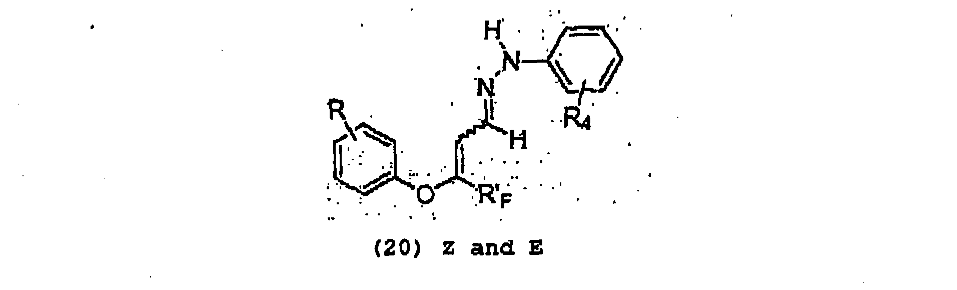 in which R and R'_{F} are as defined in claim 1 and in which R₄ is the same as R₁ defined in claim 22, starting from a compound (1).

51. Process according to claim 50, **characterised in that** it involves reacting the compound (1) with a phenyl hydrazine.

52. Process for preparing a compound having formula (21) 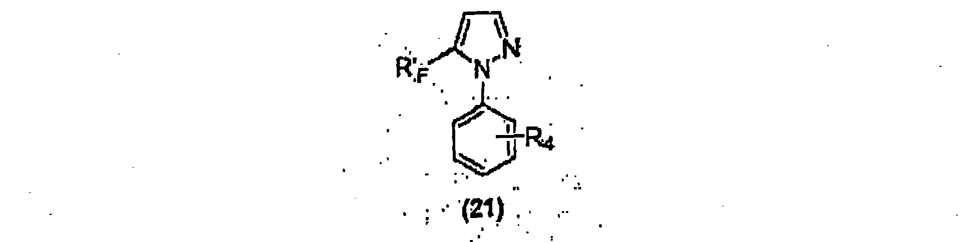 in which R'_{F} is as defined in claim 1 and in which R₄ is the same as R₁ defined in claim 22, starting from a compound (20) obtained by a process according to claim 50.

53. Process according to claim 52, **characterised in that** it involves cyclising the compound (20) in the presence of pyridine in refluxing toluene.

54. Process for preparing a compound having formula (22) 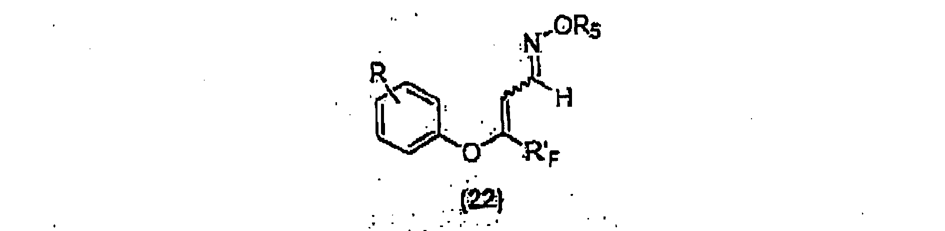 in which R and R'_{F} are as defined in claim 1 and in which R₅ is the same as R₁ defined according to claim 22, starting from a compound (1).

55. Process according to claim 54, **characterised in that** it involves reacting the compound (1) with a compound having the general formula NH₂OR₅.

56. Process for preparing a compound having formula (23) 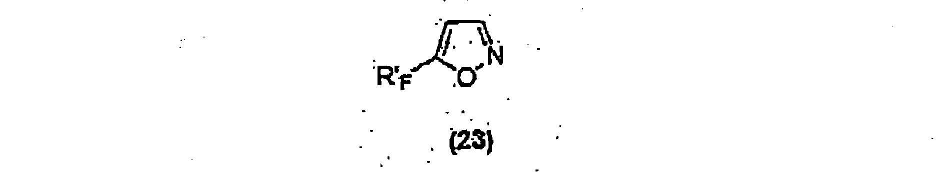 in which R'_{F} is as defined in claim 1, starting from a compound (22) obtained by the process according to claim 54, in which R₅ is a hydrogen atom.

57. Preparation process according to claim 56,
**characterised in that** it involves cyclising a compound (22) in which R₅ is a hydrogen atom, in the presence of pyridine in refluxing toluene.

58. Process for preparing a compound having formula (24) 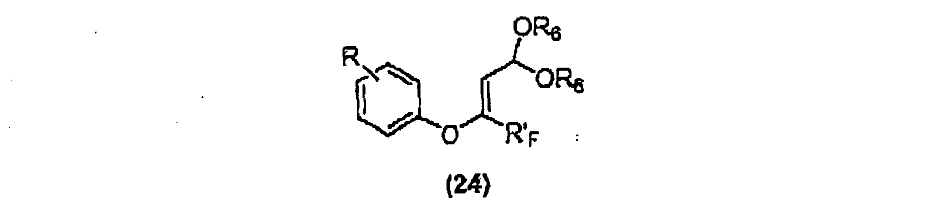 in which R and R'_{F} are as defined in claim 1 and in which R₆ is the same as R₁ defined according to claim 22, starting from a compound (1).

59. Process according to claim 58, **characterised in that** it involves reacting the compound (1) with a compound having the general formula CH(OR₆)₃·

60. Process for preparing a compound having formula (25) 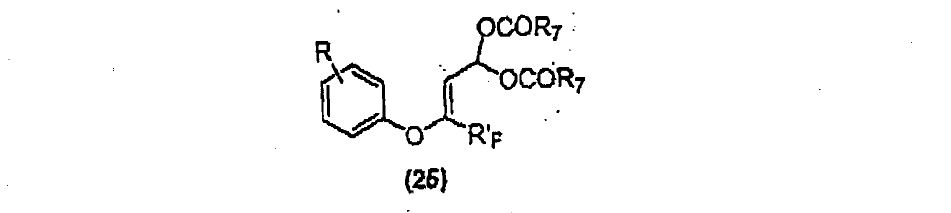 in which R and R'_{F} are as defined in claim 1 and in which R₇ is the same as R₁ defined according to claim 22, starting from a compound (1).

61. Process according to claim 60, **characterised in that** it involves reacting the compound (1) with a compound having the general formula (R₇COX), in which X represents a halogen atom.

62. Process for preparing a compound having formula (26) 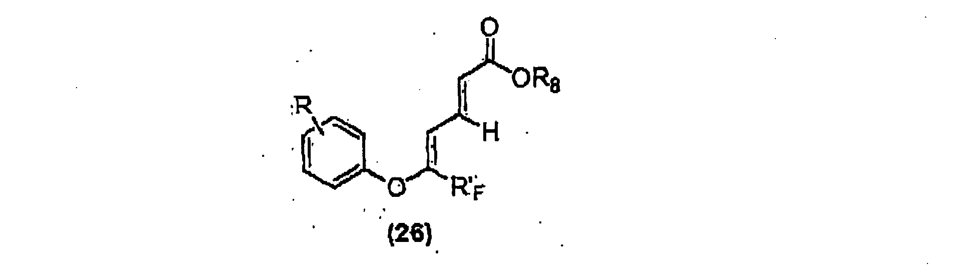 in which R and R'_{F} are as defined in claim 1 and in which R₈ is the same as R₁ defined according to claim 22, starting from a compound (1).

63. Preparation process according to claim 62,
**characterised in that** it involves reacting the compound (1) with a phosphonoacetate-type compound.

64. Process for preparing a compound having formula (27) 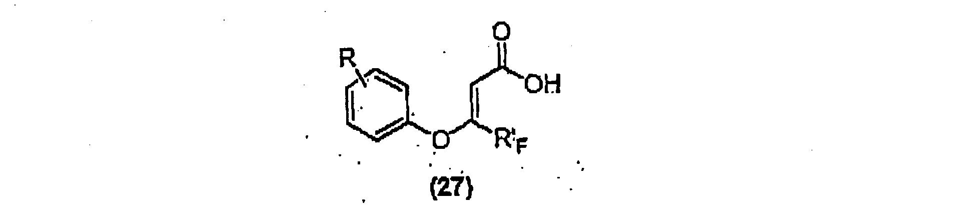 in which R and R'_{F} are as defined in claim 1, starting from a compound (1).

65. Process according to claim 64, **characterised in that** it involves reacting the compound (1) with sodium chlorite and hydrogen peroxide.

66. Process for preparing a compound having formula (28) 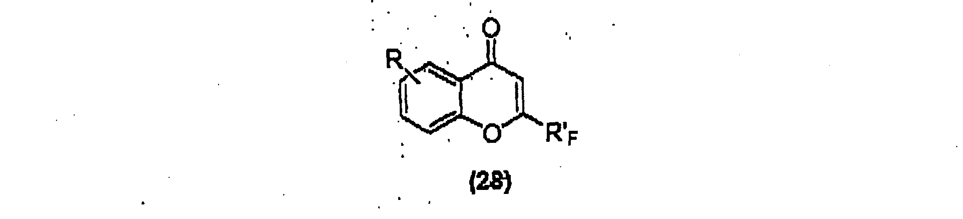 in which R and R'_{F} are as defined in claim 1, starting from a compound (27) obtained by a process according to claim 64.

67. Preparation process according to claim 66,
**characterised in that** it involves cyclising the compound (27) in the presence of polyphosphoric acid.

68. Process for preparing a compound having formula (29) 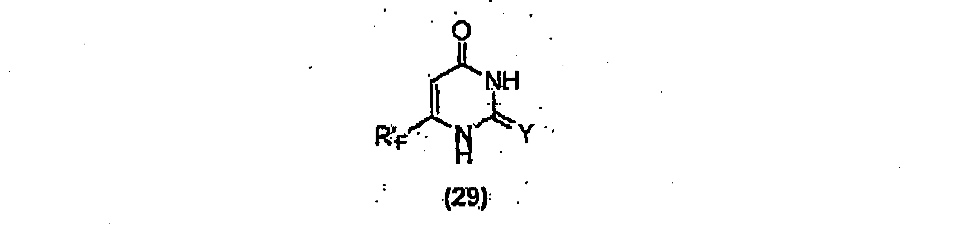 in which R'_{F} is as defined in claim 1 and in which Y represents O or S, starting from a compound (27) obtained according to the process of claim 64.

69. Process according to claim 68, **characterised in that** it involves reacting the compound (27) with urea if Y is O or with thiourea if Y is S.

70. Process for preparing a compound having formula (30) 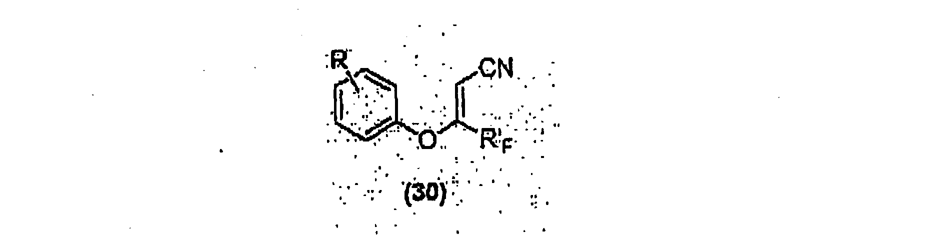 in which R and R'_{F} are as defined in claim 1 and in which Y is O or S, starting from a compound (22) obtained by a process according to claim 54.

71. Process according to claim 70, **characterised in that** it involves reacting the compound (22) with a compound having formula (PX₅) in which X represents a halogen atom.

## Patentansprüche

1. Verbindung der Formel (1) 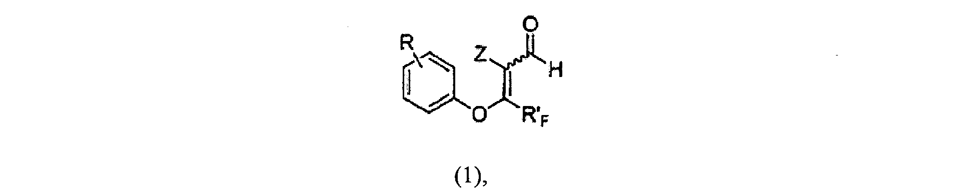 wobei
- Z ein Wasserstoffatom oder ein Halogenatom darstellt;
- R'_{F} eine CₙF₂ₙ₊₁-Perfluoralkylgruppe darstellt, wobei n eine ganze Zahl ist, die im Bereich von 1 bis 12 liegen kann;
- R sich in ortho-, meta- oder para-Position befindet und eine elektronenschiebende Gruppe, eine elektronenziehende Gruppe, eine Arylgruppe oder eine Heteroarylgruppe darstellt, mit der Maßgabe, dass, wenn R ein Aryl oder ein Heteroaryl ist, es eine aromatische Gruppe mit kondensierten Kernen mit der Phenylgruppe, die sie trägt, bilden kann, oder R ein Wasserstoffatom ist.

2. Verbindung nach Anspruch 1, **dadurch gekennzeichnet, dass** R eine elektronenschiebende Gruppe ist, die aus Alkylgruppen, die 1 bis 6 Kohlenstoffatomen einschließen, oder Alkoxygruppen, die 1 bis 6 Kohlenstoffatome einschließen, oder aus der OH-Gruppe ausgewählt ist.

3. Verbindung nach Anspruch 1, **dadurch gekennzeichnet, dass** R die Methoxygruppe darstellt.

4. Verbindung nach Anspruch 1, **dadurch gekennzeichnet, dass** eine R eine elektronenziehende Gruppe ist, die ausgewählt ist aus:
- NO₂- und Nitril-Gruppen,
- Halogenatomen,
- einer Carbonylgruppe -C(=O)-R₉, wobei R₉ ein Wasserstoffatom, eine OH-Gruppe, eine Alkylgruppe, eine Alkoxygruppe, eine Arylgruppe, eine Aryloxygruppe, eine Heteroarylgruppe oder eine Heteroaryloxygruppe ist, wobei die Carbonylgruppe an der Gruppe, die sie trägt, entweder direkt oder über eine Alkylengruppe, eine Arylengruppe, eine Heteroarylengruppe gebunden ist;
- eine Gruppe -O-C(=O)-R₁₀, wobei R₁₀ ein Wasserstoffatom, eine Alkylgruppe, eine Arylgruppe, eine Heteroarylgruppe ist, wobei die Gruppe -O-C(=O)-R₁₀ an der Gruppe, die sie trägt, entweder direkt oder über eine Alkylengruppe, eine Arylengruppe, eine Heteroarylengruppe gebunden ist.

5. Verbindung nach einem der Ansprüche 1 bis 4, **dadurch gekennzeichnet, dass** R'_{F} eine CₙF₂ₙ₊₁-Gruppe darstellt, wobei n eine ganze Zahl von 1 bis 8 ist.

6. Verbindung nach einem der Ansprüche 1 bis 5, **dadurch gekennzeichnet, dass** sie dem Z-Isomer entspricht.

7. Verbindung nach einem der Ansprüche 1 bis 5, **dadurch gekennzeichnet, dass** sie dem E-Isomer entspricht.

8. Verbindung nach einem der Ansprüche 1 bis 7, **dadurch gekennzeichnet, dass** Z ein Wasserstoffatom darstellt (Verbindung 1a).

9. Verbindung nach einem der Ansprüche 1 bis 7, **dadurch gekennzeichnet, dass** Z ein Halogenatom darstellt (Verbindung 1b).

10. Verfahren zur Herstellung einer Verbindung 1a nach Anspruch 8, **dadurch gekennzeichnet, dass** es darin besteht, in einem Lösungsmittel eine Verbindung der Formel (2) 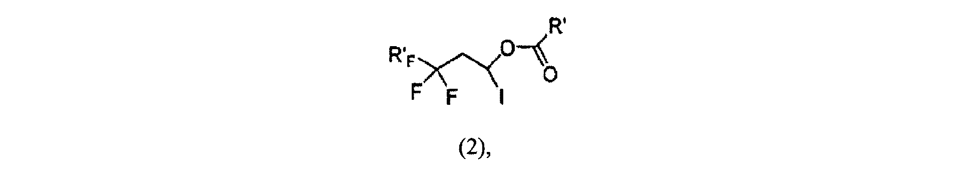 wobei R'_{F} wie in Anspruch 1 definiert ist und R' ein C1- bis C4-Alkyl oder ein Aryl ist, mit einer Verbindung der Formel (3) 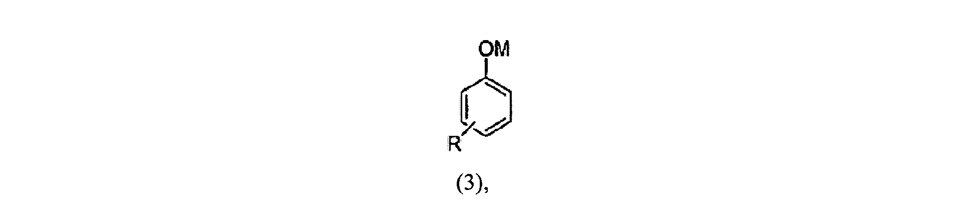 wobei R wie in Anspruch 1 definiert ist und M ein Alkalimetall darstellt, reagieren zu lassen.

11. Verfahren nach Anspruch 10, **dadurch gekennzeichnet, dass** R' eine Methylgruppe darstellt und M Na ist.

12. Verfahren nach Anspruch 10, **dadurch gekennzeichnet, dass** die Umsetzung unter den folgenden Bedingungen durchgeführt wird:
- das Molverhältnis zwischen der Verbindung der Formel (3) und der Verbindung der Formel (2) beträgt 3 bis 3,5,
- die Temperatur beträgt 0 bis 70 °C;
- das Lösungsmittel ist ein Alkan oder ein Gemisch von flüssigen Alkanen mit einer Siedetemperatur unter 100 °C oder ein aromatischer Kohlenwasserstoff oder ein Petrolether.

13. Herstellungsverfahren nach Anspruch 12, **dadurch gekennzeichnet, dass** das Molverhältnis zwischen der Verbindung der Formel (3) und der Verbindung der Formel (2) 3 beträgt.

14. Verfahren nach Anspruch 12 zur Herstellung einer Verbindung (1a), wobei R eine elektronenschiebende Gruppe ist, **dadurch gekennzeichnet, dass** die Reaktionstemperatur etwa 20 °C beträgt.

15. Verfahren nach Anspruch 12 zur Herstellung einer Verbindung (1a), wobei R eine elektronenziehende Gruppe ist, **dadurch gekennzeichnet, dass** die Reaktionstemperatur etwa 70 °C beträgt.

16. Verfahren nach Anspruch 10, **dadurch gekennzeichnet, dass** die Verbindung (2) durch Umsetzung einer Verbindung der Formel R'_{F}-CF₂I, wobei R'_{F} CₙF₂ₙ₊₁ ist, n eine ganze Zahl von 1 bis 12 ist, mit einer Verbindung CH₂=CH-O-CO-R' in Gegenwart eines Radikalstarters hergestellt wird.

17. Verfahren nach Anspruch 16, **dadurch gekennzeichnet, dass** n eine gerade Zahl ist und die Verbindung R'_{F}-CF₂I durch Telomerisation von CF₃I mit Tetrafluorethylen hergestellt wird.

18. Verfahren nach Anspruch 16, **dadurch gekennzeichnet, dass** n eine ungerade Zahl ist und die Verbindung R'_{F}-CF₂I durch Telomerisation von Tetrafluorethylen mit IF₅ hergestellt wird.

19. Verfahren zur Herstellung einer Verbindung (1b) nach Anspruch 9, **dadurch gekennzeichnet, dass** es darin besteht, mit einer Verbindung (1a) nach Anspruch 8 in Tetrachlorkohlenstoff und unter UV-Bestrahlung eine Halogenierung durchzuführen.

20. Verfahren zur Herstellung eines 2-Perfluoralkylpyridins der Formel (5) 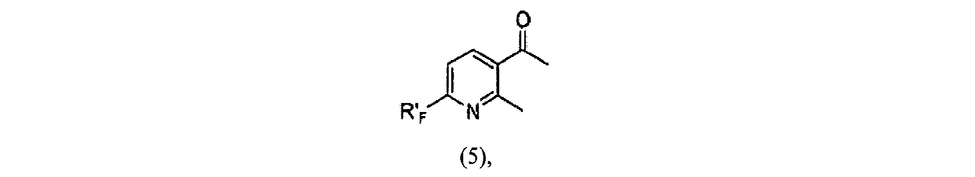 wobei R'_{F} wie in Anspruch 1 definiert ist, aus einer Verbindung der Formel (1).

21. Verfahren nach Anspruch 20, **dadurch gekennzeichnet, dass** es darin besteht, eine Verbindung (1) mit Acetylaceton in Ethanol in Gegenwart von Ammoniumacetat und bei Umgebungstemperatur reagieren zu lassen, um die Verbindung der Formel (4), nachstehend, 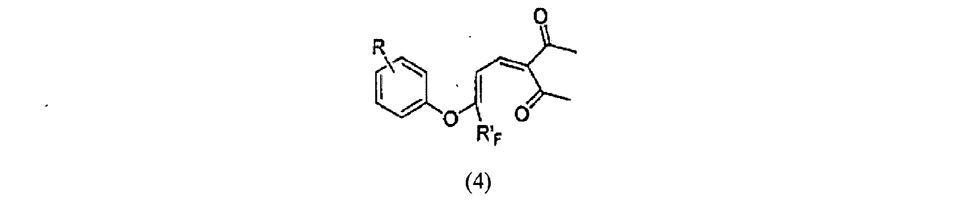 zu erhalten, und **dadurch**, anschließend die Verbindung der Formel (4) mit Ammoniak in Ethanol reagieren zu lassen.

22. Verfahren zur Herstellung eines 2-Perfluoralkylchinolins der Formel (6) 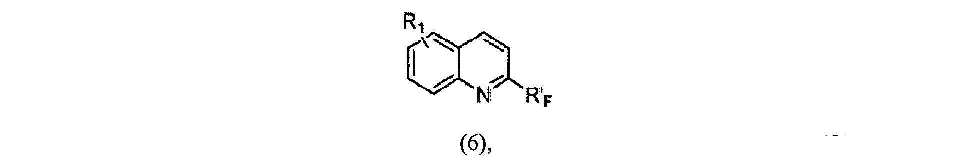 wobei R'_{F} wie in Anspruch 1 definiert ist, und wobei R₁ wie für R in Anspruch 1 definiert ist, aus einer Verbindung der Formel (1).

23. Verfahren nach Anspruch 22, **dadurch gekennzeichnet, dass** R₁ ein Wasserstoffatom, eine Alkylgruppe, die 1 bis 6 Kohlenstoffatome einschließt, eine Arylgruppe, eine Heteroarylgruppe, eine Alkoxygruppe, die 1 bis 6 Kohlenstoffatome einschließt, eine Aryloxygruppe, eine Hydroxylgruppe, eine Nitrogruppe oder ein Halogenatom darstellt.

24. Verfahren nach Anspruch 22, **dadurch gekennzeichnet, dass** es darin besteht, eine Verbindung der Formel (1) mit einem Anilin in Tetrahydrofuran in Gegenwart von Ameisensäure bei einer Temperatur von 60 bis 65 °C reagieren zu lassen.

25. Verfahren zur Herstellung einer Verbindung der Formel (7) 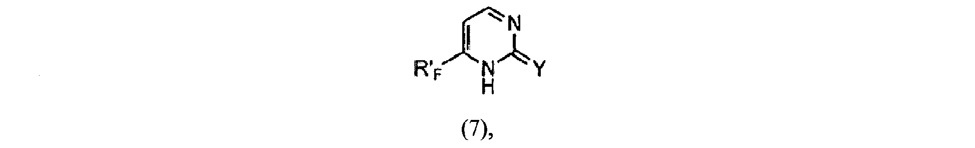 wobei R'_{F} wie in Anspruch 1 definiert ist und wobei Y ein Sauerstoffatom oder ein Schwefelatom darstellt, aus einer Verbindung der Formel (1).

26. Verfahren nach Anspruch 25, **dadurch gekennzeichnet, dass** es darin besteht, eine Verbindung der Formel (1) mit einer Verbindung der Formel (7') 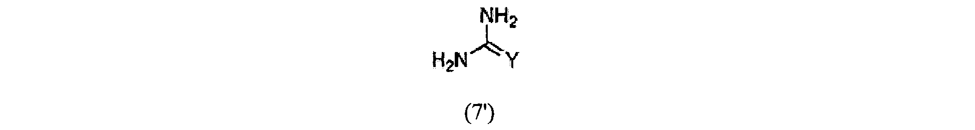 in Ethanol bei einer Temperatur von 78 bis 85 °C und in Gegenwart von Schwefelsäure oder Chlorwasserstoffsäure reagieren zu lassen.

27. Verfahren zur Herstellung einer Verbindung der Formel (8) 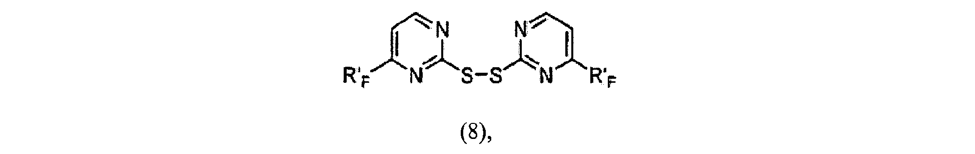 wobei R'_{F} wie in Anspruch 1 definiert ist, durch Dimerisierung an Luft einer Verbindung der Formel (7), die durch das Verfahren nach Anspruch 25 erhalten wird, wobei Y ein Schwefelatom darstellt.

28. Verfahren zur Herstellung eines 2-Perfluoralkylchrom-3-en-2-ols der Formel (9) 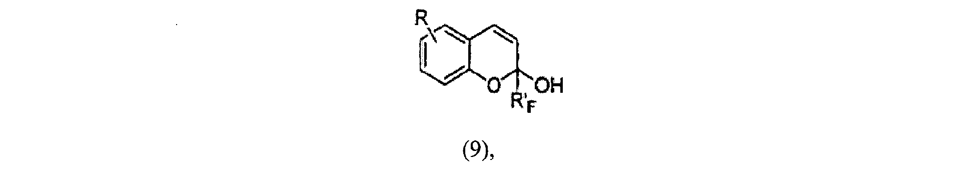 wobei R'_{F} und R wie in Anspruch 1 definiert sind, aus einer Verbindung (1).

29. Verfahren nach Anspruch 28, **dadurch gekennzeichnet, dass** es darin besteht, die Verbindung der Formel (1) mit einer Lewis-Säure in 1,2-Dichlorethan oder Dichlormethan bei einer Temperatur von 85 bis 90 °C reagieren zulassen.

30. Verfahren zur Herstellung einer Verbindung der Formel (10) 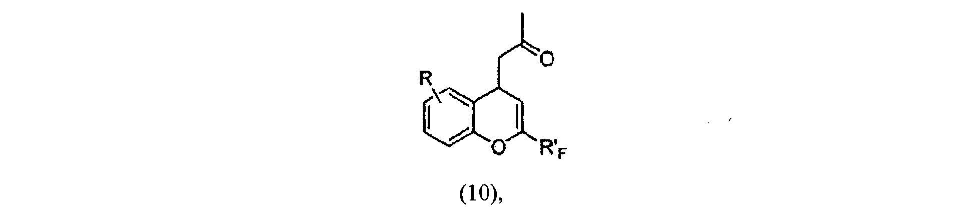 wobei R'_{F} und R wie in Anspruch 1 definiert sind, aus einer Verbindung der Formel (9), die durch das Verfahren nach Anspruch 28 erhalten wird.

31. Verfahren nach Anspruch 30, **dadurch gekennzeichnet, dass** es darin besteht, die Verbindung der Formel (9) mit 2-(Trimethylsilyloxy)-propen in einem Alkan-Lösungsmittel oder in einem Alkangemisch in Gegenwart von SnCl₄ bei einer Temperatur von -30 bis -20 °C reagieren zu lassen.

32. Verbindung der Formel (10) 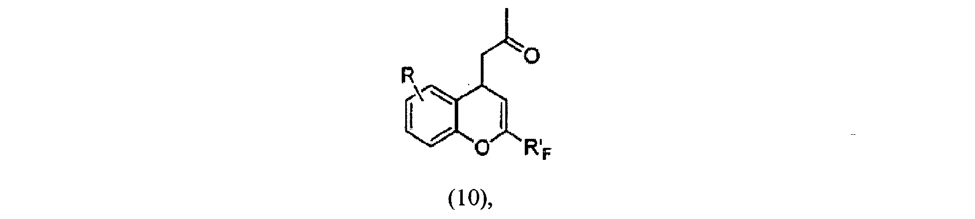 wobei R und R'_{F} wie in Anspruch 1 definiert sind.

33. Verfahren zur Herstellung einer Verbindung der Formel (11) 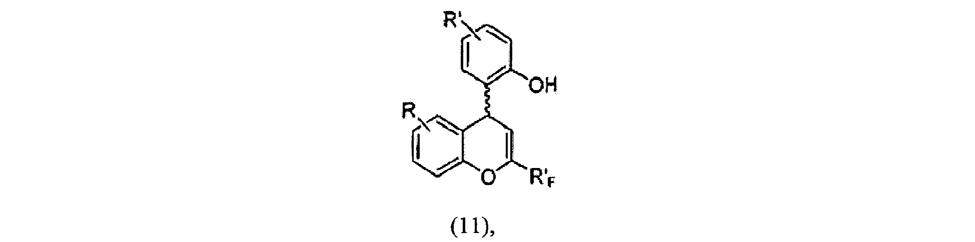 wobei R und R'_{F} wie in Anspruch 1 definiert sind und wobei R' wie in Anspruch 10 definiert ist, aus einer Verbindung der Formel (9), die durch ein Verfahren nach Anspruch 28 erhalten wird.

34. Verfahren nach Anspruch 33, **dadurch gekennzeichnet, dass** es darin besteht, die Verbindung der Formel (9) mit 4-Methoxyphenol in Tetrahydrofuran in Gegenwart von Ameisensäure bei einer Temperatur von 0 bis 20 °C reagieren zu lassen.

35. Verbindung der Formel (11) 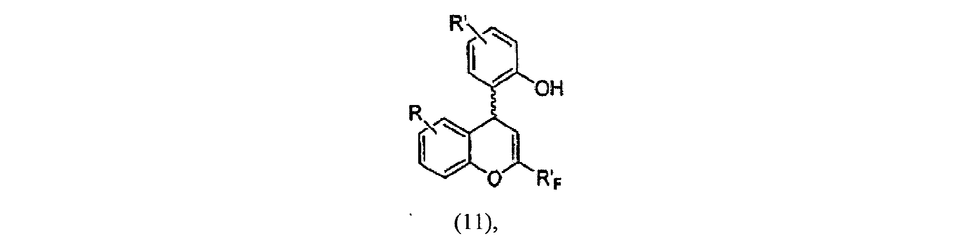 wobei R und R'_{F} wie in Anspruch 1 definiert sind und wobei R' wie in Anspruch 10 definiert ist.

36. Verfahren zur Herstellung einer Verbindung der Formel (12) 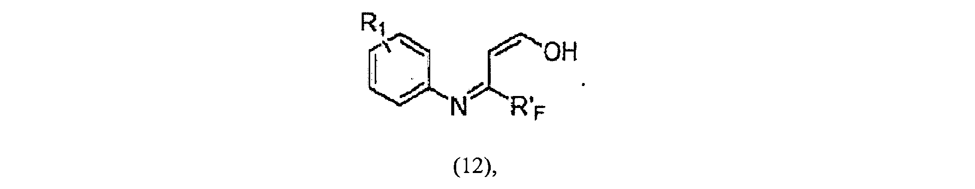 wobei R'_{F} wie in Anspruch 1 definiert ist und R₁ wie in Anspruch 23 definiert ist, aus einer Verbindung der Formel (1).

37. Verfahren nach Anspruch 36, **dadurch gekennzeichnet, dass** es darin besteht, die Verbindung (1) mit einem Anilin in Methanol in Gegenwart von Chlorwasserstoffsäure reagieren zu lassen.

38. Verfahren zur Herstellung einer Verbindung der Formel (13) 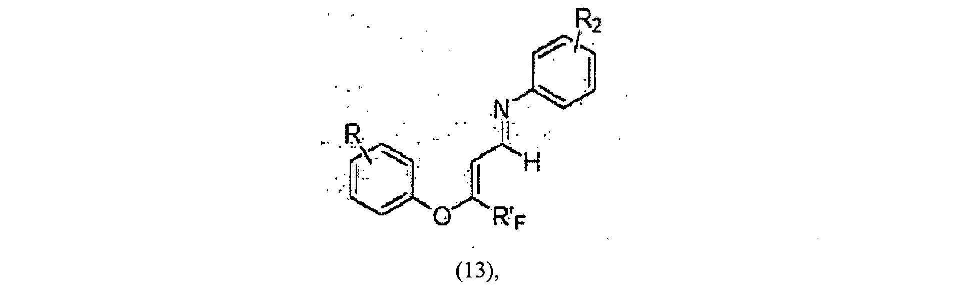 wobei R'_{F} wie in Anspruch 1 definiert ist und wobei R₂ wie für R₁ in Anspruch 22 definiert ist, aus einer Verbindung der Formel (1).

39. Verfahren nach Anspruch 38, **dadurch gekennzeichnet, dass** es darin besteht, die Verbindung (1) mit einem Anilin in Dichlormethan reagieren zu lassen.

40. Verfahren zur Herstellung eines asymmetrischen Diazapentadiens der Formel (14) 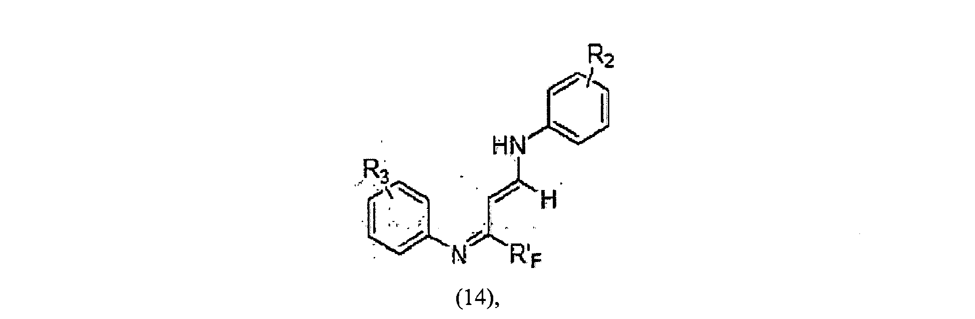 wobei R'_{F} wie in Anspruch 1 definiert ist und wobei R₂ und R₃ unabhängig voneinander wie R₁, definiert in Anspruch 22, sind, aus einer Verbindung (13), die durch das Verfahren nach Anspruch 38 erhalten wird.

41. Verfahren nach Anspruch 40, **dadurch gekennzeichnet, dass** es darin besteht, die Verbindung (13) mit einem Anilin in Dichlormethan reagieren zu lassen.

42. Verfahren zur Herstellung einer Verbindung der Formel (15) 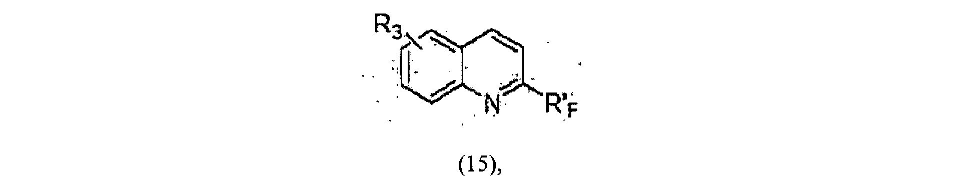 wobei R'_{F} wie in Anspruch 1 definiert ist und wobei R₃ wie R₁, definiert nach Anspruch 22, ist, aus einer Verbindung (13), die durch das Verfahren nach Anspruch 38 erhalten wird.

43. Verfahren nach Anspruch 42, **dadurch gekennzeichnet, dass** es darin besteht, die Verbindung (13) mit einem Anilin in Dichlormethan unter Rückfluss reagieren zu lassen.

44. Verfahren zur Herstellung eines Iminoenols der Formel (16) 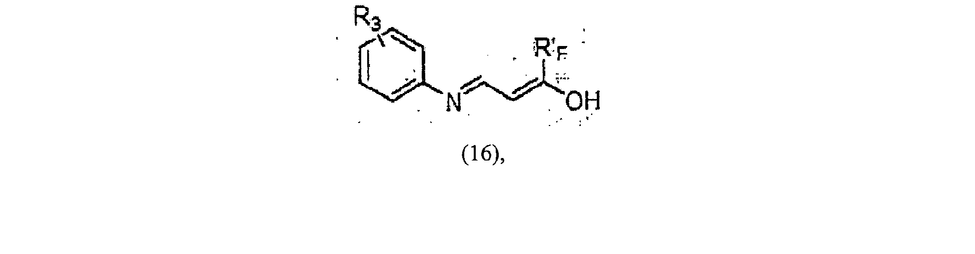 wobei R'_{F} wie in Anspruch 1 definiert ist, und wobei R₃ wie R₁, definiert nach Anspruch 22, ist, aus einer Verbindung (1).

45. Verfahren nach Anspruch 44, **dadurch gekennzeichnet, dass** es darin besteht, die Verbindung (1) mit einem Anilin in Gegenwart von Paratoluolsulfonsäure reagieren zu lassen.

46. Verfahren zur Herstellung einer Verbindung der Formel (18) 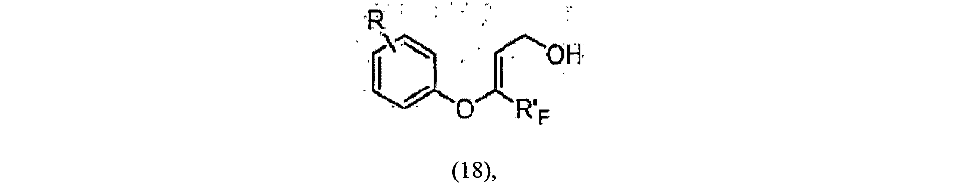 wobei R und R'_{F} wie in Anspruch 1 definiert sind, aus einer Verbindung (1).

47. Verfahren nach Anspruch 46, **dadurch gekennzeichnet, dass** es darin besteht, die Verbindung (1) mit Natriumborhydrid reagieren zu lassen.

48. Verfahren zur Herstellung einer Verbindung der Formel (19) 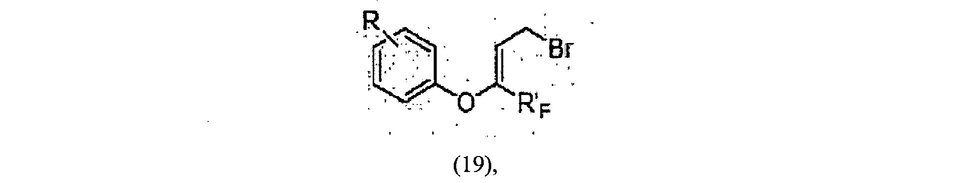 wobei R und R'_{F} wie in Anspruch 1 definiert sind, aus einer Verbindung (18), die durch das Verfahren nach Anspruch 46 erhalten wird.

49. Verfahren nach Anspruch 48, **dadurch gekennzeichnet, dass** es darin besteht, die Verbindung (18) mit PX₃, wobei X ein Halogenatom darstellt, reagieren zu lassen.

50. Verfahren zur Herstellung einer Verbindung der Formel (20) 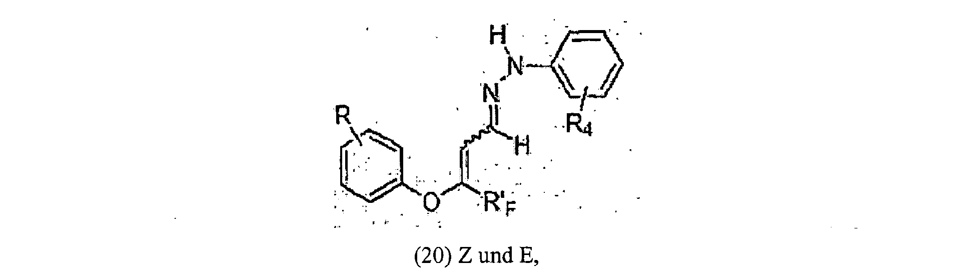 wobei R und R'_{F} wie in Anspruch 1 definiert sind und wobei R₄ wie R₁, definiert in Anspruch 22, ist, aus einer Verbindung (1).

51. Verfahren nach Anspruch 50, **dadurch gekennzeichnet, dass** es darin besteht, die Verbindung (1) mit einem Phenylhydrazin reagieren zu lassen.

52. Verfahren zur Herstellung einer Verbindung der Formel (21) 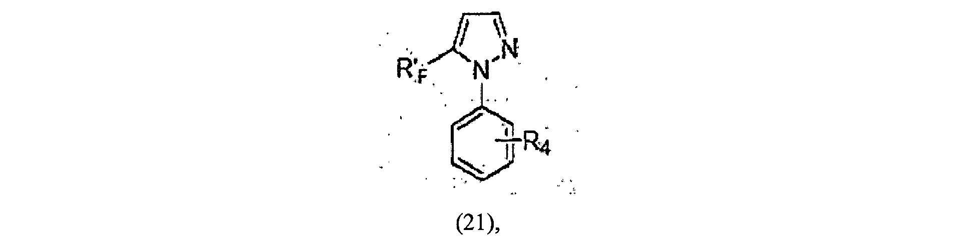 wobei R'_{F} wie in Anspruch 1 definiert ist und wobei R₄ wie R₁, definiert in Anspruch 22, ist, aus einer Verbindung (20), die durch ein Verfahren nach Anspruch 50 erhalten wird.

53. Verfahren nach Anspruch 52, **dadurch gekennzeichnet, dass** es darin besteht, die Verbindung (20) in Gegenwart von Pyridin in Toluol unter Rückfluss zu cyclisieren.

54. Verfahren zur Herstellung einer Verbindung der Formel (22) 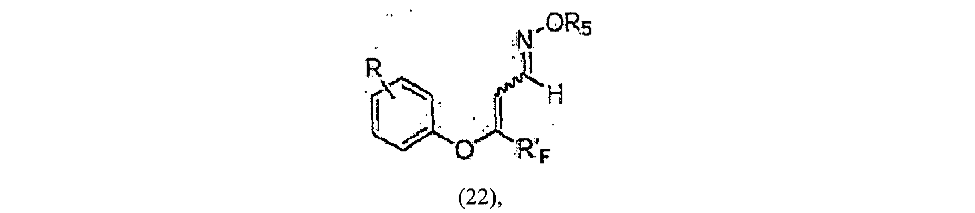 wobei R und R'_{F} wie in Anspruch 1 definiert sind und wobei R₅ wie R₁, definiert nach Anspruch 22, ist, aus einer Verbindung (1).

55. Verfahren nach Anspruch 54, **dadurch gekennzeichnet, dass** es darin besteht, die Verbindung (1) mit einer Verbindung der allgemeinen Formel NH₂OR₅ reagieren zu lassen.

56. Verfahren zur Herstellung einer Verbindung der Formel (23) 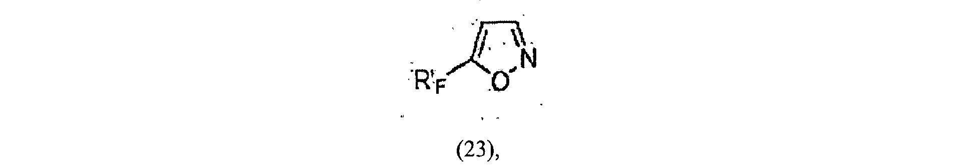 wobei R'_{F} wie in Anspruch 1 definiert ist, aus einer Verbindung (22), die durch das Verfahren nach Anspruch 54 erhalten wird, wobei R₅ ein Wasserstoffatom ist.

57. Verfahren zur Herstellung nach Anspruch 56, **dadurch gekennzeichnet, dass** es darin besteht, eine Verbindung (22), wobei R₅ ein Wasserstoffatom ist, in Gegenwart von Pyridin in Toluol unter Rückfluss zu cyclisieren.

58. Verfahren zur Herstellung einer Verbindung der Formel (24) 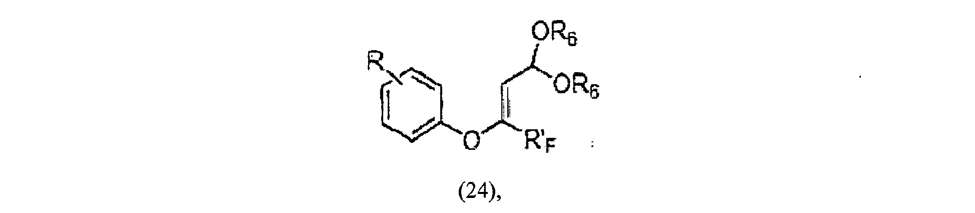 wobei R und R'_{F} wie in Anspruch 1 definiert sind und wobei R₆ wie R₁, definiert nach Anspruch 22, ist, aus einer Verbindung (1).

59. Verfahren nach Anspruch 58, **dadurch gekennzeichnet, dass** es darin besteht, die Verbindung (1) mit einer Verbindung der allgemeinen Formel CH(OR₆)₃ reagieren zu lassen.

60. Verfahren zur Herstellung einer Verbindung der Formel (25) 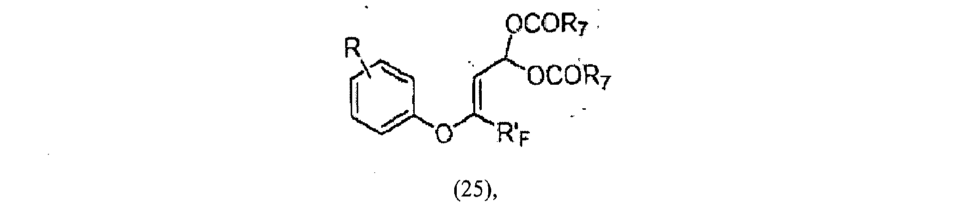 wobei R und R'_{F} wie in Anspruch 1 definiert sind und wobei R₇ wie R₁, definiert nach Anspruch 22, ist, aus einer Verbindung (1).

61. Verfahren nach Anspruch 60, **dadurch gekennzeichnet, dass** es darin besteht, die Verbindung (1) mit einer Verbindung der allgemeinen Formel (R₇COX) reagieren zu lassen, wobei X ein Halogenatom darstellt.

62. Verfahren zur Herstellung einer Verbindung der Formel (26) 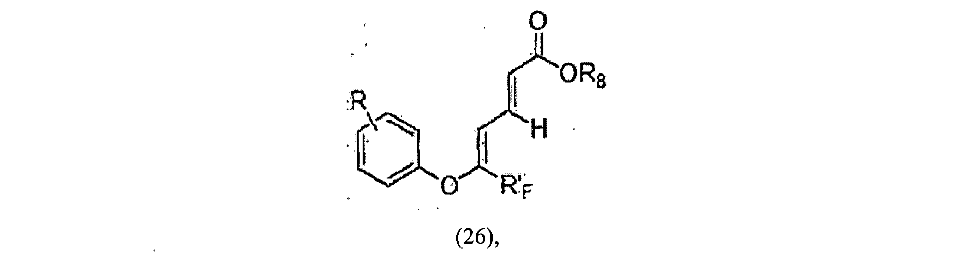 wobei R und R'_{F} wie in Anspruch 1 definiert sind und wobei R₈ wie R₁, definiert nach Anspruch 22, ist, aus einer Verbindung (1).

63. Verfahren zur Herstellung nach Anspruch 62, **dadurch gekennzeichnet, dass** es darin besteht, die Verbindung (1) mit einer Phosphonoacetat-Verbindung reagieren zu lassen.

64. Verfahren zur Herstellung einer Verbindung der Formel (27) 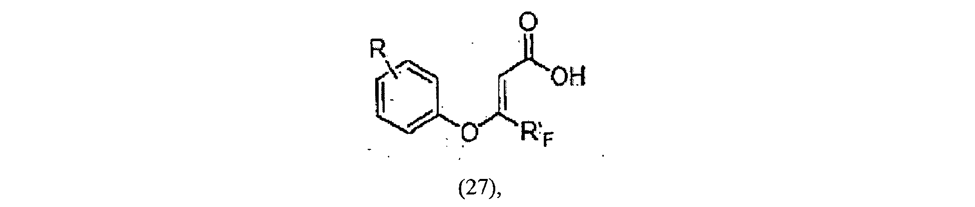 wobei R und R'_{F} wie in Anspruch 1 definiert sind, aus einer Verbindung (1).

65. Verfahren nach Anspruch 64, **dadurch gekennzeichnet, dass** es darin besteht, die Verbindung (1) mit Natriumchlorit und Wasserstoffperoxid reagieren zu lassen.

66. Verfahren zur Herstellung einer Verbindung der Formel (28) 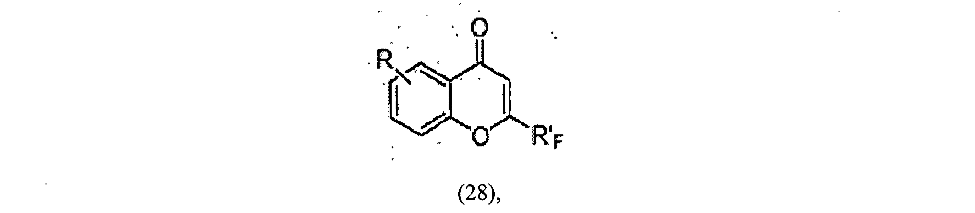 wobei R und R'_{F} wie in Anspruch 1 definiert sind, aus einer Verbindung (27), die durch ein Verfahren nach Anspruch 64 erhalten wird.

67. Verfahren zur Herstellung nach Anspruch 66, **dadurch gekennzeichnet, dass** es darin besteht, die Verbindung (27) in Gegenwart von Polyphosphorsäure zu cyclisieren.

68. Verfahren zur Herstellung einer Verbindung der Formel (29) 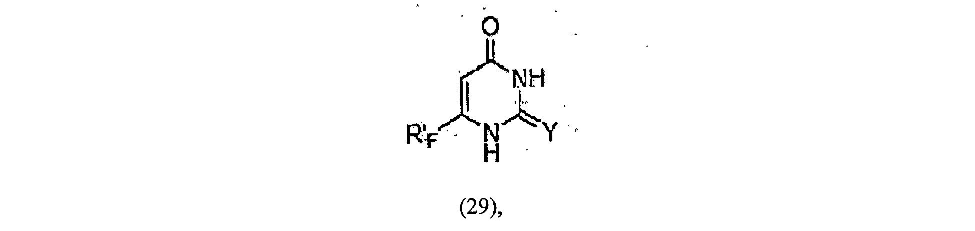 wobei R'_{F} wie in Anspruch 1 definiert ist und wobei Y O oder S darstellt, aus einer Verbindung (27), die durch das Verfahren des Anspruchs 64 erhalten wird.

69. Verfahren nach Anspruch 68, **dadurch gekennzeichnet, dass** es darin besteht, die Verbindung (27) mit Harnstoff reagieren zu lassen, wenn Y O ist, oder mit Thioharnstoff reagieren zu lassen, wenn Y S ist.

70. Verfahren zur Herstellung einer Verbindung der Formel (30) 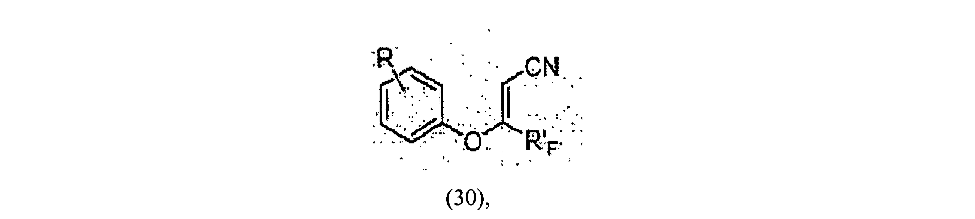 wobei R und R'_{F} wie in Anspruch 1 definiert sind, und wobei Y O oder S ist, aus einer Verbindung (22), die durch ein Verfahren nach Anspruch 54 erhalten wird.

71. Verfahren nach Anspruch 70, **dadurch gekennzeichnet, dass** es darin besteht, die Verbindung (22) mit einer Verbindung der Formel (PX₅), wobei X ein Halogenatom darstellt, reagieren zu lassen.
